# EUROPEAN PATENT SPECIFICATION

(11) **EP 2 233 511 B1**
(45) Date of publication and mention of the grant of the patent: **22.03.2017**
(21) Application number: 09700414.7
(22) Date of filing: 06.01.2009
(51) Int. Cl.: C08G 65/26, A61K 8/04, A61K 8/86, C08F 8/00, C08L 71/02, C09D 5/02, C09D 7/12, C09D 11/02, C09D 153/00

(54) **NOVEL POLYMER PARTICLE AND USE THEREOF**
NEUE POLYMERPARTIKEL UND IHRE ANWENDUNG
NOUVELLE PARTICULE DE POLYMÈRE ET SON UTILISATION

(30) Priority: 07.01.2008 JP 2008000444; 27.03.2008 JP 2008084864; 13.06.2008 JP 2008155244
(43) Date of publication of application: 29.09.2010
(73) Proprietor: Mitsui Chemicals, Inc., Tokyo 105-7117 (JP)
(72) Inventor: MATOISHI, Kaori, Sodegaura-shi Chiba 299-0265 (JP); NAKATSUKA, Shiro, Sodegaura-shi Chiba 299-0265 (JP); NAGAI, Naoshi, Sodegaura-shi Chiba 299-0265 (JP); TAKAKI, Toshihiko, Sodegaura-shi Chiba 299-0265 (JP); FUKUMOTO, Haruhiko, Sodegaura-shi Chiba 299-0265 (JP); NAKAYAMA, Norio, Sodegaura-shi Chiba 299-0265 (JP)
(74) Representative: Raynor, Stuart Andrew
(86) International application number: PCT/JP2009/000014
(87) International publication number: WO 2009/087961

(56) References cited:
- EP-A1- 1 719 794
- JP-A- 2006 131 870
- JP-A- 2006 131 870
- KUANG, DAIBIN ET AL.: 'Smarsly, Bernd, Hierarchical Porous Silica Materials with a Trimodal Pore System Using Surfactant Templates' JOURNAL OF THE AMERICAN CHEMICAL SOCIETY vol. 126, no. 34, 2004, pages 10534 - 10535, XP008138432
- SERTCHOOK, HANAN ET AL.: 'Composite particles of polyethylene @ silica' JOURNAL OF THE AMERICAN CHEMICAL SOCIETY vol. 129, no. 1, 15 December 2006, pages 98 - 108, XP008138430
- THOMAS, ARNE ET AL.: 'Replication of Lyotropic Block Copolymer Mesophases into Porous Silica by Nanocasting: Learning about Finer Details of Polymer Self-Assembly' LANGMUIR vol. 19, no. 10, 2003, pages 4455 - 4459, XP008138421

## Description

### TECHNICAL FIELD

The present invention relates to novel polymer particles and use thereof.

### BACKGROUND ART

A dispersion solution of polyolefin oligomer has been used as an ink or a coating material, while an emulsion composed of polyolefin oligomer and maleic acid-modified polyolefin oligomer has already been reported (Patent Document 1).

It is essential to add a surfactant to such an emulsion in order to reduce its average particle size of 50% by volume. Further, there were restrictions on environments for its stable use, such as causing aggregation and precipitation in an acidic region because such an emulsion is anionic, and the like.

Since some of dispersion solutions exhibit acidic properties in an ink, a coating material and the like, or some of them preferably exhibit neutral properties to weak acidic properties in a cosmetic preparation and the like, there has been required a dispersion solution which can be stably used, containing particles which are dispersed without adding a surfactant and do not cause aggregation and precipitation in a wide range of pH.

Incidentally, there has not been heretofore known a dispersion solution or the like in which other dispersoids are added to the particles contained in the dispersion solution and particles are taken out from the dispersion solution and the particles are dispersed again in a solvent.

A terminally branched copolymer used for the dispersion solution of the present invention corresponds to a vicinal-substitution type of functional group-containing polymer as described in Patent Documents 2 and 3. Patent Documents 2 and 3 disclose the use of it as a dispersion solution as well, but an average particle size of 50% by volume of micelle particles contained in the dispersion solution is not sufficiently small for practical use, and a step of preparing a dispersion solution becomes complicated such that it is necessary to remove a portion of the compound that is not soluble in toluene, and the like. In addition, it fails to disclose whether other dispersoids are contained and particles are taken out. Furthermore, the melting point of the toluene soluble portion is low so that it is difficult to obtain rigid particles.

Patent Document 1: Japanese Patent Laid-open No. 1993-051762
Patent Document 2: Japanese Patent Laid-open No. 2006-131870
Patent Document 3: International Publication Pamphlet No. 2005/073282

### DISCLOSURE OF THE INVENTION

An object of the present invention is to provide polymer particles in which an average particle size of 50% by volume is small, which can be dispersed again in a solvent, in which the particle diameter is constant regardless of the dilute concentration, and which can further contain various dispersoids. A further object is to provide a dispersion solution which contains polymer particles whose average particle size of 50% by volume is small even when a surfactant or the like is not added for the production thereof, and which is obtained by dispersing polymer particles containing various dispersoids without causing aggregation and precipitation in a wide range of pH and temperature, a composite in which polymer particles are dispersed in a matrix composed of a metal oxide, and a mixed composition which can produce the composite. A still further object is to provide the uses of polymer particles, the dispersion solution and the composite.

That is, the present invention relates to the following inventions.
[1] Polymer particles composed of a terminally branched copolymer represented by the following general formula (1) and having a number average molecular weight of not more than 2.5 × 10⁴, 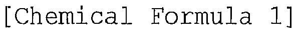 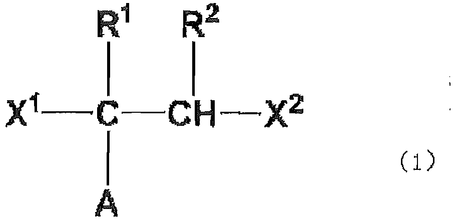 wherein, in the formula, A represents a polyolefin chain; R¹ and R² each represent a hydrogen atom or an alkyl group having 1 to 18 carbon atoms, and at least one of R¹ and R² is a hydrogen atom; and X¹ and X² may be the same or different, and each represent a linear or branched polyalkylene glycol group, or the following general formula (2) or (4),

   [Chemical Formula 2] -E-X³ (2)

   wherein, in the formula, E represents an oxygen atom or a sulfur atom; and X³ represents a polyalkylene glycol group or a group represented by the following general formula (3),

   [Chemical Formula 3] -R³-(G)ₘ (₃)

   wherein, in the formula, R³ represents an (m+1)-valent hydrocarbon group; G may be the same or different, and represents a group represented by -OX⁴ or -NX⁵X⁶ (X⁴ to X⁶ each represent a polyalkylene glycol group); and m is the bonding number of R³ and G, and represents an integer of 1 to 10, 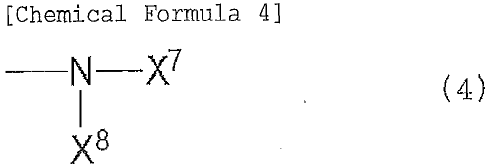 wherein, in the formula, X⁷ and X⁸ may be the same or different, and each represent a polyalkylene glycol group or a group represented by the above-described general formula (3),
   wherein, in the polymer particles, the melting point of the polyolefin chain portion of the terminally branched copolymer is not less than 80 degrees centigrade; and
   wherein an average particle size of 50% by volume is from not less than 1 nm and not more than to 100 nm.
[2] The polymer particles as set forth in [1], wherein, in the terminally branched copolymer represented by the general formula (1), any one of X¹ and X² represents the following general formula (5), 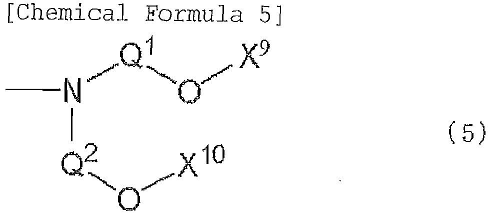 wherein, in the formula, X⁹ and X¹⁰ may be the same or different, and each represent a polyalkylene glycol group; and Q¹ and Q² may be the same or different, and each represent a divalent alkylene group.
[3] The polymer particles as set forth in [1] or [2], wherein, in the terminally branched copolymer represented by the general formula (1), at least one of X¹ and X² represents the following general formula (6),

   [Chemical Formula 6] -O-X¹¹ (6)

   wherein, in the formula, X¹¹ represents a polyalkylene glycol group.
[4] The polymer particles as set forth in any one of [1] to [3], wherein the terminally branched copolymer is represented by the following general formula (1a), 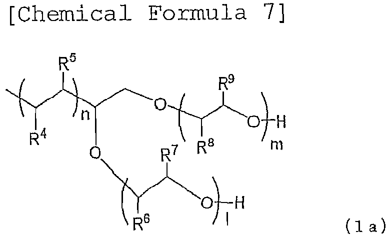 wherein, in the formula, R⁴ and R⁵ each represent a hydrogen atom or an alkyl group having 1 to 18 carbon atoms, and at least one of R⁴ and R⁵ is a hydrogen atom; R⁶ and R⁷ each represent a hydrogen atom or a methyl group, and at least one of R⁶ and R⁷ is a hydrogen atom; R⁸ and R⁹ each represent a hydrogen atom or a methyl group, and at least one of R⁸ and R⁹ is a hydrogen atom; 1+m represents an integer of not less than 2 and not more than 450; and n represents an integer of not less than 20 and not more than 300.
[5] The polymer particles as set forth in any one of [1] to [3], wherein the terminally branched copolymer is represented by the following general formula (1b), 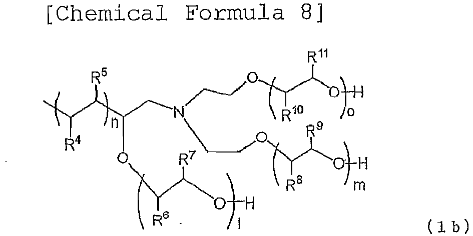 wherein, in the formula, R⁴ and R⁵ each represent a hydrogen atom or an alkyl group having 1 to 18 carbon atoms, and at least one of R⁴ and R⁵ is a hydrogen atom; R⁶ and R⁷ each represent a hydrogen atom or a methyl group, and at least one of R⁶ and R⁷ is a hydrogen atom; R⁸ and R⁹ each represent a hydrogen atom or a methyl group, and at least one of R⁸ and R⁹ is a hydrogen atom; R¹⁰ and R¹¹ each represent a hydrogen atom or a methyl group, and at least one of R¹⁰ and R¹¹ is a hydrogen atom; l+m+o represents an integer of not less than 3 and not more than 450; and n represents an integer of not less than 20 and not more than 300.
[6] The polymer particles as set forth in any one of [1] to [5], wherein, in the polymer particles, the polyolefin chain portion of the terminally branched copolymer has crystallinity.
[7] The polymer particles as set forth in any one of [1] to [6], wherein other dispersoid is contained in an amount of 0.001 weight parts to 20 weight parts, based on 100 weight parts of the terminally branched copolymer.
[8] The polymer particles as set forth in [7], wherein other dispersoid is encapsulated in the polymer particles.
[9] A dispersion solution containing a dispersoid composed of the polymer particles as set forth in any one of [1] to [8], and water and/or an organic solvent having an affinity for water with the dispersoid dispersed therein.
[10] The dispersion solution as set forth in [9], wherein the dispersoid is dispersed in water and/or an organic solvent having an affinity for water.
[11] The dispersion solution as set forth in [9] or [10], wherein the pH is from 1 to 13.
[12] A mixed composition containing the following (A) to (D),
   (A) the polymer particles as set forth in any one of [1] to [8],
   (B) metal alkoxide and/or a hydrolysis condensate thereof,
   (C) water and/or a solvent for dissolving a part of water or entire water in any proportions, and
   (D) a catalyst to be used for the sol-gel reaction.
[13] The mixed composition as set forth in [12], wherein a metal of the metal alkoxide and/or hydrolysis condensate thereof (B) is one or more kinds selected from the group consisting of silicon, zirconium, aluminum and titanium.
[14] The mixed composition as set forth in [12] or [13], wherein the solvent (C) is water.
[15] The mixed composition as set forth in any one of [12] to [14], wherein the solvent (C) is mono alcohol having 1 to 3 carbon atoms.
[16] A composite obtained by dispersing the polymer particles as set forth in any one of [1] to [8] in a matrix composed of a metal oxide.
[17] The composite as set forth in [16] obtained by the sol-gel reaction of the mixed composition as set forth in any one of [12] to [15] is.
[21] The composite as set forth in [16] or [17], wherein the metal oxide is formed by the sol-gel reaction of metal alkoxide and/or a hydrolysis condensate thereof.
[19] The composite as set forth in any one of [16] to [18], further containing alkali metal salts as an anti-static agent.
[20] The composite as set forth in [19], wherein alkali metal salts contain at least one anionic lithium salt selected from the group consisting of trifluoromethanesulfonic acid, bis(trifluoromethanesulfonyl)imide and tri(trifluoromethanesulfonyl)methane.
[21] An ink composition containing the polymer particles as set forth in any one of [1] to [8] or the dispersion solution as set forth in any one of [9] to [11].
[22] A coating agent containing the polymer particles as set forth in any one of [1] to [8] or the dispersion solution as set forth in any one of [9] to [11].
[23] A cosmetic preparation containing the polymer particles as set forth in any one of [1] to [8] or the dispersion solution as set forth in any one of [9] to [11].
[24] An anti-static film obtained by applying the coating agent as set forth in [22].
[25] A plastic laminate having a coating layer composed of the composite as set forth in any one of [16] to [20] on a plastic base material.
[26] The plastic laminate as set forth in [25], having a deposited film layer composed of an inorganic compound between the plastic base material and the coating layer.
[27] A gas barrier material composed of the plastic laminate as set forth in [25] or [26].
[28] A hard coat material composed of the plastic laminate as set forth in [25] or [26].
[29] An anti-static film containing the plastic laminate as set forth in [25] or [26].

According to the present invention, it is possible to provide polymer particles in which an average particle size of 50% by volume is small, which can be dispersed again in a solvent, in which the particle diameter is constant regardless of the dilute concentration, and which can further contain various dispersoids. Furthermore, it is possible to provide a dispersion solution which contains polymer particles whose average particle size of 50% by volume is small even when a surfactant or the like is not added for the production thereof, and which is obtained by dispersing polymer particles containing various dispersoids without causing aggregation and precipitation in a wide range of pH and temperature, a composite in which polymer particles are dispersed in a matrix composed of a metal oxide, and a mixed composition which can produce the composite. Furthermore, it is possible to provide various uses of the polymer particles, the dispersion solution and the composite.

### BRIEF DESCRIPTION OF THE DRAWINGS

Fig. 1 is an observation view of polymer particles in the dispersion solution obtained in Example A1 using a transmission electron microscope.
Fig. 2 is an observation view of polymer particles in the dispersion solution obtained in Example A3 using a transmission electron microscope.
Fig. 3 is an observation view of polymer particles in the dispersion solution obtained in Example A21 using a transmission electron microscope.
Fig. 4 is an observation view of polymer particles in the dispersion solution obtained in Example A23 using a transmission electron microscope.
Fig. 5 is an observation view of polymer particles in the dispersion solution obtained in Example A28 using a transmission electron microscope.

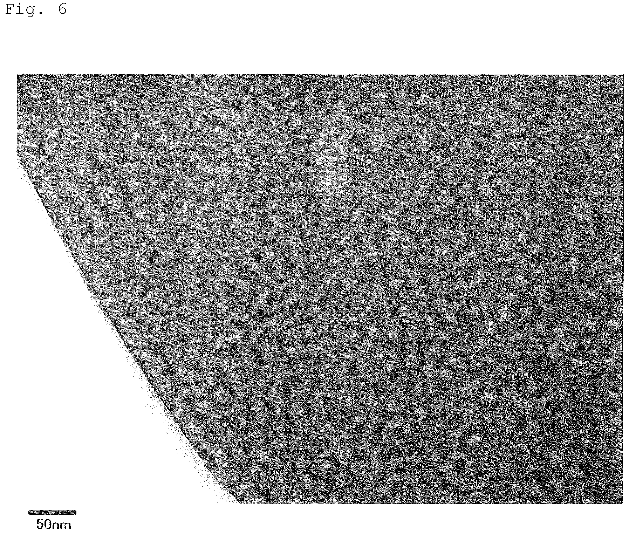

Fig. 6 is an observation view of a cross section of composite particles of the copolymer and silica obtained in Example A20 using a transmission electron microscope.

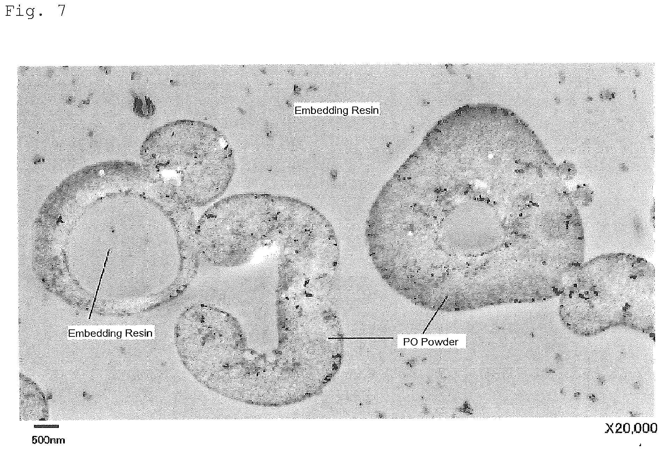

Fig. 7 is an observation view of a cross section of particles obtained in Comparative Example A21 using a transmission electron microscope.

### BEST MODE FOR CARRYING OUT THE INVENTION

The polymer particles of the present invention are composed of a terminally branched copolymer. First, the terminally branched copolymer will be described.

### Terminally Branched Copolymer

The terminally branched copolymer constituting the polymer particles of the present invention has a structure represented by the following general formula (1), 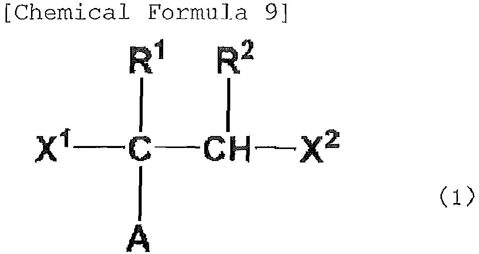 wherein, in the formula, A represents a polyolefin chain; R¹ and R² each represent a hydrogen atom or an alkyl group having 1 to 18 carbon atoms, and at least one of R¹ and R² is a hydrogen atom; and X¹ and X² may be the same or different, and each represent a linear or branched polyalkylene glycol group.

The number average molecular weight of the terminally branched copolymer represented by the general formula (1) is not more than 2.5 × 10⁴, preferably from 5.5 × 10² to 1.5 × 10⁴ and more preferably from 8 × 10² to 4.0 × 10³. Its number average molecular weight is represented by the sum of the number average molecular weight of the polyolefin chain represented by A, the number average molecular weight of the polyalkylene glycol group represented by X¹ and X², and the molecular weight of the R¹, R² and C₂H portions.

If the number average molecular weight of the terminally branched copolymer is in the above range, it is preferable because the stability of particles in the dispersion solution, and the dispersion properties into water and/or an organic solvent having an affinity for water tend to be excellent, and preparation of the dispersion solution becomes easy when the terminally branched copolymer is used as a dispersoid.

The polyolefin chain represented by A in the general formula (1) is obtained by polymerizing an olefin having 2 to 20 carbon atoms. Examples of the olefin having 2 to 20 carbon atoms include α-olefins such as ethylene, propylene, 1-butene, 1-hexene and the like. In the present invention, the polymer may be a homopolymer or copolymer of these olefins, or even a product of copolymerization with other polymerizable unsaturated compounds within the scope of not impairing the characteristics. Among these olefins, ethylene, propylene and 1-butene are particularly preferred.

In the general formula (1), the number average molecular weight measured by GPC, of the polyolefin chain represented by A, is from 400 to 8,000, preferably from 500 to 4,000 and further preferably from 500 to 2, 000. The number average molecular weight used herein is a value calibrated with polystyrene standards.

When the number average molecular weight of the polyolefin chain represented by A is in the above range, it is preferable because crystallinity of the polyolefin portion tends to be excellent, the stability of the dispersion solution tends to be excellent, and preparation of the dispersion solution with low melt viscosity tends to be easy.

The ratio of the weight average molecular weight (Mw) to the number average molecular weight (Mn), both measured by GPC, of the polyolefin chain represented by A in the general formula (1), that is, the molecular weight distribution (Mw/Mn), is not particularly limited and may range from 1.0 to a few tens; however, the ratio is more preferably not more than 4.0 and further preferably not more than 3.0.

When the molecular weight distribution (Mw/Mn) of the group represented by A in the general formula (1) is in the above range, it is preferable in view of the shape of particle in the dispersion solution and uniformity of the particle size.

According to GPC, the number average molecular weight (Mn) and the molecular weight distribution (Mw/Mn) of the group represented by A can be measured using, for example, GPC-150 available from Millipore Corp. under the following conditions.
Separating column: TSK GNH HT (column size: diameter 7.5 mm, length: 300 mm)
Column temperature: 140 degrees centigrade
Mobile phase: o-dichlorobenzene (a product of Wako Pure Chemical Industries, Ltd.)
Anti-oxidant: 0.025 weight % of butylhydroxytoluene (a product of Takeda Pharmaceutical Co., Ltd.)
Flow rate: 1.0 ml/min
Sample concentration: 0.1 weight %
Sample injection amount: 500 µl
Detector: differential refractometer

Incidentally, the molecular weight of the polyolefin chain represented by A can be measured by measuring the molecular weight of the polyolefin having an unsaturated group at one terminal as described later and subtracting the corresponding amount of the terminal molecular weight.

R¹ and R² are each a hydrogen atom or a hydrocarbon group having 1 to 18 carbon atoms, which is a substituent attached to a double bond of the olefin constituting A and preferably a hydrogen atom or an alkyl group having 1 to 18 carbon atoms. Preferred examples of the alkyl group include a methyl group, an ethyl group and a propyl group.

In the general formula (1), X¹ and X² may be the same or different, and each represent a linear or branched polyalkylene glycol group, whose respective number average molecular weights are 50 to 10,000. The branched embodiment of the branched alkylene glycol group is a branch linked through a polyvalent hydrocarbon group or a nitrogen atom, and the like. Examples thereof include a branch by a hydrocarbon group bonded to two or more nitrogen atoms, oxygen atoms or sulfur atoms in addition to the main skeleton, a branch by a nitrogen atom bonded to two alkylene groups in addition to the main skeleton, and the like.

When the number average molecular weight of the polyalkylene glycol group is in the above range, it is preferable because the dispersion properties of the dispersion solution tends to be excellent, and preparation of the dispersion solution with low melt viscosity becomes easy.

X¹ and X² of the general formula (1) have the above-described structure, whereby there are obtained polymer particles composed of a terminally branched copolymer having a particle size with an average particle size of 50% by volume of from 1 nm to 100 nm without using a surfactant.

In the general formula (1), X¹ and X² may be the same or different, and each represent a group represented by the following general formula (2) or (4),

[Chemical Formula 10] -E-E-X³ (2)

wherein, in the formula, E represents an oxygen atom or a sulfur atom; and X³ represents a polyalkylene glycol group or a group represented by the following general formula (3),

[Chemical Formula 11] -R³-(G)ₘ (3)

wherein, in the formula, R³ represents an (m+1)-valent hydrocarbon group; G may be the same or different, and represents a group represented by -OX⁴ or -NX⁵X⁶ (X⁴ to X⁶ each represent a polyalkylene glycol group); and m is the bonding number of R³ and G, and represents an integer of 1 to 10, 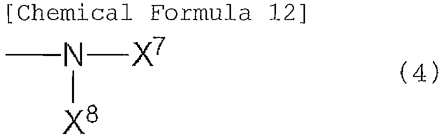 wherein, in the formula, X⁷ and X⁸ may be the same or different, and each represent a polyalkylene glycol group or a group represented by the above-described general formula (3).

In the general formula (3), the group represented by R³ is an (m+1)-valent hydrocarbon group having 1 to 20 carbon atoms. m is 1 to 10, preferably 1 to 6 and particularly preferably 1 to 2.

A preferred example of the general formula (1) includes a terminally branched copolymer in which, in the general formula (1), one of X¹ and X² is a group represented by the general formula (4). A further preferred example include a terminally branched copolymer in which one of X¹ and X² is a group represented by the general formula (4) and the other is represented by the general formula (2).

Another preferred example of the general formula (1) include a terminally branched copolymer in which, in the general formula (1), one of X¹ and X² is a group represented by the general formula (2), and a further preferred example include a terminally branched copolymer in which both X¹ and X² are each a group represented by the general formula (2).

A further preferred construction of X¹ and X² represented by the general formula (4) includes a group represented by the general formula (5), 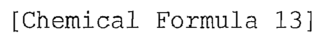 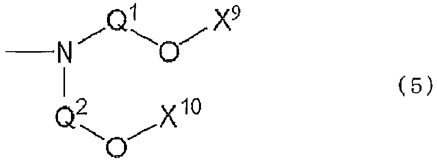 wherein, in the formula, X⁹ and X¹⁰ may be the same or different, and each represent a polyalkylene glycol group; and Q¹ and Q² may be the same or different, and each represent a divalent hydrocarbon group.

The divalent hydrocarbon group represented by Q¹ and Q² in the general formula (5) is preferably a divalent alkylene group, and more preferably an alkylene group having 2 to 20 carbon atoms. The alkylene group having 2 to 20 carbon atoms may have or may not have substituent(s), and examples thereof include an ethylene group, a methylethylene group, an ethylethylene group, a dimethylethylene group, a phenylethylene group, a chloromethylethylene group, a bromomethylethylene group, a methoxymethylethylene group, an aryloxymethylethylene group, a propylene group, a trimethylene group, a tetramethylene group, a hexamethylene group, a cyclohexylene group and the like. The alkylene group is preferably a hydrocarbon based alkylene group, particularly preferably an ethylene group or a methylethylene group, and further preferably an ethylene group. Q¹ and Q² may be one alkylene group or may be two or more kinds of alkylene groups in mixture.

A further preferred structure of X¹ and X² represented by the general formula (2) includes a group represented by the general formula (6),

[Chemical Formula 14] -O-X¹¹ (6)

wherein, in the formula, X¹¹ represents a polyalkylene glycol group.

The polyalkylene glycol group represented by X³ to X¹¹ is a group obtained by the addition polymerization of alkylene oxide. Examples of the alkylene oxide constituting the polyalkylene glycol group represented by X³ to X¹¹ include ethylene oxide, propylene oxide, butylene oxide, styrene oxide, cyclohexene oxide, epichlorohydrin, epibromohydrin, methyl glycidyl ether, allyl glycidyl ether and the like. Among these, preferably used are propylene oxide, ethylene oxide, butylene oxide and styrene oxide. More preferably used are propylene oxide and ethylene oxide, and particularly preferably used is ethylene oxide. The polyalkylene glycol group represented by X³ to X¹¹ may be a group obtained by homopolymerization of these alkylene oxides, or may be a group obtained by copolymerization of two or more kinds thereof. Preferred examples of the polyalkylene glycol group include a polyethylene glycol group, a polypropylene glycol group, or a group obtained by copolymerization of polyethylene oxide and polypropylene oxide, and a particularly preferred example includes a polyethylene glycol group.

If, in the general formula (1), X¹ and X² have the above-described structure, it is preferable because the dispersion properties of water and/or an organic solvent having an affinity for water become excellent when the terminally branched copolymer of the present invention is used as a dispersoid.

As the terminally branched copolymer which can be used for the present invention, it is preferable to use a polymer represented by the following general formula (1a) or (1b), 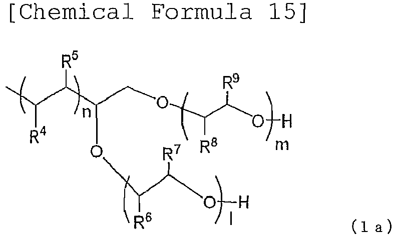 wherein, in the formula, R⁴ and R⁵ each represent a hydrogen atom or an alkyl group having 1 to 18 carbon atoms, and at least one of R⁴ and R⁵ is a hydrogen atom; as the alkyl group, preferably used is an alkyl group having 1 to 9 carbon atoms and further preferably used is an alkyl group having 1 to 3 carbon atoms; R⁶ and R⁷ each represent a hydrogen atom or a methyl group, and at least one of R⁶ and R⁷ is a hydrogen atom; R⁸ and R⁹ each represent a hydrogen atom or a methyl group, and at least one of R⁸ and R⁹ is a hydrogen atom; 1+m represents an integer of not less than 2 and not more than 450 and preferably an integer of not less than 5 and not more than 200; and n represents an integer of not less than 20 and not more than 300 and preferably an integer of not less than 25 and not more than 200, 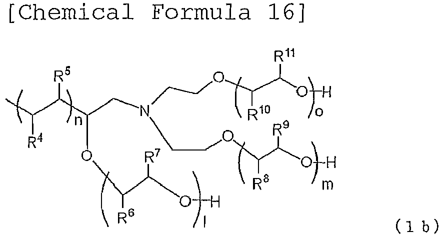 wherein, in the formula, R⁴ and R⁵ each represent a hydrogen atom or an alkyl group having 1 to 18 carbon atoms, and at least one of R⁴ and R⁵ is a hydrogen atom; as the alkyl group, preferably used is an alkyl group having 1 to 9 carbon atoms and further preferably used is an alkyl group having 1 to 3 carbon atoms; R⁶ and R⁷ each represent a hydrogen atom or a methyl group, and at least one of R⁶ and R⁷ is a hydrogen atom; R⁸ and R⁹ each represent a hydrogen atom or a methyl group, and at least one of R⁸ and R⁹ is .a hydrogen atom; R¹⁰ and R¹¹ each represent a hydrogen atom or a methyl group, and at least one of R¹⁰ and R¹¹ is a hydrogen atom; l+m+o represents an integer of not less than 3 and not more than 450 and preferably an integer of not less than 5 and not more than 200; and n represents an integer of not less than 20 and not more than 300 and preferably an integer of not less than 25 and not more than 200.

As the polymer represented by the general formula (1b), further preferably used is a polymer represented by the following general formula (1c), 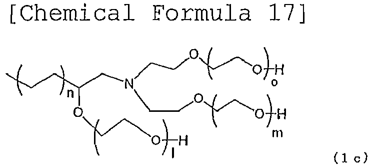 wherein, in the formula, 1+m+o and n are the same as those in the general formula (1b).

The number of ethylene units (n) of the polyethylene chain is calculated by dividing the number average molecular weight (Mn) of the polyolefin group A in the general formula (1) by the molecular weight of the ethylene unit. Furthermore, the total number of ethylene glycol units of the polyethylene glycol chain (1+m or 1+m+o) is calculated on the assumption that the weight ratio of the polymer raw material to ethylene oxide in use during the addition reaction of the polyethylene glycol group is the same as the ratio of the polymer raw material to the number average molecular weight (Mn) of the polyethylene glycol group.

For example, in the terminally branched copolymer (T-5) obtained in Synthesis Example A5 of this Example A, since the weight ratio of the polymer raw material (I-1) to ethylene oxide in use is 1:1, Mn of the polymer raw material (I-1) is 1,223 and Mn of extended ethylene glycol unit also becomes 1,223. The total number of ethylene glycol units of the PEG chain (1+m+o) can be calculated by dividing this value by the molecular weight of the ethylene glycol unit.

Meanwhile, n, 1+m or 1+m+o can be measured by ¹H-NMR. For example, in the terminally branched copolymer (T-1) obtained in Synthesis Example A1 and particles in the dispersion system containing the copolymer (T-1), it can be calculated from the integral value for the methylene group of the polyolefin group A (shift value: 1.06 to 1.50 ppm) and the integral value for the alkylene group of PEG (shift value: 3.33 to 3.72 ppm) when the integral value for the methyl group at the terminal of the polyolefin group A in the general formula (1) (shift value: 0.88 ppm) is taken as the three-proton fraction.

Specifically, the number average molecular weight of the polyolefin group A and alkylene group can be calculated from the respective integral values from the facts that the molecular weight of the methyl group is 15, the molecular weight of the methylene group is 14, and the molecular weight of the alkylene group is 44. n can be calculated by dividing the number average molecular weight of the polyolefin group A obtained herein by the molecular weight of the ethylene unit, while the total number of the ethylene glycol unit of the PEG chain (1+m or 1+m+o) can be calculated by dividing the number average molecular weight of the alkylene group by the molecular weight of the ethylene glycol unit.

Similarly to the terminally branched copolymer (T-3) obtained in Synthesis Example A3 and particles in the dispersion system containing the copolymer (T-3), when the polyolefin group A is composed of an ethylene-propylene copolymer, n and 1+m or 1+m+o can be calculated by using both the content of propylene which can be measured by IR, ¹³C-NMR and the like, and the integral value in ¹H-NMR. In ¹H-NMR, a method of using an internal standard is also effective.

### Method for Preparing Terminally Branched Copolymer

The terminally branched copolymer can be prepared by the following methods.

First, among the target terminally branched copolymers, a polyolefin represented by the general formula (7) and having a double bond at one terminal is prepared as the polymer corresponding to the structure of A represented by the general formula (1), 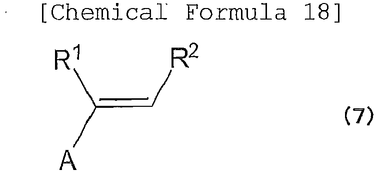 wherein, in the formula, A represents a group of an olefin having 2 to 20 carbon atoms, whose number average molecular weight is 400 to 8,000; and R¹ and R² each represent a hydrogen atom or an alkyl group having 1 to 18 carbon atoms, and at least one of R¹ and R² is a hydrogen atom.

This polyolefin may be prepared according to the following methods:
(1) A polymerization method of using a transition metal compound having a salicylaldimine ligand as described in Japanese Patent Laid-open No. 2000-239312, Japanese Patent Laid-open No. 2001-2731, Japanese Patent Laid-open No. 2003-73412 and the like, as the polymerization catalyst;
(2) A polymerization method of using a titanium based catalyst composed of a titanium compound and an organic aluminum compound;
(3) A polymerization method of using a vanadium based catalyst composed of a vanadium compound and an organic aluminum compound; and
(4) A polymerization method of using a Ziegler type catalyst composed of a metallocene compound such as zirconocene and an organic aluminum oxy compound (aluminoxane).

Among the aforementioned methods (1) to (4), particularly according to method (1), the aforementioned polyolefin can be prepared with good efficiency. In method (1), the aforementioned polyolefin having a double bond at one terminal can be prepared by polymerizing or copolymerizing the above-mentioned olefin in the presence of the aforementioned transition metal compound having a salicylaldimine ligand.

The polymerization of olefin according to method (1) can be carried out by either a liquid phase polymerization method such as solution polymerization or suspension polymerization, or a gas phase polymerization method. Detailed conditions and the like are already known, and the polyolefin can be prepared by referring to the above-described patent documents.

The molecular weight of the polyolefin obtained according to method (1) can be adjusted by adding hydrogen to the polymerization system, by varying the polymerization temperature, or by changing the kind of catalyst in use.

Subsequently, the polyolefin is epoxified, that is, the double bonds at the terminals of the polyolefin are oxidized, to obtain a polymer containing a terminal epoxy group as represented by the general formula (8), 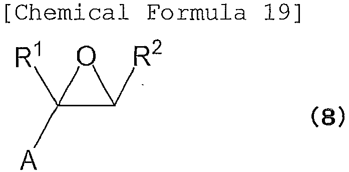 wherein, in the formula, A, R¹ and R² are the same as those described above.

The method for epoxidating a polyolefin is not particularly limited, but the following methods can be mentioned:
(1) Oxidation by peracid such as performic acid, peracetic acid, perbenzoic acid, or the like;
(2) Oxidation by titanosilicate and hydrogen peroxide;
(3) Oxidation by a rhenium oxide catalyst such as methyltrioxorhenium, and hydrogen peroxide;
(4) Oxidation by a porphyrin complex catalyst such as manganese porphyrin, iron porphyrin or the like, and hydrogen peroxide or hypochlorite;
(5) Oxidation by a salen complex such as manganese salen or the like, and hydrogen peroxide or hypochlorite;
(6) Oxidation by a TACN complex such as manganese triazacyclononane (TACN) complex or the like, and hydrogen peroxide; and
(7) Oxidation by hydrogen peroxide in the presence of a Group VI transition metal catalyst such as a tungsten compound or the like, and a phase transfer catalyst.

Among the methods (1) to (7), methods (1) and (7) are particularly preferred in view of activity.

Further, for example, an epoxy-terminated polymer having a low molecular weight Mw of about 400 to 600 that can be used is VIKOLOX™ (registered trademark, a product of Arkema Inc.).

It is possible to obtain a polymer (polymer (I)) in which various substituents Y¹ and Y² are introduced into α- and β- positions of the polymer end as represented by the general formula (9) by reacting various reaction reagents with the epoxy-terminated polymer represented by the general formula (8) obtained according to the aforementioned methods, 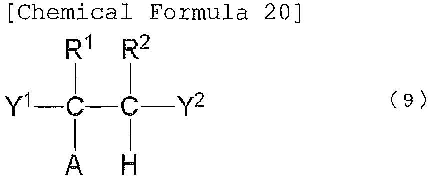 wherein, in the formula, A, R¹ and R² are the same as those described above; and Y¹ and Y² may be the same or different, and each represents a hydroxyl group, an amino group or the following general formulae (10a) to (10c),

[Chemical Formula 21] -E-R³-(T)ₘ (10a)

[Chemical Formula 22] -N-R³-(T)ₘ)₂ (10b)

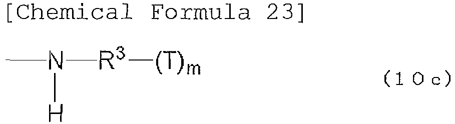

wherein, in the general formulae (10a) to (10c), E represents an oxygen atom or a sulfur atom; R³ represents an (m+1)-valent hydrocarbon group; T may be the same or different, and each represents a hydroxyl group or an amino group; and m represents an integer of 1 to 10.

For example, there is obtained a polymer in which, in the general formula (9), both of Y¹ and Y² are each a hydroxyl group by hydrolyzing the epoxy-terminated polymer represented by the general formula (8), while there is obtained a polymer in which one of Y¹ and Y² is an amino group and the other one is a hydroxyl group by reacting with ammonia.

Furthermore, there is obtained a polymer in which, in the general formula (9), one of Y¹ and Y² is a group represented by the general formula (10a) and the other one is a hydroxyl group by reacting the epoxy-terminated polymer represented by the general formula (8) with a reaction reagent A represented by the general formula (11a),

[Chemical Formula 24] HE-R³-(T)ₘ (1 1 a)

wherein, in the formula, E, R³, T and m are the same as those described above.

Furthermore, there is obtained a polymer in which, in the general formula (9), one of Y¹ and Y² is a group represented by the general formula (10b) or (10c) and the other one is a hydroxyl group by reacting the epoxy-terminated polymer with a reaction reagent B represented by the general formula (11b) or (11c),

[Chemical Formula 25] HN-R³-(T)ₘ)₂ (11b)

[Chemical Formula 26] H₂N-R³- (11c)

wherein, in the formula, R³, T and m are the same as those described above.

Examples of the reaction reagent A represented by the general formula (11a) include glycerin, pentaerythritol, butanetriol, dipentaerythritol, polypentaerythritol, dihydroxybenzene, trihydroxybenzene and the like.

Examples of the reaction reagent B represented by the general formula (11b) or (11c) include ethanolamine, diethanolamine, aminophenol, hexamethyleneimine, ethylenediamine, diaminopropane, diaminobutane, diethylenetriamine, N-(aminoethyl)propanediamine, iminobispropylamine, spermidine, spermine, triethylenetetraamine, polyethyleneimine and the like.

The addition reaction of an epoxy compound with alcohols, and amines is well known, and the reaction can be easily carried out according to a usual method.

The general formula (1) can be prepared by carrying out an addition polymerization of the alkylene oxide using the polymer (I) represented by the general formula (9) as a raw material. Examples of the alkylene oxide include propylene oxide, ethylene oxide, butylene oxide, styrene oxide, cyclohexene oxide, epichlorohydrin, epibromohydrin, methyl glycidyl ether, allyl glycidyl ether and the like. These may be used in combination of two or more kinds. Among these, preferably used are propylene oxide, ethylene oxide, butylene oxide and styrene oxide, and more preferably used are propylene oxide and ethylene oxide.

For the catalyst, polymerization conditions and the like, known ring-opening polymerization methods for alkylene oxide can be used, and examples of obtaining polyol by polymerizing various monomers are disclosed in "Revised Polymer Synthesis Chemistry," written by Otsu Takayuki, Kagaku-Dojin Publishing Company, Inc., January 1971, pp. 172-180. Examples of the catalyst used in the ring-opening polymerization include, as described in the above literature, Lewis acids such as AlCl₃, SbCl₅, BF₃ and FeCl₃ exclusively for cationic polymerization; hydroxides or alkoxides of alkali metals, amines and phosphazene catalysts exclusively for anionic polymerization; and oxides, carbonates and alkoxides of alkaline earth metals, or alkoxides of Al, Zn, Fe and the like exclusively for coordinate anionic polymerization. Here, the phosphazene catalysts may be exemplified by those compounds described in Japanese Patent Laid-open No. 1998-77289, specifically the products resulting from changing the anion of commercially available tetrakis[tris(dimethylamino)phosphoranilidenamino] phosphonium chloride into an alkoxy anion by using an alkali metal alkoxide.

When the reaction solvent is used, those inert to the polymer (I) and the alkylene oxide can be used, and examples thereof include n-hexane, alicyclic hydrocarbons such as cyclohexane and the like, aromatic hydrocarbons such as toluene, xylene and the like, ethers such as dioxane and the like, and halogenated hydrocarbons such as dichlorobenzene and the like.

The amount of the catalyst to be used for the catalysts other than phosphazene catalysts is preferably in the range of 0.05 to 5 moles, and more preferably in the range of 0.1 to 3 moles, based on 1 mole of the polymer (I) as a raw material. The amount of phosphazene catalyst to be used is preferably from 1 × 10⁻⁴ to 5 × 10⁻¹ moles and more preferably from 5 × 10⁻⁴ to 1 × 10⁻¹ moles, based on 1 mole of the polymer (I), from the viewoints of rate of polymerization, economic efficiency and the like.

The reaction temperature is usually from 25 to 180 degrees centigrade and preferably from 50 to 150 degrees centigrade, and although the reaction time varies depending on the reaction conditions such as the amount of catalyst in use, reaction temperature, reactivity of olefins and the like, it is usually from a few minutes to 50 hours.

The number average molecular weight of the general formula (1) can be calculated by a method of calculating it from the number average molecular weight of the polymer (I) represented by the general formula (8) as described above and the weight of the alkylene oxide to be polymerized, or a method of using NMR.

### Polymer Particles

The polymer particles composed of such a terminally branched copolymer of the present invention have a structure wherein the polyolefin chain portion represented by A in the general formula (1) is oriented in an internal direction, and are rigid particles in which this polyolefin chain portion has crystallinity.

The polymer particles of the present invention can be dispersed again in a liquid such as a solvent or the like even after particles are taken out by drying of the dispersion solution since the polyolefin chain portion thereof has crystallinity. The polymer particles of the present invention are rigid particles in which the melting point of the polyolefin chain portion contained in the particles is not less than 80 degrees centigrade and preferably not less than 90 degrees centigrade.

In Examples 52 and 53 of Patent Document 3, there has been disclosed a method of obtaining micelles having an average particle size of from 15 to 20 nm using this terminally branched copolymer. However, the method disclosed therein is a method of using the toluene soluble fraction in which the terminally branched copolymer is fractionated into a toluene soluble portion and a toluene insoluble portion, and the polyethylene chain portion of the terminally branched copolymer has a low molecular weight. Specifically, the terminally branched copolymer is melted under heating in the presence of toluene, and then a slurry liquid after cooling is separated by filtration and toluene is distilled off from the toluene solution and dried to obtain a polymer. The resultant polymer is mixed with water, stirred while boiling under the normal pressure, further stirred using ultrasonic waves and cooled to room temperature.

In polyethylene, there is a correlation between the molecular weight and the melting point such that the lower molecular weight indicates the lower melting point. Also, Example 52 and 53 of Patent Document 3 disclose that the melting point of the toluene insoluble portion is not less than 100 degrees centigrade, and the melting point of the toluene soluble portion is lower or higher than 70 degrees centigrade. Even though micelles disclosed in the Patent Document are cooled, it is possible to obtain particles obtained by crystallizing the polyethylene chain portion, whereas it is not possible to obtain rigid particles since the melting point is low for deteriorating crystallinity. Furthermore, there are some points to be improved, for example, micelles are easily obtained by heating, and particle properties are lost for easily disintegrating particles.

On the other hand, the polymer particles of the present invention are rigid particles with excellent crystallinity since the melting point of the polyolefin chain portion is in the above-described range, and disintegration of particles is suppressed even under heating at a higher temperature.

Accordingly, in the production process and use situations for various uses as described later, disintegration of particles is suppressed so that the yield of the products and quality of the products are more stabilized without losing characteristics of the polymer particles of the present invention.

Even when the polymer particles of the present invention are dispersed in a solvent or the like, the particle size is constant regardless of the dilute concentration. Namely, the polymer particles are different from micelle particles dispersed in a liquid because the polymer particles have redispersion properties and uniform dispersion particle size.

Incidentally, an average particle size of 50% by volume of the polymer particles of the present invention from 1 nm to 100 nm. The particle size of the polymer particles is measured using a dynamic light-scattering nanotrak particle size analyzer (Microtrack UPA-EX150, a product of Nikkiso Co., Ltd.). Specifically, the prepared dispersion is added dropwise to the analyzer so as to have an appropriate concentration and uniformly dispersed, and then average particle sizes of 10%, 50% and 90% by volume can be measured.

The polymer particles of the present invention can contain a prescribed substance (other dispersoids as described later), and can be attempted to be developed for various uses.

Hereinafter, the dispersion solution containing the polymer particles of the present invention, the composite, and uses thereof will be described.

### Dispersion Solution

The dispersion solution of the present invention contains the aforementioned terminally branched copolymer for a dispersoid, wherein the dispersoid is dispersed in water and/or an organic solvent having an affinity for water as particles.

In the present invention, the dispersion solution refers to a dispersion solution in which the terminally branched copolymer particles are dispersed, and which also contains any of the following:
(1) A dispersion solution containing the polymer particles obtained during production of the terminally branched copolymer particles;
(2) A dispersion solution obtained by further dispersing or dissolving other dispersoid, an additive or the like in the dispersion solution containing the polymer particles obtained during production of the terminally branched copolymer particles; or
(3) A dispersion solution obtained by dispersing the terminally branched copolymer particles in water or an organic solvent having an affinity for water, and at the same time dispersing or dissolving other dispersoid, an additive or the like.

The content of the aforementioned terminally branched copolymer in the dispersion solution of the present invention is preferably from 0.1 to 50 weight %, more preferably from 1 to 40 weight % and further preferably from 1 to 20 weight %.

When the content of the terminally branched copolymer is in the above range, it is preferable because practical properties of the dispersion solution are excellent, and its viscosity can be properly maintained, and the dispersion solution becomes easily handled.

Meanwhile, an average particle size of 50% by volume of the particle in the dispersion solution of the present invention is preferably from not less than 1 and not more than 1,000 nm, more preferably from not less than 1 and not more than 500 nm and further preferably fromnot less than 1 and not more than 100 nm.

The average particle size of 50% by volume of the particle can be adjusted by varying a structure of the polyolefin portion of the aforementioned terminally branched copolymer and a structure of the terminal branched portion.

Incidentally, the average particle size of 50% by volume in the present invention refers to a diameter of the particle at 50% of the cumulative volume when the total volume is 100%, and can be measured by using a dynamic light-scattering particle size measuring apparatus or a Microtrack particle size distribution measuring apparatus. Furthermore, the zeta potential can be measured by using a zeta potential measuring apparatus.

Besides, its shape can be observed, for example, using a transmission electron microscope after carrying out negative staining with phosphotungstic acid.

The dispersion solution in the present invention is obtained by dispersing the terminally branched copolymer in water and/or an organic solvent having an affinity for water.

Water is not particularly limited, and there can be used distilled water, ion exchange water, city water, water for industrial use and the like. Preferably used are distilled water and ion exchange water.

The organic solvent having an affinity for water is not particularly limited as long as the terminally branched copolymer can be dispersed therein, but examples thereof include ethylene glycol, tetraethylene glycol, isopropyl alcohol, acetone, acetonitrile, methanol, ethanol, dimethyl sulfoxide, dimethylformamide, dimethylimidazolidinone and the like.

Dispersion in the present invention can be carried out in a method of physically dispersing the terminally branched copolymer in water and/or an organic solvent having an affinity for water by the mechanical shearing force.

The dispersion method is not particularly limited, but various dispersion methods can be used. Specifically, there can be mentioned a method of dispersing the terminally branched copolymer with a high-pressure homogenizer, a high-pressure homomixer, an extrusion kneader, an autoclave or the like in a molten state after mixing the terminally branched copolymer represented by the general formula (1) and water and/or an organic solvent having an affinity for water, a method of jet grinding at a high pressure, and a method of spraying from a pore. Also, there can also be used a method of dispersing the terminally branched copolymer with a high-pressure homogenizer, a high-pressure homomixer or the like by mixing water and/or an organic solvent having an affinity for water after dissolving the aforementioned terminally branched copolymer in a solvent other than water in advance. At this time, a solvent used for dissolution of the terminally branched copolymer is not particularly limited as long as the terminally branched copolymer is dissolved, but examples thereof include toluene, cyclohexane, the aforementioned organic solvent having an affinity for water and the like. When it is not preferable that an organic solvent other than water is mixed into the dispersion solution, the organic solvent can be removed by distillation or the like.

Further specifically, for example, the dispersion solution can be obtained by heating it with stirring while applying a shearing force at a temperature of not less than 100 degrees centigrade and preferably from 120 to 200 degrees centigrade in an autoclave equipped with a stirrer capable of applying a shearing force.

When the temperature is within the above range, the aforementioned terminally branched copolymer is easily dispersed because the dispersion solution is in a molten state, and the aforementioned terminally branched copolymer is hardly deteriorated by heating; therefore, such a temperature is preferable.

The time required for dispersion is different depending on the dispersion temperature or other dispersion conditions, but it is about 1 to 300 minutes.

The dispersion can be fully carried out during the aforementioned stirring time, and the aforementioned terminally branched copolymer is hardly deteriorated; therefore, such time is preferable. After the reaction, it is preferable to maintain the state of the shearing force as applied until the temperature in the dispersion solution becomes not more than 100 degrees centigrade and preferably not more than 60 degrees centigrade.

In the production of the dispersion solution in the present invention, it is not essential to add a surfactant, but, for example, anionic surfactants, cationic surfactants, amphoteric surfactants, nonionic surfactants and the like may coexist.

Examples of the anionic surfactant include carboxylic acid salt, simple alkyl sulfonate, modified alkyl sulfonate, alkyl allyl sulfonate, alkyl sulfate ester salt, sulphonated oil, sulfuric acid ester, sulfonated fatty acid monoglyceride, sulphonated alkanol amide, sulphonated ether, alkyl phosphate ester salt, alkylbenzene phosphoric acid salt, naphthalenesulfonic acid-formalin condensate and the like.

Examples of the cationic surfactant include simple amine salt, modified amine salt, tetraalkyl quaternary ammonium salt, modified trialkyl quaternary ammonium salt, trialkylbenzyl quaternary ammonium salt, modified trialkylbenzyl quaternary ammonium salt, alkyl pyridinium salt, modified alkyl·pyridinium salt, alkyl quinolinium salt, alkyl phosphonium salt, alkyl sulfonium salt and the like.

Examples of the amphoteric surfactant include betaine, sulfobetaine, sulfate betaine and the like.

Examples of the nonionic surfactant include monoglycerin fatty acid ester, polyglycol fatty acid ester, sorbitan fatty acid ester, sucrose fatty acid ester, fatty acid alkanol amide, fatty acid polyethylene glycol condensate, fatty acid amide polyethylene glycol condensate, fatty acid alcohol polyethylene glycol condensate, fatty acid amine polyethylene glycol condensate, fatty acid mercaptan polyethylene glycol condensate, alkyl phenol polyethylene glycol condensate, polypropylene glycol polyethylene glycol condensate and the like.

These surfactants may be used singly or in combination of two or more kinds.

In the production of the dispersion solution of the present invention, for the purpose of removing foreign substances or the like, a filtration step during the process may be carried out. In such a case, for example, a stainless steel filter (wire diameter: 0.035 mm, plain weave) of about 300 meshes may be arranged and pressure filtration (air pressure: 0.2 MPa) may be carried out.

The dispersion solution to be obtained according to the above-described method does not cause aggregation and precipitation even though the pH varies from 1 to 13 by adding various acids or bases, for example, acids such as hydrochloric acid, sulfuric acid, phosphoric acid and the like, or bases such as potassium hydroxide, sodium hydroxide, calcium hydroxide and the like. Furthermore, this dispersion solution does not cause aggregation and precipitation even in a wide temperature range such that heating and refluxing or freezing and thawing under the normal pressure are repeatedly carried out.

Meanwhile, the dispersion solution in the present invention contains a dispersoid other than the aforementioned terminally branched copolymer in particles of the dispersion solution, and is capable of dispersing the dispersoid in a state that the dispersoid is encapsulated in particles. The dispersoid is not particularly limited, but examples thereof include compounds of thermoplastic resins, dyes, pigments, carbon black, carbon nanotube, fullerene, paints, fluorescent substances, luminescent substances, pharmaceutical drugs, radionuclides and the like. The dispersion solution containing the dispersoid can be used for various uses, for example, paint dispersing agents, paint additives, coating agents, carriers for enzymes, cosmetic base materials, drugs, diagnostic drugs, test drugs and the like.

A method in which those other than the aforementioned terminally branched copolymer are contained in the dispersion solution in the present invention as other dispersoid is not particularly limited, but examples thereof include a method of dispersing both dispersoids in the coexistence thereof by adding the aforementioned terminally branched copolymer and the dispersoid at the same time in the production of the dispersion solution.

The method of dispersing both dispersoids in the coexistence thereof is suitable for use in a dispersoid which is hardly dissolved in a compound having a high melting point such as thermoplastic resin or the like, and water and/or an organic solvent having an affinity for water at a room temperature.

In addition, for a dispersoid which is soluble in a compound unstable at a high temperature, and in water and/or an organic solvent having an affinity for water, suitably used is a method of adding the dispersoid dissolved in water and/or an organic solvent having an affinity for water to the dispersion solution.

Water in the above method is not particularly limited, and distilled water, ion exchange water, city water, water for industrial use and the like can be used. However, preferably used are distilled water and ion exchange water.

Meanwhile, the organic solvent having an affinity for water in the above method is not particularly limited as long as the dispersoid is soluble, but examples thereof include ethylene glycol, tetraethylene glycol, isopropyl alcohol, acetone, acetonitrile, methanol, ethanol, dimethyl sulfoxide, dimethylformamide, dimethylimidazolidinone and the like. When mixing of the organic solvent into the dispersion solution is not desired, the aforementioned organic solvent can be removed by distillation or the like after the preparation of the dispersion solution containing the dispersoid.

For the dispersion solution of the present invention, when the aforementioned terminally branched copolymer is contained in an amount of 100 weight parts, the dispersoid can be contained in an amount of 0.001 weight parts to 20 weight parts, preferably in an amount of 0.01 weight parts to 10 weight parts and further preferably in an amount of 0.1 weight parts to 5 weight parts.

When the content of the dispersoid is in the above range, it is preferable because physical properties of the dispersion solution are excellent in the practical point of view, and the dispersion solution hardly causes aggregation and precipitation.

Particles in the dispersion solution can be taken out from the dispersion solution in the present invention. Particles can be taken out by drying or the like. A freeze dryer is preferably used for drying, and further a spray dryer, a rotary dryer, a flash dryer, an agitator dryer and the like can also be used.

For example, in case of performing freeze drying, the dispersion solution of the present invention is fully frozen by using a cooling agent such as liquid nitrogen or the like beforehand, and then drying under reduced pressure may be performed by using a commercial freeze dryer. In case of spray drying, if an inlet temperature is adjusted at 100 degrees to 150 degrees centigrade and preferably at 110 degrees to 130 degrees centigrade, and an outlet temperature is adjusted at 20 degrees to 80 degrees centigrade and preferably at 40 degrees to 60, drying can be performed with good efficiency while suppressing aggregation of particles in the dispersion solution.

When a dispersoid other than the terminally branched copolymer is contained in the dispersion solution, other dispersoid can be encapsulated in particles composed of the obtained terminally branched copolymer.

The taken-out particles can produce a dispersion solution again by stirring with a magnetic stirrer, a three-one motor or the like, or an irradiation with ultrasonic waves after addition of water and/or an organic solvent having an affinity for water, thereby without using an autoclave or the like.

Water in the above method is not particularly limited, and there can be used distilled water, ion exchange water, city water, water for industrial use and the like. Preferably used are distilled water and ion exchange water.

Furthermore, the organic solvent having an affinity for water in the above method is not particularly limited as long as the aforementioned terminally branched copolymer can be dispersed therein, and examples thereof include ethylene glycol, tetraethylene glycol, isopropyl alcohol, acetone, acetonitrile, methanol, ethanol, dimethyl sulfoxide, dimethylformamide, dimethylimidazolidinone and the like.

The dispersion solution in the present invention is, in general, highly transparent and highly glossy, is excellent in lubricability including anti-static properties, anti-fogging properties, water resistance, gas barrier properties, rub resistance and antisticking properties, and can be imparted with the aforementioned properties by use.

Furthermore, the dispersion solution in the present invention can be used for the expected use of the dispersion solution having a small average particle size of 50% by volume, for example, for the use of inkjet, from the fact that the average particle size of 50% by volume is small.

As described above, the dispersion solution in the present invention does not cause aggregation and precipitation in a wide range of pH and temperature so that it can be used under various environments.

Incidentally, in the dispersion solution of the present invention, it is possible to properly combine with an additive, for example, a plasticizer, a defoaming agent, a leveling agent, an anti-mold agent, a rust prevention agent, a matting agent, a flame retardant, a thixotropic agent, a tacking agent, a thickening agent, a lubricant, an anti-static agent, a surfactant, a reaction retardant, an anti-oxidant, a UV absorbent, a hydrolysis inhibitor, a weather resistant stabilizer, an anti-tacking agent or the like, in the ranges in which the excellent effect of the present invention is not impaired. The proportion of various additives is properly selected according to purposes and uses.

The dispersion solution of the present invention can be used for an ink composition.

The ink composition in the present invention is a water based printing ink composition containing the dispersion solution of the present invention, and is prepared according to a known method by adding a coloring agent such as pigment or the like, and as necessary an additive such as filler or the like, in addition to a vehicle component, fatty acid or fatty acid salt. Dispersing an oil-soluble coloring agent using the dispersion solution of the present invention enables also using for a coloring agent which could not be used in a conventional water based printing ink composition.

Meanwhile, a printed matter can be obtained by printing the water based printing ink composition of the present invention on a known printing material such as plastic, paper or the like by a gravure printing method or a flexographic printing method.

As an aqueous resin to be used as a vehicle resin of the ink composition, there are mentioned water-soluble or water-dispersible resins such as an emulsion, a dispersion and the like to be used for inks, paints, recording agents and the like in general. These resins may be used singly or as a mixture thereof.

Specific examples thereof include polyurethane resin, polyurethane urea resin, acryl-modified urethane resin, acryl-modified urethane urea resin, acrylic resin, styrene-acrylic acid copolymer resin, styrene-maleic acid copolymer resin, ethylene-acrylic acid copolymer resin, polyester resin, shellac, rosin-modified maleic acid resin, vinyl chloride-vinyl acetate copolymer resin, vinyl chloride-acrylic acid copolymer resin, chlorinated polypropylene resin, hydroxyethyl cellulose, hydroxypropyl cellulose, butyral and the like. These may be used singly or in combination of two or more kinds.

The water based printing ink composition of the present invention may be used, for example, for inkjet paints, protective paints, various lubricant paints for stainless steels, various overprint varnishes for printed matters, overcoat varnishes for thermal paper, paints for floor polishing, paints for building construction, paints for vehicle bodies and the like.

Meanwhile, the dispersion solution of the present invention can also be used for a coating agent.

The coating agent in the present invention is an aqueous coating agent containing the dispersion solution of the present invention, and can form a laminate by applying it to a resin, metal, paper, wood, fiber, leather, glass, rubber and the like.

Specific examples of the resin include polyolefinic resin such as polyethylene, polypropylene and the like; polyester resin such as polyethylene terephthalate, polybutylene terephthalate, polyethylene-2,6-naphthalate and the like; polyamide resin such as nylon 6, nylon 12 and the like; ethylene-vinyl acetate copolymer resin or a saponifiable material thereof; polystyrene resin; polycarbonate resin; polysulfone resin; polyphenylene oxide resin; polyphenylene sulfide resin; aromatic polyamide resin; polyimide resin; polyamideimide resin; cellulose resin; acetate cellulose resin; polyvinyl alcohol resin and the like, and a copolymer thereof.

The aforementioned coating agent can also be used for a base film and sheet having the aforementioned resin as a component. The base material in use and a method of molding the base material are not particularly limited, but examples thereof include biaxially stretched polyethylene terephthalate resin film and sheet, extrusion-molded polypropylene resin film and sheet, biaxially stretched polypropylene resin film and sheet, and the like.

For example, extrusion-molded film and sheet of a polypropylene based resin can be obtained by melt-extruding a pellet using an extruder and a circular die, and extruding the pellet using a coat-hanger die and a T-die, followed by cooling and molding. The molding conditions are not particularly limited, but the molding temperature is from 190 to 280 degrees centigrade and the cooling temperature of a chill roll is preferably from 10 to 80 degrees centigrade.

Stretched film and sheet of a polypropylene based resin can be prepared by a method for preparing biaxially stretched film and sheet, such as, a known simultaneous biaxial stretching method, a sequential biaxial stretching method or the like. In the biaxial stretching conditions, the preparation conditions of known OPP film and sheet, for example, a sequential biaxial stretching method, the longitudinal stretching temperature may be from 125 to 145 degrees centigrade, the stretch ratio may be in the range of 4.5 to 9, the vertical stretching temperature may be from 165 to 190 degrees centigrade, and the stretch ratio may be in the range of 7 to 11.

The base film and sheet may be subjected to, as necessary, plasma treatment, tackiness improving treatment, corona treatment, anti-static treatment or the like.

Examples of the plasma treatment include a normal pressure plasma treatment, a low pressure (vacuum) plasma treatment and the like. Herein, the normal pressure plasma treatment will be exemplified. The term normal pressure plasma treatment refers to a process of causing electrolytic dissociation of a gas for activating it in the discharge plasma state by applying an electric field using a high frequency to the gas such as nitrogen or the like under the normal pressure, and then causing the particles of the gas brought into the plasma state to collide against the surface of the base material. Accordingly, since the molecular bond on the surface of the base material is decomposed and an OH group or the like is formed on the surface of the base material, the surface of the base material can be hydrophilized and/or a concave and convex structure at the molecular level can be fixed on the surface of the base material. In general, it is known that the film and sheet base materials are subjected to this treatment, whereby wetting properties or adhesion properties of the surface are improved. For the normal pressure plasma treatment, any kinds of gases such as air, argon, nitrogen, oxygen and the like can be fundamentally used. As a method of the normal pressure plasma treatment, there are a method of passing the base material to discharge space for directly subjecting the surface of the base material to plasma treatment, or a method of blowing the active species from the discharge space for subjecting the surface of the base material to plasma treatment. It is preferable to apply the former treatment method in view of uniform surface treatment of the base material. The frequency of a power supply used for discharge plasma is different depending on the quality of the material or the thickness of the object to be treated, but in the present invention, it is from 50 to 30,000,000 Hz, preferably from 1,000 to 50,000 Hz and further preferably from 5,000 to 40, 000 Hz.

As the tackiness improving treatment, base film and sheet obtained by applying primer thereto beforehand is exemplified. Examples of the primer include compositions obtained by mixing a composition having active hydrogen and/or a hydroxyl group with a curing agent having an isocyanate group or the like capable of reacting with active hydrogen and/or a hydroxyl group.

Examples of the aforementioned composition having active hydrogen and/or a hydroxyl group include TAKELAC series (products of Mitsui Chemicals Polyurethanes Inc.) and the like, and examples of the curing agent include TAKENATE series (products of Mitsui Chemicals Polyurethanes Inc.), ELASTRON BN series (products of Dai-ichi Kogyo Seiyaku Co., Ltd.) and the like.

The coating agent of the present invention may contain a binder resin or a crosslinking agent. The binder resin and the crosslinking agent are not particularly limited, but examples of the binder resin include urethane emulsion and acrylic emulsion, and examples of the crosslinking agent include an isocyanate crosslinking agent, an epoxy crosslinking agent and a melamine crosslinking agent.

A method of applying the aforementioned coating agent is not particularly limited, but examples thereof include hard coating, spray coating, curtain coating, flow coating, roll coating, gravure coating, brush coating, dip coating and the like.

The aforementioned coating agent can be used, for example, for an anti-static agent, an anti-fogging agent, an overprint varnish, a protective coating agent and the like.

The dispersion solution of the present invention can also be used for a cosmetic preparation.

The cosmetic preparation in the present invention can be used for the preparation of cream, ointment, lotion, gel and the like exclusively for cosmetic and medical fields, and is excellent in long-term stability at a high temperature, storage stability in a wide range of pH, and dispersion properties of the dispersoid to be contained.

The dispersion solution of the present invention can be used for other uses such as an emulsifying agent, a thickening agent, a strengthening agent for paper, a surfactant, a skin care product (cleanser, cosmetics, emulsion, cream, foundation), a hair care product (shampoo, rinse, hold hair spray), an agricultural-chemical preparation, a medical drug (drug delivery), a dispersing agent (pigment, paint, carbon black, carbon nanotube, fullerene), soap, a soil improvement agent, a lubricating agent, a cement dispersing agent, an asphalt modifier, an adhesive, a surface-treating agent, a toner composition, a carrier for enzyme, a mold release agent, a slipping agent, a resin modifier, a compatibilizing agent, a paper quality improver (blocking resistance, wear resistance, moisture resistance, luster, surface hardness), a fiber process aid (flexibility, lubricity) and the like.

The composite containing polymer particles of the present invention will be illustrated below.

### Composite

The composite of the present invention has a structure in which the above-described polymer particles are dispersed in a matrix composed of a metal oxide. This composite is prepared from the mixed composition. First, the mixed composition will be illustrated.

### Method for Preparing Mixed Composition

The mixed composition which is used in the present invention is prepared, for example, by mixing the dispersion solution of the present invention obtained by dispersing polymer particles (A) (aqueous dispersion solution) and metal alkoxide and/or a hydrolysis condensate of metal alkoxide (B).

Specifically, the mixed composition is prepared by adding a catalyst to be used for the sol-gel reaction (D), and further, as necessary, water to the component (B) or a solution obtained by dissolving the component (B) in water and/or a solvent for dissolving a part of water or entire water in any proportions (C) for stirring and mixing, and then adding the aqueous dispersion solution of polymer particles (A) for stirring and mixing.

Further, the mixed composition is prepared by adding the aqueous dispersion solution of polymer particles (A) to the component (B) or a solution obtained by dissolving the component (B) in the aforementioned solvent (C) for stirring and mixing, and then adding a catalyst, and further, as necessary, water for stirring and mixing.

Incidentally, as described above, it is possible to obtain the aqueous dispersion solution containing polymer particles by dispersing the terminally branched copolymer in the solvent (C), but it is possible to obtain the aqueous dispersion solution containing polymer particles by dispersing the taken-out polymer particles in the solvent (C).

### Metal Alkoxide and/or Hydrolysis Condensate thereof (B)

The metal alkoxide in the present invention indicates those represented by the following formula (12),

(R1) x M(OR2)y (12)

wherein, in the formula, R1 represents a hydrogen atom, an alkyl group (methyl group, ethyl group, propyl group and the like), an aryl group (phenyl group, tolyl group and the like), a carbon-carbon double bond-containing organic group (acryloyl group, methacryloyl group, vinyl group and the like), a halogen-containing group (halogenated alkyl group such as chloropropyl group, fluoromethyl group or the like) and the like; R2 represents a lower alkyl group having 1 to 6 carbon atoms and preferably having 1 to 4 carbon atoms; and in x and y, x + y is 4 and x represents an integer of not more than 2.

Examples of M include Li, Na, Mg, Al, Si, K, Ca, Sc, Ti, V, Cr, Mn, Fe, Co, Ni, Cu, Zn, Rb, Sr, Y, Nb, Zr, Mo, Ag, Cd, In, Sn, Sb, Cs, Ba, La, Ta, Hf, W, Ir, Tl, Pb, Bi, rare earth metal and the like, and preferably used are metals (alkoxide) to be colorless metal oxides in the sol-gel reaction, such as, Si, Al, Zn, Zr, In, Sn, Ti, Pb, Hf and the like from the viewpoint of use as a coating film. Of the metals, preferably used are silicon (Si), aluminum (Al), zirconium (Zr), titanium (Ti) and the like, or these metals may be used in combination. Among these, a silicon compound is relatively low-priced and easily obtainable, and has high industrial usefulness since the reaction slowly proceeds. Also, the metal alkoxide and/or a hydrolysis condensate thereof (B) (hereinafter referred to as the component (B)) may be a compound composed of metal oxide as described below by addition of water and a catalyst for carrying out the sol-gel reaction.

Concrete examples include alkoxysilanes such as tetramethoxysilane (TMOS), tetraethoxysilane (TEOS), tetrapropoxysilane, tetraisopropoxysilane, methyltrimethoxysilane, methyltriethoxysilane, methyltripropoxysilane, methyltributoxysilane, ethyltrimethoxysilane, ethyltriethoxysilane, n-propyltrimethoxysilane, n-propyltriethoxysilane, isopropyltrimethoxysilane, isopropyltriethoxysilane, dimethyldimethoxysilane, dimethyldiethoxysilane, diphenyldimethoxysilane, diphenyldiethoxysilane, vinyltrimethoxysilane, vinyltriethoxysilane, phenyltrimethoxysilane, phenyltriethoxysilane, p-styryltrimethoxysilane, 3-methacryloxypropylmethyldimethoxysilane, 3-methacryloxypropylmethyldiethoxysilane, 3-methacryloxypropyltrimethoxysilane, 3-methacryloxypropyltriethoxysilane, 3-acryloxypropyltrimethoxysilane, 3-acryloxypropyltriethoxysilane, 3-chloropropyltriethoxysilane, trifluoromethyltrimethoxysilane, trifluoromethyltriethoxysilane and the like, and alkoxy aluminum, alkoxy zirconium and alkoxy titanium corresponding to these.

Furthermore, in addition to these metal alkoxides, metal alkoxide having various functional groups can also be used for R1 as shown in the following 1) to 4) in order to introduce an interaction such as hydrogen bond, ionic bond, covalent bond or compatibility between a water-soluble polymer and metal oxide to be added for the purposes of improving gas barrier properties and hard coat properties as described below.

1) Compounds having an amino group and an alkoxysilyl group such as 3-aminopropyltrimethoxysilane, 3-aminopropyltriethoxysilane, 3-aminopropylmethyldimethoxysilane, 3-aminopropylmethyldiethoxysilane, N-2-(aminoethyl)-3-aminopropylmethyldimethoxysilane, N-2-(aminoethyl)-3-aminopropyltrimethoxysilane, 2-aminoethylaminomethyltrimethoxysilane, 3-aminopropyldimethylethoxysilane, 2-(2-aminoethylthioethyl)triethoxysilane, p-aminophenyltrimethoxysilane, N-phenyl-3-aminopropylmethyldimethoxysilane, N-phenyl-3-aminopropylmethyldiethoxysilane, N-phenyl-3-aminopropyltrimethoxysilane, N-phenyl-3-aminopropyltriethoxysilane and the like;
2) Compounds having a glycidyl group and an alkoxysilyl group such as 3-glycidoxypropylpropyltrimethoxysilane, 3-glycidoxypropylpropyltriethoxysilane, 3-glycidoxypropylmethyldiethoxysilane and the like;
3) Compounds having a thiol group and an alkoxysilyl group such as 3-mercaptopropylmethyldimethoxysilane, 3-mercaptopropyltrimethoxysilane and the like; and
4) Compounds having a ureide group and an alkoxysilyl group such as 3-ureidepropyltrimethoxysilane and the like.

In the present invention, as metal alkoxide, in the above formula (12), preferably used are alkoxy silane in which M is silicon (Si), alkoxy zirconium in which M is zirconium (Zr), alkoxy aluminum in which M is aluminum (Al) and alkoxy titanium in which M is titanium (Ti).

As the gas barrier properties of a coating film layer (II), preferably used are tetrafunctional metal alkoxide such as tetramethoxysilane, tetraethoxysilane and the like because the structure of the metal oxide to be generated becomes dense when the number of alkoxy groups is high, thus the gas barrier properties tends to be improved. Further, these metal alkoxides may be used singly or as a mixture of two or more kinds.

The hydrolysis condensate of metal alkoxide is obtained by hydrolysis of one or more of these metal alkoxides using the catalyst to be used for the sol-gel reaction (D), and polycondensation of the resulting hydrolysate. The hydrolysis condensate of metal alkoxide is for example, a hydrolysis polycondensation compound of metal alkoxide.

In the present invention, as the hydrolysis condensate, preferably used are condensates of alkoxysilane, condensates of alkoxy zirconium, condensates of alkoxy aluminum, and condensates of alkoxy titanium.

### Water and/or Solvent for Dissolving Part of Water or Entire Water in Any Proportions (C)

In the composition of the present invention, the component (C) is added for the purpose of hydrolysis of metal alkoxide in metal alkoxide and/or a hydrolysis condensate thereof (B).

Meanwhile, the component (C) contains both a solvent to be used to obtain an aqueous dispersion solution by using the terminally branched copolymer, and a solvent to be used for mixture of the aqueous dispersion solution, the component (B) and the catalyst to be used for the sol-gel reaction (D) (hereinafter referred to as the component (D)) as described below.

Water is not particularly limited, and there can be used distilled water, ion exchange water, city water, water for industrial use and the like. However, preferably used are distilled water and ion exchange water.

The solvent for dissolving a part of water or entire water in any proportions is an organic solvent having an affinity for water, and is not particularly limited as long as a polyolefin based terminally branched copolymer can be dispersed therein. Examples thereof include methanol, ethanol, propyl alcohol, isopropyl alcohol, acetone, acetonitrile, dimethyl sulfoxide, dimethylformamide, dimethylimidazolidinone, ethylene glycol, tetraethylene glycol, dimethylacetamide, N-methyl-2-pyrrolidone, tetrahydrofuran, dioxane, methyl ethyl ketone, cyclohexanone, cyclopentanone, 2-methoxyethanol (methyl cellosolve), 2-ethoxyethanol (ethyl cellosolve), ethyl acetate and the like. Among these, preferably used are methanol, ethanol, propyl alcohol, isopropyl alcohol, acetonitrile, dimethyl sulfoxide, dimethylformamide, acetone, tetrahydrofuran and dioxane because they have a high affinity for water.

When water is used, the amount of water to be added is usually, for example, in the range of 1 weight part to 1,000,000 weight parts and preferably in the range of 10 weight parts to 10,000 weight parts, based on 100 weight parts of the mixture of the aforementioned components (B) and (D).

As the solvent for dissolving a part of water or entire water in any proportions, the amount of the solvent to be added is usually, for example, in the range of 1 weight part to 1,000,000 weight parts and preferably in the range of 10 weight parts to 10,000 weight parts, based on 100 weight parts of the mixture of the aforementioned components (B) and (D).

Furthermore, at the time of hydrolysis polycondensation of metal alkoxides, the reaction temperature is preferably from 1 degree centigrade to 100 degrees centigrade and more preferably from 20 degrees centigrade to 60 degrees centigrade, while the reaction time is preferably from 10 minutes to 72 hours and more preferably from 1 hour to 24 hours.

### Catalyst to be used for Sol-Gel Reaction (D)

The composition of the present invention may contain a material described in the following, which can act as a catalyst for the hydrolysis polymerization reaction, for the purpose of promoting the reaction in a hydrolysis polycondensation reaction of metal alkoxide.

Those used as the catalyst for a hydrolysis polycondensation reaction of metal alkoxide are the catalysts used in general sol-gel reactions, which are described in "Recent Technology for Functional Thin Film Production According to Sol-Gel Method" (Hirashima, Hiroshi, Comprehensive Technology Center Co., Ltd., p. 29), "Science of Sol-Gel Method" (Sakka, Sumio, Agne Shofu, p. 154), or the like.

Examples of the catalyst (D) include an acid catalyst, an alkali catalyst, an organic tin compound, and metal alkoxide such as titanium tetraisopropoxide, diisopropoxytitanium bis(acetylacetonate), zirconium tetrabutoxide, zirconium tetrakis(acetylacetonate), aluminum triisopropoxide, aluminum trisethylacetonate, trimethoxyborane and the like.

Among these catalysts, suitably used are an acid catalyst and an alkali catalyst. Specific examples of the acid catalyst include inorganic and organic acids such as hydrochloric acid, nitric acid, sulfuric acid, phosphoric acid, acetic acid, oxalic acid, tartaric acid, toluenesulfonic acid and the like. Examples of the alkali catalyst include alkali metal hydroxides such as ammonium hydroxide, potassium hydroxide, sodium hydroxide and the like; quaternary ammonium hydroxides such as tetramethylammonium hydroxide, tetraethylammonium hydroxide, tetrabutylammonium hydroxide and the like; amines such as ammonia, triethylamine, tributylamine, morpholine, pyridine, piperidine, ethylenediamine, diethylenetriamine, ethanolamine, diethanolamine, triethanolamine and the like; and aminosilanes such as 3-aminopropyltriethoxysilane, N-(2-aminoethyl)-3-aminopropyltrimethoxysilane and the like.

From the viewpoint of the reactivity, it is preferable to use acid catalysts such as hydrochloric acid, nitric acid and the like, with which the reaction proceeds relatively mildly. The preferred amount of the catalyst to be used is from about 0.001 moles to 0.05 moles, preferably from about 0.001 moles to 0.04 moles and further preferably from about 0.001 moles to 0.03 moles, based on 1 mole of the metal alkoxide of the aforementioned component (B).

### Sol-Gel Reactant

The mixed composition of the present invention can also be used, for example, (a) in the form of a sol-gel reactant obtained by applying the mixed composition to a base material for heating the resultant for a prescribed time to remove the solvent (C) and subjecting the mixed composition to the sol-gel reaction. Or, it can also be used, for example, (b) in the form of a sol-gel reactant obtained by subjecting the mixed composition to the sol-gel reaction in the presence of the catalyst (D) without removing the solvent (C) before applying the mixed composition to a base material. Furthermore, it can also be used (c) in the form of a sol-gel reactant obtained by applying a sol-gel reactant obtained by the sol-gel reaction without removing the aforementioned solvent (C) to a base material for heating the resultant for a prescribed time to remove the solvent (C), and further subjecting the mixed composition to the sol-gel reaction.

The metal oxide is obtained from the component (B) by this sol-gel reaction to form a matrix composed mainly of this metal oxide. This matrix is constructed such that polymer microparticles composed of the terminally branched copolymer are dispersed.

### Metal Oxide

The metal oxide in the sol-gel reactant is contained as a structure body in which it is dispersed or aggregated in the form of microparticles in the composition, or becomes a continuous structure body as a matrix of the composition. Metal oxide is not particularly limited as described above, but as a coating film, it is better to be a continuous matrix structure body for the metal oxide in view of improvement of gas barrier properties, hard coat properties and the like, and such a structure body of metal oxide is obtained by subjecting metal alkoxide to hydrolysis and/or hydrolysis and polycondensation, that is, by the sol-gel reaction.

### Terminally Branched Copolymer Microparticles

The average particle size of microparticles to be dispersed in a matrix formed from the aforementioned metal oxide is preferably in the range of 1 nm to 1,000 nm and further preferably in the range of 1 nm to 500 nm from the viewpoints of transparency, gas barrier properties and hard coat properties in case of using it as a coating film.

Meanwhile, in the sol-gel reactant, when microparticles of the aforementioned terminally branched copolymer are contained in an amount of 100 weight parts, metal oxide can be contained in an amount of 10 weight parts to 1,000 weight parts, preferably in an amount of 20 weight parts to 950 weight parts and further preferably in an amount of 30 weight parts to 900 weight parts.

When the content of microparticles is within the above range, the composition is excellent in physical properties such as water resistance, drug resistance, film formability, strength, heat resistance and the like, and is excellent in gas barrier properties and hard coat properties in case of using it as a coating film.

### Composition of Mixed Composition

The mixed composition to be used for coating in the present invention is a composition containing an aqueous dispersion solution obtained by dispersing at least the polymer particles (A) composed of the terminally branched copolymer in the solvent (C), and metal alkoxide and/or a hydrolysis condensate thereof (B). Further, a water-soluble polymer and/or saccharides may be added to the composition to the extent that water resistance and hard coat properties are not impaired as described above. Combination of these components will be illustrated hereinafter.

### Water Soluble Polymer

The water-soluble polymer to be used for the present invention is one or more compounds, and examples thereof include polymers having a hydroxyl group such as polyvinyl alcohol, poly 2-hydroxyethyl methacrylate and the like; polymers having a carboxyl group such as polyacrylic acid, carboxymethyl cellulose and the like; polymers having an amino group such as polyallylamine, polyethyleneimine and the like; polymers having an amide group such as polyacrylamide, poly N,N-dimethyacrylamide, poly N-isopropylacrylamide and the like; polymers having a sulfonic acid group such as polystyrene sulfonic acid, polyvinylsulfonic acid and the like; polymers having a polyether group such as polyethylene oxide, polyethylene glycol and the like; and polyvinyl pyrrolidone, polyoxazoline and the like. Among the aforementioned water-soluble polymers, preferably used are polymers having a hydrogen bond group because they can be uniformly made into a composite with inorganic oxide, and particularly preferably used are water-soluble polymers having a hydroxyl group, polyamides, polyethers, polyvinyl pyrrolidone, polyoxazoline and the like. Besides, in order to make an excellent and strong coating film for film formation as a coating layer of a gas barrier material, preferably used are polymers having a hydroxyl group. The water-soluble polymer having a hydroxyl group to be used for the present invention is a polymer containing at least two hydroxyl groups in the polymer chain and exhibiting water-solubility, and preferably one or more compounds selected from the group consisting of polyvinyl alcohol, copolymers containing vinyl alcohol units such as ethylene-vinyl alcohol copolymer and the like. These compounds may be used singly or in combination of two or more kinds.

Polyvinyl alcohol may be a homopolymer of vinyl alcohol or may be a copolymer containing other monomer units. It is preferable that the degree of saponification is close to 100% from the viewpoint of gas barrier properties, but it is usually not less than 90% and preferably not less than 95%. The number average polymerization degree is usually from not less than 50 and not more than 5,000.

In the present invention, much higher gas barrier properties are exhibited by adding the above-described water-soluble polymer to the polymer particles (A) and component (B). The reason is not necessarily clear, but it is considered that the water-soluble polymer is not compatible with a non-water-soluble resin, and can be uniformly made into a composite with a metal oxide so that the water-soluble polymer is hybridized with a metal oxide in the coating film layer to form a continuous matrix structure. It is considered that the hybrid obtained as a composite of such a metal oxide and the water-soluble polymer exhibits high barrier properties from the viewpoint of its effect of suppressing generation of defects in the process of film formation.

In the present invention, saccharides can also be used. Such sacchaqrides may be dissolved in a mixed solvent (aqueous medium) of water and/or an organic solvent having an affinity for water, or may be obtained by being uniformly dispersed and mixed with the aforementioned metal oxide for forming a dry film, and monosaccharides, oligosaccharides and polysaccharides may also be used. Monosaccharides, oligosaccharides and polysaccharides may be used each individually or in combination of two or more kinds.

Specific examples of the monosaccharide include aldose, glyceraldehyde, erythrose, threose, ribose, lyxose, xylose, arabinose, allose, talose, gulose, glucose, altrose, mannose, galactose, idose, dihydroxyacetone, erythrulose, xylulose, ribulose, psicose, fructose, sorbose, tagatose and the like.

Specific examples of the disaccharide include sucrose, maltose, isomaltose, cellobiose, lactose, trehalose and the like; examples of the trisaccharide include raffinose, panose, melezitose, gentianose and the like; examples of the tetrasaccharide include stachyose and the like; and examples of the oligosaccharide include cyclodextrin and the like.

Specific examples of the polysaccharide include starchs such as starch, oxidized starch, esterified starch and the like and starch derivatives; cellulose derivatives such as hemicellulose, methylcellulose, carboxymethyl cellulose, hydroxypropyl cellulose and the like; glycogen, pectin, pectic acid, alginic acid, carrageenin, agarose, partially deacetylated chitin, polyuronic acids and the like. Furthermore, these saccharides may be used each individually or in combination of two or more kinds.

Much higher gas barrier properties are exhibited by adding these saccharides to the mixed composition of the present invention. The reason is not necessarily clear, but it is considered that saccharides are not compatible with a non-water-soluble resin, and can be uniformly made into a composite with a metal oxide so that a continuous matrix structure is formed by hybrid configuration with a metal oxide in the coating film layer. It is considered that the hybrid obtained as a composite of such a saccharide and a water-soluble polymer exhibits high barrier properties from the viewpoint of its effect of suppressing generation of defects in the process of film formation.

As the weight ratio of the terminally branched copolymer constituting the polymer particles (A) contained in the aforementioned aqueous dispersion solution to the aforementioned metal alkoxide and/or a hydrolysis condensate thereof (B), the content of the component (B) is from 10 to 3,500 weight parts, based on 100 weight parts of the terminally branched copolymer. As described later, for the purpose of forming a continuous phase (matrix) formed by the metal oxide, it is better to increase the proportion of the component (B), and the content of the component (B) is preferably from 20 to 3,500 weight parts and further preferably from 30 to 3, 500 weight parts, based on 100 weight parts of the terminally branched copolymer.

Meanwhile, in order to generally enhance gas barrier properties and hard coat properties, it is better to increase the proportion of the metal oxide, but when the thickness of the coating film is thick, defects such as cracks and the like may occur in the process of formation of the coating film in some cases. For example, in order to form a coating film having not less than 1 µm, as the weight ratio of the terminally branched copolymer to the aforementioned component (B), the content of the component (B) is preferably from 10 to 2,500 weight parts and further preferably from 10 to 1,800 weight parts, based on 100 weight parts of the terminally branched copolymer.

The content of the water-soluble polymer to be added to the extent that water resistance and hard coat properties are not impaired is from 0.5 to 100 weight parts and preferably from 1 to 50 weight parts, based on the total 100 weight parts of the terminally branched copolymer and the aforementioned component (B).

### Other Components

The mixed composition in the present invention may further contain other components as follows.

### Metal Oxide Microparticles

The mixed composition may further contain metal oxide microparticles. Abrasion resistance of a hard coating material composed of a composite is improved by adding the metal oxide. The metal oxide microparticles refer to the oxide microparticles composed of at least one or more elements selected from silicon, aluminum, zirconium, titanium, indium, tin, zinc and antimony. The average particle size according to the BET method is not less than 1 nm in view of further enhancing abrasion resistance, and preferably not more than 100 nm in view of further enhancing transparency. Specific examples of the metal oxide microparticles include the following.

Silica microparticles are available from Nissan Chemical Industries, Ltd., under the trade name of Methanol Silica Sol, MA-ST-MA, MEK-ST, MIBK-ST, IPA-ST, IPA-ST-UP, IPA-ST-MS, IPA-ST-L, IPA-ST-ZL, NPC-ST-30, NBA-ST, XBA-ST, EG-ST, DMAC-ST, ST-20, ST-30, ST-40, ST-C, ST-N, ST-O, ST-S, ST-50, ST-20L, ST-OL, ST-XS, ST-XL, ST-YL, ST-ZL, QAS-40, LSS-35, LSS-45, ST-UP, ST-OUP and ST-AK; from Nippon Aerosil Co., Ltd., under the trade name of Aerosil 50, Aerosil 90G, Aerosil 130, Aerosil 200, Aerosil 200V, Aerosil 200CF, Aerosil 200FAD, Aerosil 300, Aerosil 300CF, Aerosil 380, Aerosil R972, Aerosil R972V, Aerosil R972CF, Aerosil R974, Aerosil R202, Aerosil R805, Aerosil R812, Aerosil R812S, Aerosil MOX80, Aerosil MOX170, Aerosil COK84, Aerosil TT600 and Aerosil OX50; and the like.

Alumina particles are available from Nissan Chemical Industries, Ltd., under the trade name of Alumina Sol-100, Alumina Sol-200, Alumina Sol-520 and the like.

Powder and molten material dispersion products of alumina, titanium oxide, indium oxide, tin oxide and zinc oxide are available from CI Kasei Co. , Ltd. , under the trade name of Nanotek.

These metal oxide microparticles are contained in an amount ranging from 1 weight part to 100 weight parts and preferably from 1 weight part to 60 weight parts, based on 100 weight parts of the mixed composition. When too much of the metal oxide microparticles is contained, the transparency of the coating film is deteriorated. When too little of the metal oxide microparticles is contained, the effect due to addition thereof is insufficient. Within the above-mentioned range, it is possible to obtain a coating film excellent in balancing transparency, water resistance, gas barrier properties and abrasion resistance.

### Anti-static Agent

The mixed composition may further contain an anti-static agent. As the anti-static agent, alkali metal salts can be used.

Examples of the cation of alkali metal salts include Li⁺, Na⁺, K⁺ and the like. Preferably used are Li⁺ and Na⁺ having a small ionic radius, and further preferably used is Li⁺. Also, as the anion, there can be used NO₃⁻, SCN⁻, ClO₄⁻, CF₃SO₃⁻, BF₄⁻, (CF₃SO₂)₂N⁻ and (CF₃SO₂)₃C⁻.

As the alkali metal salts, preferably used are lithium perchlorate, lithium trifluoromethanesulfonate, lithium bis(trifluoromethanesulfonyl)imide and lithium tri(trifluoromethanesulfonyl)methane, and more preferably used are lithium trifluoromethanesulfonate, lithium bis(trifluoromethanesulfonyl)imide and lithium tri(trifluoromethanesulfonyl)methane.

The composite of the present invention can be made in the form of a particle or a film. Or, the composite may be laminated on a substrate or a porous support to form a laminate composite.

As a method for producing a particulate composite, there are a method of forming it by drying the mixed dispersion solution of the present invention at a prescribed temperature, and then subjecting the resultant solid to a treatment such as grinding, classifying or the like, a method of forming it by drying it for removing a solvent at a low temperature as in the freeze-drying method, and then subjecting the resultant solid to a treatment such as grinding, classifying or the like, a method of obtaining white powder by spraying composite microparticles of not more than 10 µm using a spray-dryer and volatilizing a solvent, and the like.

As a method for producing a film-like composite, there can be used, depending on the target base material and shape, a dip coating method, a spin coating method, a spray coating method, a flow coating method, a plate coating method, a bar coating method, a die coating method, and other suitable methods. As the base material, there can be used porous supports in addition to molded products of metals, glasses, ceramics, polymers and the like, sheets, films and the like.

As a method for producing a porous support and a film-like composite, there is mentioned a method of dipping the porous support in the mixed composition of the present invention and drying the porous support while maintaining it at a prescribed temperature.

Examples of the porous support used for the present invention include ceramics such as silica, alumina, zirconia, titania and the like; metals such as stainless steel, aluminum and the like; and porous material such as paper, resin and the like.

### Coated Laminate and Plastic Laminate

In the present invention, the coated laminate refers to a laminate arranged as a coating film layer (II-a) obtained by coating the mixed composition on the plastic base material (I) before the sol-gel reaction.

Furthermore, the plastic laminate refers to a laminate arranged as a coating film layer (II-b) obtained by coating the mixed composition on the plastic base material (I), removing the solvent (C) from the mixed composition and carrying out the sol-gel reaction.

All of these laminates are divided into two structures as follows. Hereinafter, the coating film layers (II-a) and (II-b) are generically named as the coating film layer (II).
(1) A laminated structure consisting of the plastic base material (I) and the coating film layer (II) in the order of (I)/(II); and
(2) A laminated structure consisting of the plastic base material (I), a metallic thin film layer (III) composed of an inorganic compound and the coating film layer (II) in the order of (I)/(III)/(II).

As the construction of the laminated structure, respective two layers of the deposited film layer (III) and the coating film layer (II) can also be alternatively laminated, for example, (I)/(III)/(II)/(III)/(II), or two or more plastic laminates may be laminated in the order of (I)/(II)/(IV)/(I)/(II) or (I)/(III)/(II)/(IV)/(I)/(III)/(II) using an adhesive layer (IV) or an adhesive material layer (V) to be described later. However, the production cost is increased when the number of layers is increased. So, the number of respective layers is practically not more than 10 layers and preferably not more than 7 layers.

### Plastic Base Material (I)

The plastic base material (I) is not particularly limited as long as the composition of the present invention can be laminated, and examples thereof include a plastic plate, a lens, a sheet, a film, a bottle or a tank-shaped molded product and the like.

Specific examples of the kind of the resin used for the plastic base material include polyolefin resins such as polyethylene, polypropylene, cycloolefin copolymer and the like; polyamide resins such as nylon 6, nylon 66 and the like; polyester resins such as polyethylene terephthalate, polyethylene naphthalate and the like; polyimide resins, polycarbonate resins, polyacrylonitrile resins, polyurethane resins, polythiourethane resins, polymethacrylate ester resins, polystyrene resins, AB resins, ABS resins, PEEK resins, PEK resins, PES resins, polylactic acid resins and the like. The resin may be a mixture of these resins or a laminate of these resins. When the resin is a film, the resin may be an unstretched film or a stretched film. Furthermore, the surface of the plastic base material (I) may be subjected to surface modification such as corona treatment, plasma treatment, UV ozone treatment or alkali treatment, whereby it is also possible to enhance adhesion of the coating film layer (II) and/or the deposited film (III).

### Coating Film Layer (II)

The coating film layer (II) used in the present invention is formed from the aforementioned mixed composition or the composition containing a sol-gel reactant.

Incidentally, a water-soluble polymer may be added to the coating film layer (II) to the extent that water resistance and hard coat properties are not impaired, in addition to metal oxides obtained from microparticles (A) of the polyolefin based terminally branched copolymer and metal alkoxide and/or a hydrolysis condensate thereof (B). Gas barrier properties, film strength and the like are enhanced under low humidity condition of the coating film layer by containing the water-soluble polymer. Examples of this water-soluble polymer include those as described above.

Meanwhile, the coating film layer (II) is formed by applying a coating composition containing metal alkoxide and/or a hydrolysis condensate thereof (C) and a dispersion solution of the polyolefin based terminally branched copolymer dispersed in water and/or an organic solvent having an affinity for water, and drying the resultant material.

In the coating film layer (II), as described above, the aforementioned terminally branched copolymer microparticles and the aforementioned metal oxide are contained in a prescribed range. When the dispersion is contained in an amount of 100 weight parts, the metal oxide may be contained in an amount of 10 weight parts to 1,000 weight parts, preferably 20 weight parts to 950 weight parts and further preferably 30 weight parts to 900 weight parts. The coating film composed only of the aforementioned terminally branched copolymer does not have sufficient water resistance, and the coating film composed only of the metal oxide does not have sufficient film formability. So, by forming a composite of both components, it is possible to obtain a coating film excellent in practical properties.

The optimum value of the thickness of the coating film layer (II) varies depending on the purpose, the use, the kinds of the terminally branched copolymer and the aforementioned metal oxide, the content and proportion. The thickness thereof is in the range of 0.02 µm to 100 µm, preferably in the range of 0.05 µm to 80 µm and further preferably in the range of 0.1 µm to 70 µm.

When the content and film thickness of the aforementioned terminally branched copolymer dispersion and metal oxide are in the above range, physical properties of the composition such as water resistance, drug resistance, film formability, strength, heat resistance and the like are excellent, and in case of using it as a coating film, gas barrier properties and hard coat properties are excellent.

### Method of Laminating Coating Film Layer (II)

The coating film layer (II) is produced by forming a film-like product of the mixed composition of the present invention or the sol-gel reactant thereof and thermally treating the film-like product.

The film-like product refers to the status in which the surface of a base material is covered with a composition. The method of forming a film-like product is not particularly limited. For example, there is a method of flow casting a solution of the mixed composition on a support such as glass plate, metal plate, thermoplastic resin film or the like, and drying, and the like. A film-like product is formed on a support to have a desired thickness, and then thermal treatment is carried out. The temperature for thermal treatment is usually in the range of not less than 50 degrees centigrade and not more than 250 degrees centigrade, more preferably in the range of not less than 80 degrees centigrade and not more than 200 degrees centigrade and further preferably in the range of not less than 80 degrees centigrade and not more than 150 degrees centigrade.

The film thickness of the coating film layer is in the range of not less than 0.02 µm and not more than 100 µm and preferably in the range of not less than 0. 05 µm and not more than 80 µm and further preferably in the range of not less than 0.1 µm and not more than 70 µm. When the film thickness is too thick, there is a possibility of causing cracks in the coating film.

### Structure of Coating Film Layer (II)

The coating film layer (II) formed in the laminate of the present invention is a composition containing at least the polymer microparticles (A) composed of the terminally branched copolymer and the component (B). As described above, the polyolefin based terminally branched copolymer constituting the polymer microparticles (A) is a hydrophobic substituent containing hydrocarbon or a hydrogen atom, in which, in the formula (1), A is a group having a number average molecular weight of 400 to 8,000 obtained by polymerizing an olefin having 2 to 20 carbon atoms, and R¹ and R² are each a hydrogen atom or an alkyl group having 1 to 18 carbon atoms and at least one of R¹ and R² is a hydrogen atom. On the other hand, X¹ and X² may be the same or different, and are each a hydrophilic substituent composed of linear or branched polyalkylene glycol groups respectively having a number average molecular weight of 50 to 10,000. The polyolefin based terminally branched copolymer is an amphiphilic compound having a hydrophobic group and a hydrophilic group in a molecule, while this aqueous dispersion solution becomes an emulsion of a core-shell structure divided into a core phase composed of a hydrophobic group and a shell phase composed of a hydrophilic group. When the cross section of the sample prepared according to the thin film cutting method is observed with a transmission electron microscope (TEM), it can be confirmed that this coating film layer (II) forms a phase separated structure divided into a metal oxide phase and a copolymer dispersion phase. It is considered that such a phase separated structure is observed from the fact that a hydrocarbon group forming a core phase of the emulsion and a metal oxide are not compatible with each other.

The metal oxide is contained as a structure body in which it is dispersed or aggregated in the form of microparticles in the composition, or becomes a continuous structure body as a matrix of the composition. It is better to be a continuous matrix structure body for the metal oxide in view of production of the coating film layer excellent in properties such as gas barrier properties, hard coat properties and the like, and such a structure body of metal oxide is obtained by subjecting metal alkoxide to hydrolysis and/or hydrolysis and polycondensation, that is, by the sol-gel reaction.

On the other hand, since the shell phase of the aforementioned emulsion is composed of a polyalkylene glycol group having high hydrophilicity, the shell phase functions to prevent aggregation or fusion between emulsions in the aqueous dispersion solution. The coating film layer (II) of the present invention is formed by proceeding with the sol-gel reaction of the metal alkoxide contained in the aforementioned mixed composition, and then applying it to the plastic base material and drying the resultant material. A hydrolysate of metal alkoxide or its polycondensation product generated during this process is not compatible with the hydrophobic core shell of the emulsion composed of hydrocarbon, thus it may be present in a dispersion medium. The hydrolysate of metal alkoxide or its polycondensation product is concentrated in gaps between emulsion particles. As the reaction proceeds, when the dispersion medium is finally evaporated, a matrix is formed as a continuous structure of the metal oxide so as to be embedded between the hydrophobic core shells.

### Deposited Film Layer (III)

The deposited film layer (III) to be used for the present invention is composed of an inorganic compound. Specifically, it is preferable that the deposited film layer (III) contains one or more oxides, nitrides or oxynitrides selected from Si, Ta, Nb, Al, In, W, Sn, Zn, Ti, Cu, Ce, Ca, Na, B, Pb, Mg, P, Ba, Ge, Li, K and Zr as a main component. In particular, oxides, nitrides or oxynitrides of Si and Al are more preferable from the viewpoints of adhesion and affinity for the coating film layer (II), production stability, safety and cost.

A method of forming the deposited film layer (III) is carried out by means of a physical vapor deposition method (PVD method), a low temperature plasma chemical vapor deposition method (CVD method), ion plating, sputtering and the like. The preferred film thickness of the deposited film layer (III) is in the range of 5 to 1,000 nm, and particularly preferably in the range of 10 to 100 nm. Within this range, it is possible to form a film excellent in gas barrier properties and flexing resistance.

### Adhesive Layer (IV)

In the present invention, a laminated film obtained by laminating with an adhesive as a gas barrier material used for food packaging has been widely used. As the layer used as this adhesive (an adhesive layer (IV)), known ones can be used as long as it is transparent. Specific examples thereof include an epoxy based adhesive, a urethane based adhesive, a cyanoacrylate instant adhesive, a modified acrylate based adhesive and the like.

### Adhesive Material Layer (V)

The adhesive material layer (V) in the present invention is used for bonding a laminate obtained by laminating the deposited film layer (III) and the coating film layer (II) on the plastic base material (I), and, in addition thereto, is used, as necessary, for laminating a functional transparent layer (VI) to be described below on the outermost layer of the gas barrier material. Also, it can be used for bonding a plurality of functional transparent layers. As the adhesive material layer (V) of the present invention, known adhesive materials can be used without limitation as long as it is transparent. Specifically, those described in Japanese Patent Laid-open No. 1998-217380 and Japanese Patent Laid-open No. 2002-323861 and the like can be adopted.

### Functional Transparent Layer (VI)

As the functional transparent layer (VI), an anti-glare film, a hard coat layer, an anti-staining layer, a charge preventive layer, a toning layer, an anti-reflection layer, a light extraction efficiency-improving layer or the like can be adopted. These layers can be used for one surface or both surfaces of the above-described transparent gas barrier film, or the inside thereof. As the functional transparent layer (VI), more specifically, those described in Japanese Patent Laid-open No. 1998-217380, Japanese Patent Laid-open No. 2002-323861 and the like can be adopted.

### Properties and Use of Coating Film

The coating film layer of the present invention is excellent in physical properties such as water resistance, drug resistance, film formability, strength, heat resistance and the like, and in case of using it as a coating film, it is excellent in gas barrier properties and hard coat properties. So, the coating film layer is effectively used because a plastic base material which is particularly low in gas barrier properties and hard coat properties can be provided with these properties by coating its surface with it.

### Hard Coat (Abrasion Resistance) Properties

Hard coat properties in the present invention refer to a function to protect the layer from abrasion or the like by covering the surface of a plastic base material which is low in abrasion resistance with a thin film. In particular, there have been strongly demanded hard coat materials for preventing any scratches on the surface of a transparent resin film or sheet, plastic lens and the like used for the purpose of display. In the present invention, hard coat properties (abrasion resistance) of the coating film onto a plastic base material were evaluated according to the steel wool test.

### Gas Barrier Properties

The gas barrier properties refer to the properties of blocking oxygen, water vapor and other gases which accelerate the quality deterioration of the material to be coated. In particular, packaging materials for food, medicine and the like or electronic materials are required to have excellent barrier properties against oxygen gas. High gas barrier properties can be achieved by laminating the plastic base material (I), the deposited film layer (III), and the coating film layer (II) in the order of (I)/(III)/(II). According to the invention, the gas-barrier properties of a coating film were evaluated by measuring the oxygen permeability of the coating film.

### EXAMPLES

Hereinafter, the present invention will be illustrated in more detail with reference to Examples A and B, but the scope of the present invention is not intended to be limited to these Examples and the like.

### Example A

In addition, the number average molecular weight (Mn), the weight average molecular weight (Mw) and the molecular weight distribution (Mw/Mn) were measured using GPC according to the method as described herein. For the melting point (Tm), the peak top temperature obtained by measuring with DSC was used. Incidentally, the melting point of the polyalkylene glycol portion is also confirmed under the measurement conditions, but indicates the melting point of the polyolefin portion unless otherwise particularly noted. The measurement by ¹H-NMR was carried out at 120 degrees centigrade after completely dissolving the poymer in deuterated-1,1,2,2-tetrachloroethane, which functions both as the lock solvent and the solvent, in a sample tube for measurement. For the chemical shift, the peak of deuterated-1,1,2,2-tetrachloroethane was set at 5.92 ppm, and the chemical shift values of other peaks were determined on this basis. For the particle size of the particle in the dispersion solution, the average particle size of 50% by volume was measured with a Microtrack UPA (a product of Honeywell, Inc.). The shape of the particle in the dispersion solution was observed under a condition of 100 kV with a transmission electron microscope H-7650 (a product of Hitachi, Ltd.), after diluting the sample 200 to 500 times and performing negative staining with phosphotungstic acid. The zeta potential was measured by using a zeta potential and particle size analyzer ELSZ-2 (a product of Otsuka Electronics Co., Ltd.). The surface specific resistivity was measured in accordance with JIS-K6911 after adjusting the conditions to a temperature of 23 ± 2 degrees centigrade and a humidity of 50 ± 5%RH.

### Synthesis Example A1

In accordance with Synthesis Example 2 of Japanese Patent Laid-open No. 2006-131870, an epoxy-terminated ethylenic polymer (E-1) (Mw: 2,058, Mn: 1,118, Mw/Mn: 1.84 (GPC)) was synthesized and used as a raw material (content of terminal epoxy group: 90 mol%).

¹H-NMR : δ(C₂D₂Cl₄) 0.88 (t, 3H, J = 6.92 Hz), 1. 18 - 1.66 (m), 2.38 (dd, 1H, J = 2.64, 5.28 Hz), 2.66 (dd, 1H, J = 4.29, 5.28 Hz) 2.80 - 2.87 (m, 1H)
Melting point (Tm): 121 degrees centigrade
Mw=2,058, Mn=1,118, Mw/Mn=1.84 (GPC)

84 weight parts of the epoxy-terminated ethylenic polymer (E-1), 39.4 weight parts of diethanolamine and 150 weight parts of toluene were introduced into a 1,000-mL flask, and stirred at 150 degrees centigrade for 4 hours. Thereafter, acetone was added while cooling the mixture to precipitate the reaction product, and the solid was collected by filtration. The obtained solid was stirred and washed with an aqueous acetone solution one time and further with acetone three times, and then the solid was collected by filtration. Thereafter, the solid was dried at room temperature under reduced pressure to obtain a polymer (I-1) (Mn: 1,223, in the general formula (9), A: a group formed by polymerization of ethylene (Mn: 1,075), R¹ and R²: a hydrogen atom, one of Y¹ and Y²: a hydroxyl group, the other of Y¹ and Y²: a bis (2-hydroxyethyl) amino group)).

¹H-NMR : δ(C₂D₂Cl₄) 0.88 (t, 3H, J = 6.6 Hz), 0.95-1.92 (m), 2.38-2.85 (m, 6H), 3.54-3.71 (m, 5H)
Melting point (Tm): 121 degrees centigrade

20.0 weight parts of the polymer (I-1) and 100 weight parts of toluene were introduced into a 500-mL flask equipped with a nitrogen inlet tube, a thermometer, a condenser and a stirring device, and heated in an oil bath at 125 degrees centigrade with stirring to completely dissolve the solid. After cooling to 90 degrees centigrade, 0.323 weight parts of 85% KOH that had been dissolved in 5.0 g of water in advance was added to the flask, and the contents were mixed under reflux for 2 hours. Subsequently, the temperature in the flask was slowly increased to 120 degrees centigrade, and water and toluene were distilled off. Furthermore, water and toluene in the flask were completely distilled off by reducing the pressure in the flask while supplying minimal nitrogen into the flask, increasing the internal temperature to 150 degrees centigrade, and then keeping the temperature for 4 hours. After cooling to room temperature, the solid solidified in the flask was broken and taken out.

18.0 weight parts of the obtained solid and 200 weight parts of dehydrated toluene were introduced into a 1.5-L stainless steel pressurized reactor equipped with a heating device, a stirring device, a thermometer, a manometer and a safety valve, and after purging the gas phase with nitrogen, the contents were heated to 130 degrees centigrade with stirring. After 30 minutes, 9.0 weight parts of ethylene oxide was added thereto. After further maintaining at 130 degrees centigrade for 5 hours, the contents were cooled to room temperature to obtain a reactant. The solvent was removed by drying from the resulting reactant to obtain a terminally branched copolymer (T-1) (Mn: 1,835, in the general formula (1), A: a group formed by polymerization of ethylene (Mn: 1,075), R¹ and R²: a hydrogen atom, one of X¹ and X²: polyethylene glycol, the other of X¹ and X²: a group represented by the general formula (5) (Q¹=Q²: an ethylene group, X⁹=X¹⁰: polyethylene glycol)).

¹H-NMR : δ(C₂D₂Cl₄) 0.88 (3H, t, J= 6.8 Hz), 1.06 - 1.50 (m), 2.80 - 3.20 (m), 3.33 - 3.72 (m)
Melting point (Tm): -16 degrees centigrade (polyethylene glycol), 116 degrees centigrade

### Synthesis Example A2

A terminally branched copolymer (T-2) (Mn: 3,669) was obtained in the same manner as in Synthesis Example A1, except that the amount of ethylene oxide in use was changed to 36.0 weight parts.

Melting point (Tm) : 50 degrees centigrade (polyethylene glycol), 116 degrees centigrade

### Synthesis Example A3

In accordance with Synthesis Example 8 of Japanese Patent Laid-open No. 2006-131870, an epoxy-terminated ethylene-propylene copolymer (E-2) (Mw: 1,576, Mn: 843, Mw/Mn: 1.87 (GPC)) was synthesized and used as a raw material.

¹H-NMR : δ(C₂D₂Cl₄) 0 . 80 - 0.88(m), 0. 9 - 1.6 (m), 2. 37 - 2.40 (1H, dd, J = 2.97, 5.28 Hz), 2.50(m), 2.66 (1H, dd, J = 3.96, 5.28 Hz) 2.80 - 2.86 (1H, m), 2.95(m)
Mw=1,576, Mw/Mn=1.87 (GPC)
Melting point (Tm): 107 degrees centigrade

A polymer (I-2) (Mn: 948, in the general formula (9), A: a group formed by copolymerization of ethylene and propylene (Mn: 800), one of R¹ and R²: a hydrogen atom, the other of R¹ and R²: a hydrogen atom or a methyl group, one of Y¹ and Y²: a hydroxyl group, the other of Y¹ and Y²: a bis (2-hydroxyethyl) amino group) was obtained in the same manner as in Synthesis Example A1, except that 63.2 weight parts of the epoxy-terminated ethylene-propylene copolymer (E-2) was used instead of the epoxy-terminated polymer (E-1).

¹H-NMR : δ(C₂D₂Cl₄) 0.80 - 0.90 (m), 0.90-1.56 (m), 2.46 (dd, 1H, J = 9.2, 13.5 Hz), 2.61 (dd, 1H, J = 3.3, 13.5 Hz), 2.61 - 2.84 (m, 4H), 3.58 - 3.68 (m, 5H)
Melting point (Tm): 106 degrees centigrade

A terminally branched copolymer (T-3) (Mn: 1,422, in the general formula (1), A: a group formed by copolymerization of ethylene and propylene (Mn: 800), one of R¹ and R²: a hydrogen atom, the other of R¹ and R²: a hydrogen atom or a methyl group, one of X¹ and X²: polyethylene glycol, the other of X¹ and X²: a group represented by the general formula (5) (Q¹=Q²: an ethylene group, X⁹=X¹⁰: polyethylene glycol)) was obtained in the same manner as in Synthesis Example A1, except that the polymer (I-2) was used instead of the polymer (I-1), and 85% KOH was used in an amount of 0.418 weight parts.

¹H-NMR : δ(C₂D₂Cl₄) 0.83 - 0.92(m), 1.08 - 1.50 (m), 2.70 - 3.00 (m), 3.55 - 3.69 (m)
Melting point (Tm): -20 degrees centigrade (polyethylene glycol), 105 degrees centigrade

### Synthesis Example A4

In accordance with Example 20 of Japanese Patent Laid-open No. 2006-131870, a polymer (I-3) (Mn: 1,136, in the general formula (9), A: a group formed by polymerization of ethylene (Mn: 1,075), R¹ and R²: a hydrogen atom, both of Y¹ and Y²: a hydroxyl group) was synthesized and used as a raw material (yield: 99%, conversion rate of a polymer having a terminal double bond: 100%). The physical properties thereof are as follows.
¹H-NMR : δ(C₂D₂Cl₄) 0.89 (3H, t, J = 6.92 Hz), 1. 05 - 1.84 (m), 3.41 (2H, dd, J = 5.94, 9.89 Hz), 3.57 - 3.63 (1H, m)
Melting point (Tm): 122 degrees centigrade
Hardness (degree of penetration): 0 mm
Melt viscosity: 214 cp (140 degrees centigrade)
Softening point: 129 degrees centigrade
Temperature for 5% weight reduction: 297 degrees centigrade (Thermogravimetric Analysis (TGA))

A terminally branched copolymer (T-4) (Mn: 1, 704, in the general formula (1), A: a group formed by polymerization of ethylene (Mn: 1,075), both of R¹ and R²: a hydrogen atom, both of X¹ and X²: a group composed of polyethylene glycol) was obtained in the same manner as in Synthesis Example A1, except that the polymer (I-3) was used instead of the polymer (I-1), and 85% KOH was used in an amount of 0.3081 weight parts.

¹H-NMR : δ(C₂D₂Cl₄) 0.88 (3H, t, J= 6.6 Hz), 1.04 - 1.47 (m), 3.32 - 3.69 (m)
Melting point (Tm): 119 degrees centigrade

### Synthesis Example A5

A terminally branched copolymer (T-5) (Mn: 2,446) was obtained in the same manner as in Synthesis Example A1, except that the amount of ethylene oxide in use was changed to 18.0 weight parts.
Melting point (Tm) : 27 degrees centigrade (polyethylene glycol), 118 degrees centigrade

### Synthesis Example A6

A terminally branched copolymer (T-6) (Mn: 6,115) was obtained in the same manner as in Synthesis Example A1, except that the amount of ethylene oxide in use was changed to 72.0 weight parts.
Melting point (Tm) : 55 degrees centigrade (polyethylene glycol), 116 degrees centigrade

### Synthesis Example A7

A terminally branched copolymer (T-7) (Mn: 1,896) was obtained in the same manner as in Synthesis Example A3, except that the amount of ethylene oxide in use was changed to 18.0 weight parts.
Melting point (Tm) : 25 degrees centigrade (polyethylene glycol), 102 degrees centigrade

### Synthesis Example A8

100 weight parts of the epoxy-terminated ethylene-propylene copolymer (E-2) and 300 weight parts of toluene were introduced into a 2,000-mL flask equipped with a nitrogen inlet tube, a thermometer, a condenser and a stirring device, and heated in an oil bath at 125 degrees centigrade with stirring to completely dissolve the solid. After cooling to 90 degrees centigrade and then heating under reflux for 30 minutes with stirring, 32.7 weight parts of formic acid was slowly added at the internal temperature of 90 to 92 degrees centigrade for carrying out the esterification reaction for 10 hours. Subsequently, while the temperature was maintained, 100 weight parts of warm water was added and allowed to stand to remove the aqueous layer. 75 weight parts of a 5% KOH n-BuOH solution was added thereto, and the contents were stirred at 105 degrees centigrade for 3 hours. After cooling to 60 degrees centigrade, 300 weight parts of methanol was slowly added to crystallize the product with cooling, and the solid collected by filtration was washed with methanol. The obtained solid was dried under reduced pressure, whereby a polymer (I-4) (Mn: 860, in the general formula (9), A: a group formed by copolymerization of ethylene and propylene (Mn: 800), one of R¹ and R²: a hydrogen atom, the other of R¹ and R²: a hydrogen atom or a methyl group, both of Y¹ and Y²: a hydroxyl group) was synthesized and used as a raw material (yield: 87%, conversion rate of a polymer having a terminal double bond: 100%). The physical properties thereof are as follows.
¹H-NMR : δ(C₂D₂Cl₄) 0.88 (m), 1.0 - 1.80 (m), 3.41 (1H, dd, J = 7.58, 11.2 Hz), 3.40 - 3.45 (2H, m)
Melting point (Tm): 106 degrees centigrade

A terminally branched copolymer (T-8) (Mn: 1,290, in the general formula (1), A: a group formed by copolymerization of ethylene and propylene (Mn: 800), one of R¹ and R²: a hydrogen atom, the other of R¹ and R²: a hydrogen atom or a methyl group, both of Y¹ and Y²: a hydroxyl group) was obtained in the same manner as in Synthesis Example A1, except that the polymer (I-4) was used instead of the polymer (I-1), and 85% KOH was used in an amount of 0.306 weight parts.
Melting point (Tm): 107 degrees centigrade

### Synthesis Example A9

A terminally branched copolymer (T-9) (Mn: 1,433) was obtained in the same manner as in Synthesis Example A8, except that the amount of ethylene oxide in use was changed to 12.0 weight parts.
Melting point (Tm): 102 degrees centigrade

### Synthesis Example A10

A terminally branched copolymer (T-10) (Mn: 1,720) was obtained in the same manner as in Synthesis Example A8, except that the amount of ethylene oxide in use was changed to 18.0 weight parts.
Melting point (Tm): 101 degrees centigrade

### Synthesis Example A11

A terminally branched copolymer (T-11) (Mn: 2,844) was obtained in the same manner as in Synthesis Example A3, except that the amount of ethylene oxide in use was changed to 36.0 weight parts.
Melting point (Tm): 103 degrees centigrade

### Synthesis Example A12

In accordance with Synthesis Example 2 of Japanese Patent Laid-open No. 2008-274066, a vinyl-terminated ethylenic polymer (A-1) (Mw: 1,380, Mn: 627, Mw/Mn: 2.20 (GPC)) was synthesized and used as a raw material.
Melting point (Tm): 116 degrees centigrade
¹H-NMR : δ(C₆D₆) 0.81 (t, 3H, J=6.9 Hz), 1.10 - 1.45 (m), 1.95 (m, 2H), 4.84 (dd, 1H, J = 9.2, 1.6Hz), 4.91 (dd, 1H, J = 17.2, 1.6 Hz), 5.67 - 5.78 (m, 1H)
GPC1: Mw=1,380, Mw/Mn=2.20

100 weight parts of the polymer A-1, 300 weight parts of toluene and 1.84 weight parts of CH₃(n-C₈H₁₇)₃NHSO₄ were introduced into a 1,000-mL separable flask equipped with a nitrogen inlet tube, a thermometer, a Dimroth condenser, an oxymeter, a mechanical stirrer and a feed pump, and the polymer was dissolved under toluene reflux and then cooled to 90 degrees centigrade. 6.50 weight parts of an aqueous solution of 40% Na₂WO₄ and 0.45 weight parts of 85% phosphoric acid were put thereinto. While maintaining the temperature at 90 ± 2 degrees centigrade, 53.60 weight parts of 30% aqueous hydrogen peroxide was added from the feed pump over 3 hours and further stirred for 3 hours, and then 100% of the modification rate was confirmed by FT-IR and ¹H-NMR. The internal temperature of the reactor was cooled to 85 degrees centigrade. While maintaining its temperature, an aqueous solution of 40% sodium thiosulfate was added over 30 minutes and further stirred for 30 minutes. Then, peroxide 0 was confirmed by using a POV test paper and stirring was stopped for solution separation. At its temperature, the aqueous layer was taken out and further washed two times to carry out a solution separating operation, and then cooled. The waxy solid was discharged to 300 weight parts of acetone and suspended, the suspension was filtered, the leached solid was washed with stirring with an aqueous 50% methanol solution and subsequently with methanol, and the leached solid was dried under reduced pressure of 10 hPa at 60 degrees centigrade to obtain a polymer (E-3) (content of terminal epoxy group: 90 mol%).

¹H-NMR : δ(C₂D₂Cl₄) 0.88 (3H, t, J = 6.59 Hz), 1.04 - 1.50 (m), 2.38 (1H, dd, J = 2.64, 5.28 Hz), 2.66 (1H, dd, J = 3.96, 5.28 Hz) 2.80-2.87 (1H, m) ppm.
Melting point (Tm): 119 degrees centigrade (DSC)
Degree of penetration: 1 (10-1 mm)
Softening point: 125.0 degrees centigrade
Melt viscosity: 86 mPa·s
Temperature for 5% weight reduction: 323.3 degrees centigrade (TGA)
Mw: 1,800, Mw/Mn: 1.96 (GPC)

A polymer (I-5) (Mn: 1,023, in the general formula (9), A: a group formed by polymerization of ethylene (Mn: 875), R¹ and R²: a hydrogen atom, one of Y¹ and Y²: a hydroxyl group, the other of Y¹ and Y²: a bis (2-hydroxyethyl) amino group) was obtained in the same manner as in Synthesis Example A1, except that the polymer (E-3) was used in an amount of 68.8 weight parts instead of the polymer (E-1).

¹H-NMR : δ(C₂D₂Cl₄) 0.87 (t, 3H, J = 6.6 Hz), 1.03 - 1.69 (m), 2.38 - 2.82 (m, 6H), 3.54 - 3.69 (m, 5H)
Melting point (Tm): 119 degrees centigrade

A terminally branched copolymer (T-12) (Mn: 2,558, in the general formula (1), A: a group formed by polymerization of ethylene (Mn: 875), R¹ and R²: a hydrogen atom, one of X¹ and X²: polyethylene glycol, the other of of X¹ and X²: a group represented by the general formula (5) (Q¹=Q²: an ethylene group, X⁹=X¹⁰: polyethylene glycol)) was obtained in the same manner as in Synthesis Example A1, except that the polymer (I-5) was used instead of the polymer (I-1), 85%KOH was used in an amount of 0.386 weight parts, and the amount of ethylene oxide in use was changed to 27.0 weight parts.

¹H-NMR : δ(C₂D₂Cl₄) 0.86 (3H, t, J= 6.6 Hz), 1. 06 - 1.80 (m), 2.80 - 3.20 (m), 3.33 - 3.84 (m)
Melting point (Tm): 116 degrees centigrade

### Synthesis Example A13

In accordance with Example 4 of Japanese Patent Laid-open No. 2003-073412, a vinyl-terminated ethylenic polymer (A-2) (Mw: 3,800, Mn: 2,171, Mw/Mn: 1.75 (GPC)) was synthesized and used as a raw material.
Melting point (Tm): 124 degrees centigrade

100 weight parts of the polymer A-2, 300 weight parts of toluene and 0.53 weight parts of CH₃(n-C₈H₁₇)₃NHSO₄ were introduced into a 1,000-mL separable flask equipped with a nitrogen inlet tube, a thermometer, a Dimroth condenser, an oxymeter, a mechanical stirrer and a feed pump, and the polymer was dissolved under toluene reflux and then cooled to 90 degrees centigrade. 1.88 weight parts of an aqueous solution of 40% Na₂WO₄ and 0.13 weight parts of 85% phosphoric acid were put thereinto. While maintaining the temperature at 90 ± 2 degrees centigrade, 15.50 weight parts of aqueous 30% hydrogen peroxide was added from the feed pump over 3 hours and further stirred for 3 hours, and then 100% of the modification rate was confirmed by FT-IR and ¹H-NMR. The internal temperature of the reactor was cooled to 85 degrees centigrade. While maintaining its temperature, an aqueous solution of 40% sodium thiosulfate was added over 30 minutes and further stirred for 30 minutes. Then, peroxide 0 was confirmed by using a POV test paper and stirring was stopped for solution separation. At its temperature, the aqueous layer was taken out and further washed two times to carry out a solution separating operation, and then cooled. The waxy solid was discharged to 300 weight parts of acetone and suspended, the suspension was filtered, the leached solid was washed with stirring with an aqueous 50% methanol solution and subsequently with methanol, and the leached solid was dried under reduced pressure of 10 hPa at 60 degrees centigrade to obtain a polymer (E-4) (content of terminal epoxy group: 91 mol%).

¹H-NMR : δ(C₂D₂Cl₄) 0.87 (3H, t, J = 6.7 Hz), 1.05 - 1.50 (m), 2.38 (1H, dd, J = 2.70, 5.13 Hz), 2.66 (1H, dd, J = 4.05, 5.13 Hz) 2.80-2.87 (1H, m) ppm.
Mw=3,484, Mw/Mn=2.02 (GPC)

A polymer (I-6) (Mn: 1,830, in the general formula (9), A: a group formed by polymerization of ethylene (Mn: 1,682), R¹ and R²: a hydrogen atom, one of Y¹ and Y²: a hydroxyl group, the other of Y¹ and Y²: a bis (2-hydroxyethyl) amino group) was obtained in the same manner as in Synthesis Example A1, except that the polymer (E-4) was used in an amount of 129.4 weight parts instead of the polymer (E-1).

¹H-NMR : δ(C₂D₂Cl₄) 0.87 (t, 3H, J = 6.5 Hz), 1.03 - 1.49 (m), 2.40 - 2.80 (m, 6H), 3.59 - 3.64 (m, 5H)
Melting point (Tm): 126 degrees centigrade

A terminally branched copolymer (T-13) (Mn: 5,490, in the general formula (1), A: a group formed by polymerization of ethylene (Mn: 1,682), R¹ and R²: a hydrogen atom, one of X¹ and X²: polyethylene glycol, the other of X¹ and X²: a group represented by the general formula (5) (Q¹=Q²: an ethylene group, X⁹=X¹⁰: polyethylene glycol)) was obtained in the same manner as in Synthesis Example A1, except that polymer (I-6) was used instead of the polymer (I-1), the amount of 85%KOH in use was 0.215 weight parts, the amount of ethylene oxide in use was changed to 36.0 weight parts, and the reaction time after addition of ethylene oxide was extended to 10 hours.

¹H-NMR : δ(C₂D₂Cl₄) 0.86 (3H, t, J= 6.6 Hz), 1. 02 - 1.88 (m), 2.85 - 4.35 (m)
Melting point (Tm) : 43 degrees centigrade (polyethylene glycol), 123 degrees centigrade

### Example A1

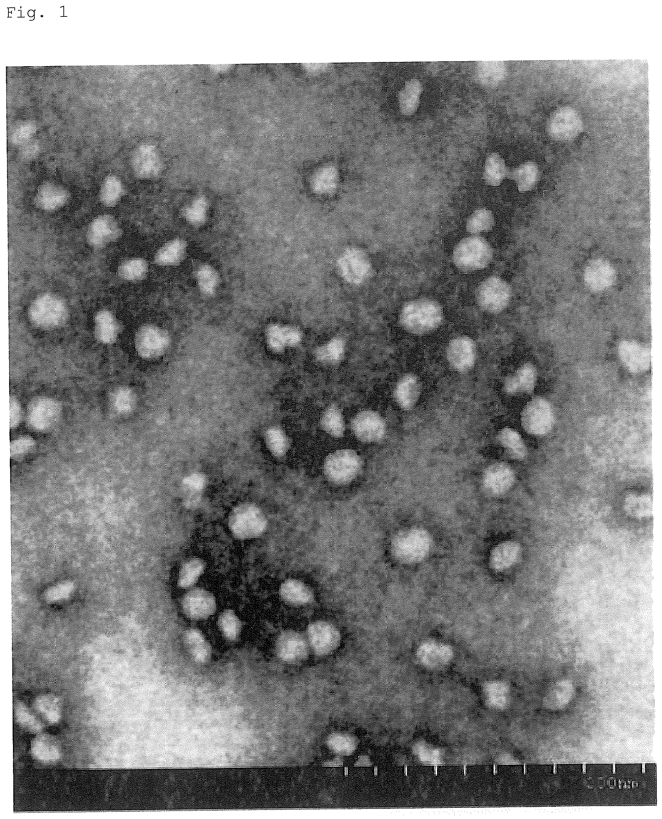

10 weight parts of the terminally branched copolymer (T-1) obtained in Synthesis Example A1 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.018 µm (average particle size of 10% by volume: 0.014 µm, average particle size of 90% by volume: 0. 022 µm). An observation view of the obtained dispersion solution using a transmission electron microscope is illustrated in Fig. 1. Incidentally, the particle size measured from Fig. 1 was from 0.015 to 0.030 µm.

### Example A2

10 weight parts of the terminally branched copolymer (T-2) obtained in Synthesis Example A2 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.015 µm (average particle size of 10% by volume: 0.012 µm, average particle size of 90% by volume: 0.028 µm).

### Example A3

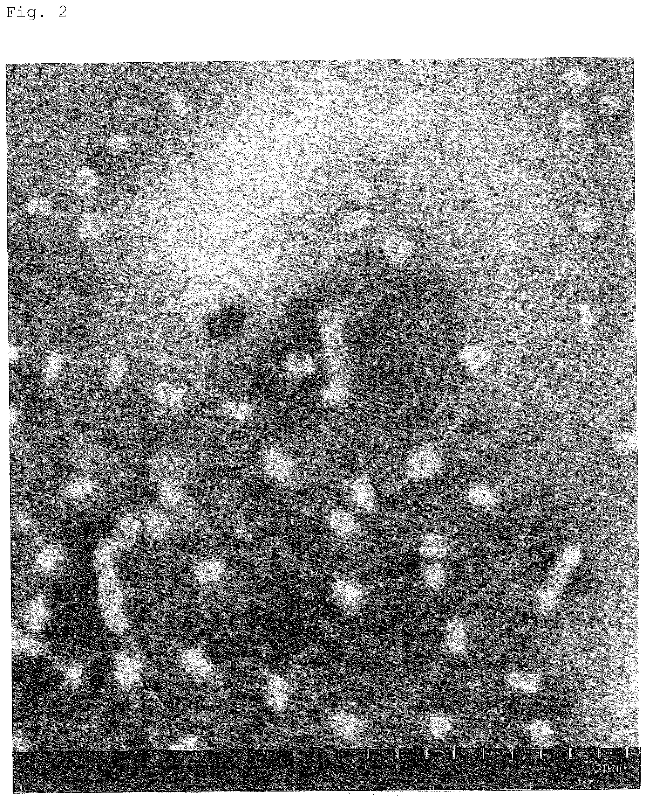

10 weight parts of the terminally branched copolymer (T-3) obtained in Synthesis Example A3 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.018 µm (average particle size of 10% by volume: 0.014 µm, average particle size of 90% by volume: 0.027 µm). An observation view of the obtained dispersion solution using a transmission electron microscope is illustrated in Fig. 2. Incidentally, the particle size measured from Fig. 2 was from 0.010 to 0.025 µm.

### Example A4

36 weight parts of the terminally branched copolymer (T-3) obtained in Synthesis Example A3 and 54 weight parts of distilled water were introduced into a high speed stirrer (T.K.FILMICS (registered trademark) 56-50 type, a product of Primix Corporation), and stirred at a rim speed of 50 m/sec. The contents were heated to 122 degrees centigrade and then stirred for 1 minute, cooled to 87 degrees centigrade while continuously stirring, and then stopped stirring and cooled to room temperature. The average particle size of 50% by volume of the obtained dispersion solution was 0.016 µm (average particle size of 10% by volume: 0.013 µm, average particle size of 90% by volume: 0.025 µm).

### Example A5

10 weight parts of the terminally branched copolymer (T-4) obtained in Synthesis Example A4 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.32 µm (average particle size of 10% by volume: 0.120 µm, average particle size of 90% by volume: 1.53 µm).

### Example A6

When 0.5 weight parts of an aqueous 10% sulfuric acid solution was added to 5.0 weight parts of the dispersion solution obtained in Example A1, the pH became 1. Although the dispersion solution was allowed to stand at room temperature for 1 month, aggregation and precipitation did not occur.

### Example A7

When 0.13 weight parts of an aqueous 10% sulfuric acid solution was added to 5.0 weight parts of the dispersion solution obtained in Example A1, the pH became 7. Although the dispersion solution was allowed to stand at room temperature for 1 month, aggregation and precipitation did not occur.

### Example A8

The pH of 5.0 weight parts of the dispersion solution obtained in Example A1 was 12. Although the dispersion solution was allowed to stand at room temperature for 1 month, aggregation and precipitation did not occur.

### Example A9

When 0.4 weight parts of an aqueous solution of 10% potassium hydroxide was added to 5.0 weight parts of the dispersion solution obtained in Example A1, the pH became 13. Although the dispersion solution was allowed to stand at room temperature for 1 month, aggregation and precipitation did not occur.

### Example A10

5.0 weight parts of the dispersion solution obtained in Example A1 was introduced into a 30-ml eggplant flask equipped with a nitrogen inlet tube, a thermometer, a condenser and a stirring device, stirred in an oil bath, and heated under reflux under the normal pressure at 100 degrees centigrade for 10 minutes. Although the obtained dispersion solution was allowed to stand at room temperature for 1 month, aggregation and precipitation did not occur.

### Example A11

5.0 weight parts of the dispersion solution obtained in Example A1 was introduced into a 30-ml eggplant flask, frozen with liquid nitrogen, and then dissolved at room temperature to give a dispersion solution. Although the obtained dispersion solution was allowed to stand at room temperature for 1 month, aggregation and precipitation did not occur.

### Example A12

10 weight parts of the terminally branched copolymer (T-1) obtained in Synthesis Example A1, 0.5 weight parts of a homopolyethylene wax (P-1) (Mw: 1, 900, Mn: 850, Mw/Mn: 2.24 (GPC)) obtained in accordance with Synthesis Example 1 of Japanese Patent Laid-open No. 2006-131870 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.019 µm.

### Example A13

10 weight parts of the terminally branched copolymer (T-1) obtained in Synthesis Example A1, 0.25 weight parts of a dye (trade name: HS-296, a product of Mitsui Chemicals, Inc.) and 40 weight parts of distilled water were introduced into a 100-ml autoclave, heated with stirring at a rate of 800 rpm at 140 degrees centigrade for 30 minutes, and then cooled to room temperature while stirring. The above-described dye was uniformly dispersed to obtain a dispersion solution, and the average particle size of 50% by volume of the resulting dispersion solution was 0.021 µm.

### Example A14

10 weight parts of the terminally branched copolymer (T-1) obtained in Synthesis Example A1, 0.5 weight parts of a dye (trade name: HS-296, a product of Mitsui Chemicals, Inc.) and 40 weight parts of distilled water were introduced into a 100-ml autoclave, heated with stirring at a rate of 800 rpm at 140 degrees centigrade for 30 minutes, and then cooled to room temperature while stirring. The above-described dye was uniformly dispersed to obtain a dispersion solution, and the average particle size of 50% by volume of the resulting dispersion solution was 0.021 µm.

### Example A15

10 weight parts of the terminally branched copolymer (T-1) obtained in Synthesis Example A1, 0.01 weight part of pyrene and 40 weight parts of distilled water were introduced into a 100-ml autoclave, heated with stirring at a rate of 800 rpm at 140 degrees centigrade for 30 minutes, and then cooled to room temperature while stirring. Pyrene was uniformly dispersed to obtain a dispersion solution, and the average particle size of 50% by volume of the resulting dispersion solution was 0.018 µm. The obtained dispersion solution was diluted 100 times with water and irradiated with an excitation light of 350 nm and as a result, strong fluorescence in the vicinity of 370 to 410 nm was observed.

### Example A16

10 weight parts of the terminally branched copolymer (T-1) obtained in Synthesis Example A1, 0.1 weight part of pyrene and 40 weight parts of distilled water were introduced into a 100-ml autoclave, heated with stirring at a rate of 800 rpm at 140 degrees centigrade for 30 minutes, and then cooled to room temperature while stirring. Pyrene was uniformly dispersed to obtain a dispersion solution, and the average particle size of 50% by volume of the resulting dispersion solution was 0.019 µm. The obtained dispersion solution was diluted 100 times with water and irradiated with an excitation light of 350 nm and as a result, strong fluorescence in the vicinity of 370 to 410 nm was observed.

### Example A17

10 weight parts of the terminally branched copolymer (T-1) obtained in Synthesis Example A1, 0.015 weight parts of 8-anilino-1-naphthalene sulfonic acid and 40 weight parts of distilled water were introduced into a 100-ml autoclave, heated with stirring at a rate of 800 rpm at 140 degrees centigrade for 30 minutes, and then cooled to room temperature while stirring. 8-anilino-1-naphthalene sulfonic acid was uniformly dispersed to obtain a dispersion solution, and the average particle size of 50% by volume of the resulting dispersion solution was 0.017 µm. 8-anilino-1-naphthalene sulfonic acid did not usually emit fluorescence in water, but the obtained dispersion solution was diluted 100 times with water and irradiated with an excitation light of 360 nm and as a result, strong fluorescence in the vicinity of 450 to 510 nm was observed.

### Example A18

0.005 weight parts of a dye (trade name: HS-296, a product of Mitsui Chemicals, Inc.) was dissolved in 1.6 weight parts of isopropyl alcohol, and then added to 10 weight parts of the dispersion solution obtained in Example A1, and the contents were stirred at room temperature using a magnetic stirrer. The dye was uniformly dispersed to obtain a dispersion solution. Although the obtained dispersion solution was allowed to stand at room temperature for 1 month, aggregation and precipitation did not occur.

### Example A19

0.005 weight parts of pyrene was dissolved in 1.6 weight parts of acetone, and then added to 10 weight parts of the dispersion solution obtained in Example A1, and the contents were stirred at room temperature using a magnetic stirrer. Pyrene was uniformly dispersed to obtain a dispersion solution. Although the obtained dispersion solution was allowed to stand at room temperature for 1 month, aggregation and precipitation did not occur. The obtained dispersion solution was diluted 100 times with water and irradiated with an excitation light of 350 nm and as a result, strong fluorescence in the vicinity of 370 to 410 nm was observed.

### Example A20

0.01 weight part of 8-anilino-1-naphthalene sulfonic acid was dissolved in 10 weight parts of water, and then added to 10 weight parts of the dispersion solution obtained in Example A1, and the contents were stirred at room temperature using a magnetic stirrer. 8-anilino-1-naphthalene sulfonic acid was uniformly dispersed to obtain a dispersion solution. Although the obtained dispersion solution was allowed to stand at room temperature for 1 month, aggregation and precipitation did not occur. The obtained dispersion solution was diluted 50 times with water and irradiated with an excitation light of 360 nm and as a result, strong fluorescence in the vicinity of 450 to 510 nm was observed.

### Example A21

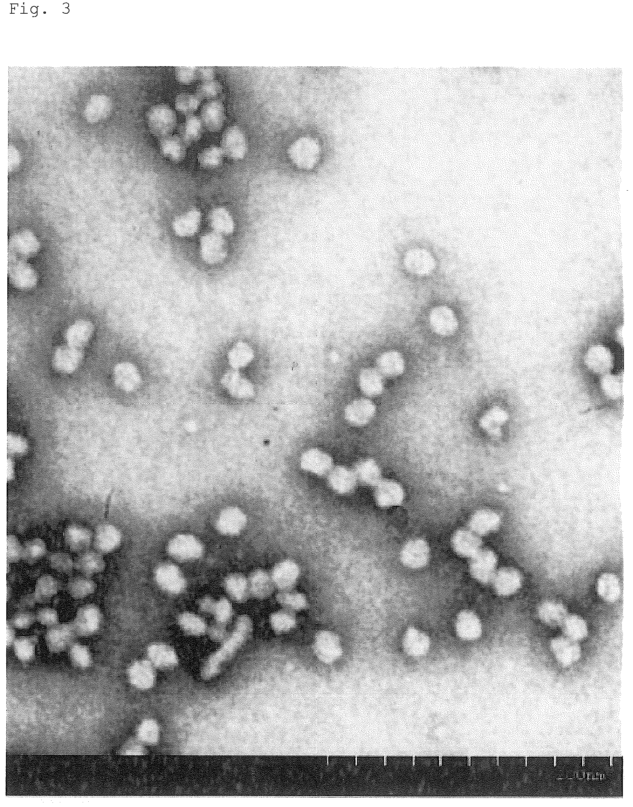

10 weight parts of the dispersion solution obtained in Example A1 was introduced into a 50-ml eggplant flask and frozen with liquid nitrogen. The resulting material was installed at a freeze dryer (FDU-2200, a product of Tokyo Rika Kogyo Co., Ltd.) and dried, whereby powdered particles were obtained. 8 weight parts of water was added to the resulting particles and the contents were stirred at room temperature using a magnetic stirrer. The average particle size of 50% by volume of the dispersion solution re-dispersed in water was 0.030 µm (average particle size of 10% by volume: 0.022 µm, average particle size of 90% by volume: 0.078 µm). Although the aforementioned dispersion solution was allowed to stand at room temperature for 1 month, aggregation and precipitation did not occur. An observation view of the aforementioned dispersion solution using a transmission electron microscope is illustrated in Fig. 3. Incidentally, the particle size measured from Fig. 3 was from 0.015 to 0.025 µm. Further, the melting point (Tm) of the obtained particles was -31 degrees centigrade (polyethylene glycol), 118 degrees centigrade.

### Example A22

8 weight parts of methanol was added to the particles prepared in the same manner as in Example A21, and the contents were stirred at room temperature using a magnetic stirrer. The average particle size of 50% by volume of the dispersion solution dispersed in methanol was 0.038 µm (average particle size of 10% by volume: 0.025 µm, average particle size of 90% by volume: 0.067 µm). Although the aforementioned dispersion solution was allowed to stand at room temperature for 1 month, aggregation and precipitation did not occur.

### Example A23

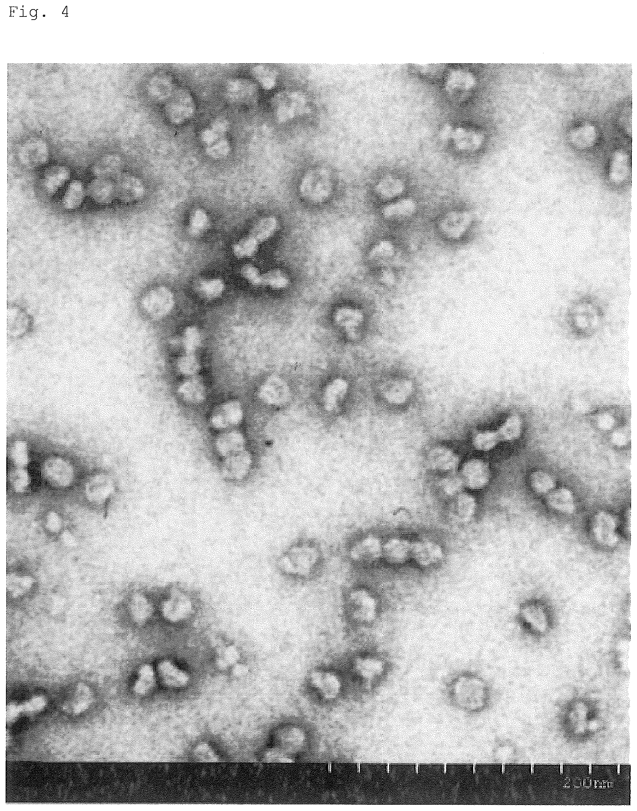

8 weight parts of ethanol was added to the particles prepared in the same manner as in Example A21, and the contents were stirred at room temperature using a magnetic stirrer. The average particle size of 50% by volume of the dispersion solution dispersed in ethanol was 0. 057 µm (average particle size of 10% by volume: 0.036 µm, average particle size of 90% by volume: 0.094 µm). Although the aforementioned dispersion solution was allowed to stand at room temperature for 1 month, aggregation and precipitation did not occur. An observation view of the aforementioned dispersion solution using a transmission electron microscope is illustrated in Fig. 4. Incidentally, the particle size measured from Fig. 4 was from 0.010 to 0.025 µm.

### Example A24

10 weight parts of the terminally branched copolymer (T-5) obtained in Synthesis Example A5 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.017 µm (average particle size of 10% by volume: 0.013 µm, average particle size of 90% by volume: 0.024 µm). Further, the zeta potential of the obtained dispersion solution diluted 100 times with water was -1.2 mV.

### Example A25

When 0.26 weight parts of an aqueous 10% sulfuric acid solution was added to 10.0 weight parts of the dispersion solution obtained in Example A24, the pH became 7. Although the dispersion solution was allowed to stand at room temperature for 1 month, aggregation and precipitation did not occur. Further, the zeta potential of the obtained dispersion solution diluted 100 times with water was 15.0 mV.

### Example A26

10 weight parts of the terminally branched copolymer (T-6) obtained in Synthesis Example A6 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.019 µm (average particle size of 10% by volume: 0.014 µm, average particle size of 90% by volume: 0.049 µm).

### Example A27

10 weight parts of the terminally branched copolymer (T-7) obtained in Synthesis Example A7 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.018 µm (average particle size of 10% by volume: 0.014 µm, average particle size of 90% by volume: 0.025 µm).

### Example A28

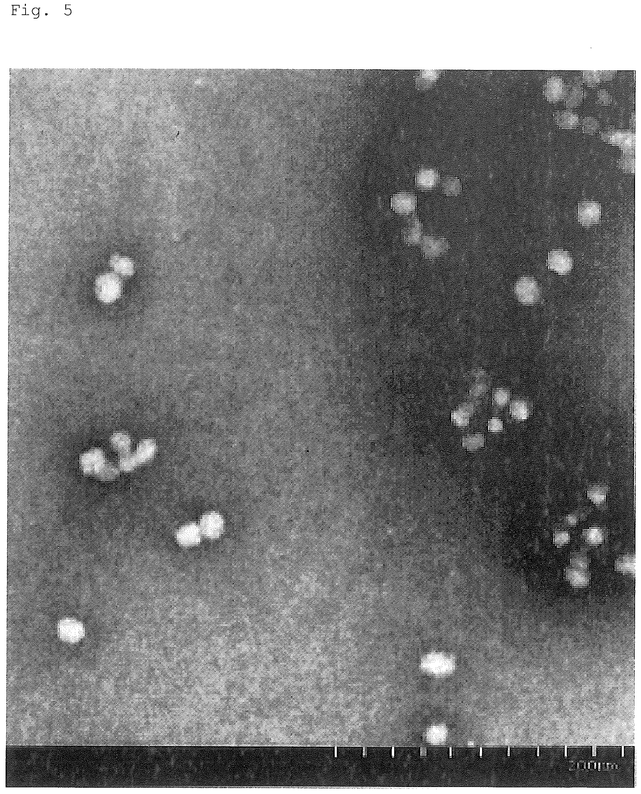

10 weight parts of the terminally branched copolymer (T-3) obtained in Synthesis Example A3 and 40 weight parts of ethylene glycol were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.024 µm. An observation view of the obtained dispersion solution using a transmission electron microscope is illustrated in Fig. 5. Incidentally, the particle size measured from Fig. 5 was from 0.015 to 0.030 µm.

### Example A29

10 weight parts of the terminally branched copolymer (T-5) obtained in Synthesis Example A5 and 40 weight parts of tetraethylene glycol were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.025 µm (average particle size of 10% by volume: 0.017 µm, average particle size of 90% by volume: 0.049 µm).

### Example A30

Powdered particles were obtained in the same manner as in Example A21, except that the dispersion solution in use was changed to the dispersion solution obtained in Example A2. The melting point (Tm) of the obtained particles was 42 degrees centigrade (polyethylene glycol), 117 degrees centigrade.

### Example A31

Powdered particles were obtained in the same manner as in Example A21, except that the dispersion solution in use was changed to the dispersion solution obtained in Example A3. The melting point (Tm) of the obtained particles was -36 degrees centigrade (polyethylene glycol), 102 degrees centigrade.

### Example A32

Powdered particles were obtained in the same manner as in Example A21, except that the dispersion solution in use was changed to the dispersion solution obtained in Example A24. The melting point (Tm) of the obtained particles was 12 degrees centigrade (polyethylene glycol), 117 degrees centigrade.

### Example A33

Powdered particles were obtained in the same manner as in Example A21, except that the dispersion solution in use was changed to the dispersion solution obtained in Example A26. The melting point (Tm) of the obtained particles was 52 degrees centigrade (polyethylene glycol), 120 degrees centigrade.

### Example A34

Powdered particles were obtained in the same manner as in Example A21, except that the dispersion solution in use was changed to the dispersion solution obtained in Example A27. The melting point (Tm) of the obtained particles was 14 degrees centigrade (polyethylene glycol), 95 degrees centigrade.

### Example A35

20 weight parts of the dispersion solution obtained in Example A1 was spray-dried using a spray dryer (Basic Unit Model GB21, a. product of Yamato Scientific Co., Ltd.). At this time, an inlet temperature was adjusted to 120 degrees centigrade, while an outlet temperature was adjusted to 50 degrees centigrade. 16 weight parts of water was added to the obtained particles and stirred at room temperature using a magnetic stirrer. The average particle size of 50% by volume of the dispersion solution re-dispersed in water was 0.030 µm. Although the aforementioned dispersion solution was allowed to stand at room temperature for 1 month, aggregation and precipitation did not occur.

### Example A36

15 weight parts of water was added to 10 weight parts of the dispersion solution (solid content: 19.5 weight %) obtained in Example A25, and the contents were stirred at room temperature. The composition prepared according to the above-described method was applied to a corona treated polyethylene terephthalate film having a thickness of 50 µm (ester film A4100, a product of Toyobo Co., Ltd.) using hard coating process, such that the thickness after curing was about 0.5 µm. Thereafter, the substrate was heated at 110 degrees centigrade for 2 minutes to prevent coloration or deformation of the substrate, thus to obtain a coating film.

The obtained coating film was allowed to stand in a thermostat-hygrostat chamber controlled to a temperature of 23 ± 2 degrees centigrade and a humidity of 50 ± 5%RH for 24 hours, and the surface resistance value was measured at an applied voltage of 500 V and as a result, it was 8.4 × 10⁸ Ω. Further, the contact angle of water was 26 degrees.

### Example A37

0.3 weight parts of water-dispersible isocyanate (Takenate WD-725, a product of Mitsui Takeda Chemicals Inc.) and 16 weight parts of water were added to 9 weight parts of the dispersion solution (solid content: 19.5 weight %) obtained in Example A25, and the contents were stirred at room temperature. The composition prepared according to the above-described method was applied in the same manner as in Example A36, heated at 110 degrees centigrade for 2 minutes, and then aged at 40 degrees centigrade for 48 hours, thus to obtain a coating film.

The obtained coating film was allowed to stand in a thermostat-hygrostat chamber controlled to a temperature of 23 ± 2 degrees centigrade and a humidity of 50 ± 5%RH for 24 hours, and the surface resistance value was measured at an applied voltage of 500 V and as a result, it was 9.5 × 10⁹ Ω. Further, the contact angle of water was 48 degrees.

### Example A38

4. 5 weight parts of an aqueous polyurethane resin (Takelac W6010, a product of Mitsui Chemicals Polyurethanes Inc.) prepared with the solid content of 19.5%, 0.3 weight parts of water-dispersible isocyanate (Takenate WD-725, a product of Mitsui Takeda Chemicals Inc.) and 16 weight parts of water were added to 4.5 weight parts of the dispersion solution (solid content: 19.5 weight %) obtained in Example A25, and the contents were stirred at room temperature. The composition prepared according to the above-described method was applied, heated and aged in the same manner as in Example A37, thus to obtain a coating film.

The obtained coating film was allowed to stand in a thermostat-hygrostat chamber controlled to a temperature of 23 ± 2 degrees centigrade and a humidity of 50 ± 5%RH for 24 hours, and the surface resistance value was measured at an applied voltage of 500 V and as a result, it was 6.6 × 10¹¹ Ω. Further, the contact angle of water was 59 degrees.

### Example A39

10 weight parts of the terminally branched copolymer (T-8) obtained in Synthesis Example A8 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.094 µm (average particle size of 10% by volume: 0.051 µm, average particle size of 90% by volume: 0.22 µm).

### Example A40

10 weight parts of the terminally branched copolymer (T-9) obtained in Synthesis Example A9 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.060 µm (average particle size of 10% by volume: 0.038 µm, average particle size of 90% by volume: 0.125 µm).

### Example A41

10 weight parts of the terminally branched copolymer (T-10) obtained in Synthesis Example A10 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.018 µm (average particle size of 10% by volume: 0.016 µm, average particle size of 90% by volume: 0.21 µm).

### Example A42

10 weight parts of the terminally branched copolymer (T-11) obtained in Synthesis Example A11 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.013 µm (average particle size of 10% by volume: 0.010 µm, average particle size of 90% by volume: 0.020 µm).

### Example A43

10 weight parts of the terminally branched copolymer (T-12) obtained in Synthesis Example A12 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.015 µm (average particle size of 10% by volume: 0.014 µm, average particle size of 90% by volume: 0.018 µm).

### Example A44

10 weight parts of the terminally branched copolymer (T-13) obtained in Synthesis Example A13 and 40 weight parts of distilled water were introduced into a 100-ml autoclave, heated with stirring at a rate of 800 rpm at 140 degrees centigrade for 30 minutes, and then cooled to room temperature while stirring. The average particle size of 50% by volume of the obtained dispersion solution was 0.021 µm (average particle size of 10% by volume: 0.014 µm, average particle size of 90% by volume: 0.47 µm).

### Example A45

10 weight parts of the terminally branched copolymer (T-5) obtained in Synthesis Example A5, 0.25 weight parts of carbon black (trade name: Carbon ECP, a product of Ketjen Black International Company) and 40 weight parts of distilled water were introduced into a 100-ml autoclave, heated with stirring at a rate of 800 rpm at 140 degrees centigrade for 30 minutes, and then cooled to room temperature while stirring. As a result, the above-described carbon black was uniformly dispersed.

### Example A46

A polypropylene pellet (J715M, a product of Prime Polymer Co., Ltd.) was subjected to extrusion molding under the conditions of a roll temperature of 40 degrees centigrade and a take-up speed of 0.5 m/min at an extruder set temperature of 210 degrees centigrade, using a single screw extruder of 20 mmϕ·L/D=28 (a product of Thermo Scientific) with a T die having a rip width of 250 mm and a degree of opening of 0.8 mm, whereby a film having a thickness of 40 µm was obtained. This film was subjected to plasma treatment under the conditions of a processing speed of 20 mm/min and an applied voltage pulse frequency of 30 kHz under an argon atmosphere using a normal pressure plasma treatment apparatus AP-T02-L (a product of Sekisui Chemical Co., Ltd.). A coating film was obtained in the same manner as in Example A36, except that the aforementioned extruded polypropylene film subjected to plasma treatment was used instead of the corona treated polyethylene terephthalate film of Example A36. The obtained coating film was allowed to stand in a thermostat-hygrostat chamber controlled to a temperature of 23 ± 2 degrees centigrade and a humidity of 50 ± 5%RH for 24 hours, and the surface resistance value was measured at an applied voltage of 500 V and as a result, it was 7.4 × 10⁸ Ω. Further, the contact angle of water was 23 degrees.

### Example A47

A coating film was obtained in the same manner as in Example A37, except that the extruded polypropylene film subjected to plasma treatment used in Example A46 was used instead of the corona treated polyethylene terephthalate film of Example A37. The obtained coating film was allowed to stand in a thermostat-hygrostat chamber controlled to a temperature of 23 ± 2 degrees centigrade and a humidity of 50 ± 5%RH for 24 hours, and the surface resistance value was measured at an applied voltage of 500 V and as a result, it was 3.6 × 10⁹ Ω. Further, the contact angle of water was 52 degrees.

### Example A48

A polypropylene pellet (F-300SP, a product of Prime Polymer Co. , Ltd.) was molded under the condition of an extruder set temperature of 250 degrees centigrade using an extruder with a T die of 30 mmϕ, whereby a polypropylene sheet having a thickness of 500 µm was obtained. The obtained sheet was preheated at 156 degrees centigrade for 1 minute, and then stretched in the machine direction 5 times and in the transverse direction 7 times at 156 degrees centigrade at a stretching rate of 6 m/min, whereby a stretched film having a thickness of 15 µm was obtained. This film was subjected to plasma treatment under the conditions of a processing speed of 20 mm/min and an applied voltage pulse frequency of 30 kHz under an argon atmosphere using a normal pressure plasma treatment apparatus AP-T02-L (a product of Sekisui Chemical Co., Ltd.). A coating film was obtained in the same manner as in Example A36, except that the aforementioned biaxially stretched polypropylene film subjected to plasma treatment was used instead of the corona treated polyethylene terephthalate film of Example A36.

The obtained coating film was allowed to stand in a thermostat-hygrostat chamber controlled to a temperature of 23 ± 2 degrees centigrade and a humidity of 50 ± 5%RH for 24 hours, and the surface resistance value was measured at an applied voltage of 500 V and as a result, it was 8.2 × 10⁸ Ω. Further, the contact angle of water was 25 degrees.

### Example A49

A coating film was obtained in the same manner as in Example A37, except that the plasma treated biaxially stretched polypropylene film used in Example A48 was used instead of the corona treated polyethylene terephthalate film of Example 37. The obtained coating film was allowed to stand in a thermostat-hygrostat chamber controlled to a temperature of 23 ± 2 degrees centigrade and a humidity of 50 ± 5%RH for 24 hours, and the surface resistance value was measured at an applied voltage of 500 V and as a result, it was 3.9 × 10⁹ Ω. Further, the contact angle of water was 51 degrees.

### Comparative Example A1

When water was used instead of the dispersion solution of Example A18, aggregation and precipitation of the dye occurred.

### Comparative Example A2

When water was used instead of the dispersion solution of Example A19, aggregation and precipitation of pyrene occurred. Further, fluorescence (region of 370 to 410 nm) corresponding to Example A19 was not observed.

### Comparative Example A3

When water was used instead of the dispersion solution of Example A20, fluorescence (region of 450 to 510 nm) corresponding to Example A20 was not observed.

### Comparative Example A4

A coating film was obtained by using an aqueous polyurethane resin (Takelac W6010, a product of Mitsui Chemicals Polyurethanes Inc.) prepared with the solid content of 19.5 weight % instead of the dispersion solution of Example A37. The obtained coating film was allowed to stand in a thermostat-hygrostat chamber controlled to a temperature of 23 ± 2 degrees centigrade and a humidity of 50 ± 5%RH for 24 hours, and the surface resistance value was measured at an applied voltage of 500 V and as a result, it was 7.7 × 10¹⁶ Ω. Further, the contact angle of water was 78 degrees.

### Comparative Example A5

When water was used instead of the terminally branched copolymer of Example A45, carbon black was not dispersed, and aggregation and precipitation occurred.

### Example B

Hereinafter, the present invention will be illustrated in more detail with reference to Example B.

### Synthesis Example B1

### Synthesis of Polyolefin Based Terminally Branched Copolymer (T-1)

In accordance with Synthesis Example 2 of Japanese Patent Laid-open No. 2006-131870, the epoxy-terminated ethylenic polymer (E-1) (Mw: 2,058, Mn: 1,118, Mw/Mn: 1.84 (GPC)) was synthesized and used as a raw material.
¹H-NMR : δ(C₂D₂Cl₄) 0.88 (t, 3H, J = 6.92 Hz), 1.18 - 1.66 (m), 2.38 (dd, 1H, J = 2.64, 5.28 Hz), 2.66 (dd, 1H, J = 4.29, 5.28 Hz) 2.80 - 2.87 (m, 1H)
Melting point (Tm): 121 degrees centigrade

84 weight parts of the epoxy-terminated ethylenic polymer (E-1), 39.4 weight parts of diethanolamine and 150 weight parts of toluene were introduced into a 1,000-mL flask, and stirred at 150 degrees centigrade for 4 hours. Thereafter, acetone was added while cooling the mixture to precipitate the reaction product, and the solid was collected by filtration. The obtained solid was stirred and washed with an aqueous acetone solution one time and further with acetone three times, and then the solid was collected by filtration. Thereafter, the solid was dried at room temperature under reduced pressure to obtain a polymer (I-1) (Mn: 1,223, in the general formula (9), A: a group formed by polymerization of ethylene (Mn: 1,075), R¹ and R²: a hydrogen atom, one of Y¹ and Y²: a hydroxyl group, the other of Y¹ and Y²: a bis (2-hydroxyethyl) amino group).
¹H-NMR : δ(C₂D₂Cl₄) 0.88 (t, 3H, J = 6.6 Hz), 0. 95 - 1.92 (m), 2.38 - 2.85 (m, 6H), 3.54 - 3.71 (m, 5H)
Melting point (Tm): 121 degrees centigrade

20.0 weight parts of the polymer (I-1) and 100 weight parts of toluene were introduced into a 500-mL flask equipped with a nitrogen inlet tube, a thermometer, a condenser and a stirring device, and heated in an oil bath at 125 degrees centigrade with stirring to completely dissolve the solid. After cooling to 90 degrees centigrade, 0.323 weight parts of 85% KOH that had been dissolved in 5.0 weight parts of water in advance was added to the flask, and the contents were mixed under reflux for 2 hours. Subsequently, the temperature in the flask was slowly increased to 120 degrees centigrade, and water and toluene were distilled off. Furthermore, water and toluene in the flask were completely distilled off by reducing the pressure in the flask while supplying minimal nitrogen into the flask, increasing the internal temperature to 150 degrees centigrade, and then keeping the temperature for 4 hours. After cooling to room temperature, the solid solidified in the flask was broken and taken out.

18.0 weight parts of the obtained solid and 200 weight parts of dehydrated toluene were introduced into a 1.5-L stainless steel pressurized reactor equipped with a heating device, a stirring device, a thermometer, a manometer and a safety valve, and after purging the gas phase with nitrogen, the contents were heated to 130 degrees centigrade with stirring. After 30 minutes, 9.0 weight parts of ethylene oxide was added thereto. After further maintaining at 130 degrees centigrade for 5 hours, the contents were cooled to room temperature to obtain a reactant. The solvent was removed by drying from the resulting reactant to obtain a terminally branched copolymer (T-1) (Mn: 1,835, in the general formula (1), A: a group formed by polymerization of ethylene (Mn: 1,075), R¹ and R²: a hydrogen atom, one of X¹ and X²: a group represented by the general formula (6) (X¹¹: a polyethylene glycol group), the other of X¹ and X²: a group represented by the general formula (5) (Q¹=Q²: an ethylene group, X⁹=X¹⁰: a polyethylene glycol group)).
¹H-NMR : δ(C₂D₂Cl₄) 0.88 (3H, t, J= 6.8 Hz), 1.06 -1.50 (m), 2. 80 - 3.20 (m), 3.33 - 3.72 (m)
Melting point (Tm): 116 degrees centigrade

### Synthesis Example B2

A terminally branched copolymer (T-2) (Mn: 2,446) was obtained in the same manner as in Synthesis Example B1, except that the amount of ethylene oxide in use was changed to 18.0 weight parts.

### Synthesis Example B3

A terminally branched copolymer (T-3) (Mn: 3,669) was obtained in the same manner as in Synthesis Example B1, except that the amount of ethylene oxide in use was changed to 36.0 weight parts.

### Synthesis Example B4

A terminally branched copolymer (T-4) (Mn: 6,115) was obtained in the same manner as in Synthesis Example B1, except that the amount of ethylene oxide in use was changed to 72.0 weight parts.

### Example B1

### Preparation of 5 weight % Polyolefin Based Terminally Branched Copolymer (T-1) Aqueous Dispersion Solution

10 weight parts of the polyolefin based terminally branched copolymer (T-1) constituting the polymer particles (A) of Synthesis Example B1 and 40 weight parts of distilled water as the solvent (C) were introduced into a 100-ml autoclave, heated with stirring at a rate of 800 rpm at 140 degrees centigrade for 30 minutes, and then cooled to room temperature while stirring. Furthermore, 225 weight parts of distilled water was added to 75 weight parts of this T-1 aqueous dispersion solution (solid content: 20 weight %), whereby a 5 weight % T-1 aqueous dispersion solution was obtained.

### Preparation of Mixed Composition

15 weight parts of methanol as the solvent (C) was added to 10 weight parts of tetramethoxysilane (TMOS) as the component (B), and stirred at room temperature. Furthermore, 15 weight parts of 0.1N hydrochloric acid as the catalyst (D) was added dropwise thereto, and then stirred at room temperature for 1.5 hours. Thereafter, 60 weight parts of the 5 weight % T-1 aqueous dispersion solution containing the copolymer was added thereto, and stirred at room temperature for 5 minutes to give a solution e11. On the other hand, 15 weight parts of methanol as the solvent (C) was added to 10 weight parts of tetramethoxysilane (TMOS) as the component (B), and stirred at room temperature. Thereafter, 10 weight parts of 0.1N hydrochloric acid as the catalyst (D) was added dropwise thereto, and stirred at room temperature for 1 hour to give a solution e12. Incidentally, the solutions e11 and e12 are each a solution containing the components (B) and (D).

The solutions e11 and e12 were mixed at a weight ratio of 8/2, and further stirred at room temperature for 5 minutes to obtain a composition.

### Preparation of Laminate

The composition prepared according to the above-described method was applied to an Al₂O₃ deposited PET film having a thickness of 12 µm (TL-PET, a product of Tohcello Co., Ltd.) using hard coating process, such that the thickness after curing was about 0.5 µm. Thereafter, the substrate was heated at 110 degrees centigrade for 1.5 hours to prevent coloration or deformation of the substrate, thus to obtain a laminate.

Incidentally, also in the following Examples B2 and B3, the 5 weight % T-1 aqueous dispersion solution containing a copolymer, and the solutions e11 and e12 were used in the same manner as in Example B1.

### Example B2

The solutions e11 and e12 were mixed at a weight ratio of 7/3, and further stirred at room temperature for 5 minutes to obtain a mixed composition. This composition was applied to an Al₂O₃ deposited PET film having a thickness of 12 µm (TL-PET, a product of Tohcello Co., Ltd.) in the same manner as in Example B1, such that the thickness was about 0.5 µm, and the contents were heated at 110 degrees centigrade for 1.5 hours, thus to obtain a laminate.

### Example B3

The solutions e11 and e12 were mixed at a weight ratio of 6/4, and further stirred at room temperature for 5 minutes to obtain a mixed composition. This composition was applied to an Al₂O₃ deposited PET film having a thickness of 12 µm (TL-PET, a product of Tohcello Co., Ltd.) in the same manner as in Example B1, such that the thickness was about 0.5 µm, and the contents were heated at 110 degrees centigrade for 1.5 hours, thus to obtain a laminate.

### Example B4

625 weight parts of 0.1N hydrochloric acid was further added to 375 weight parts of the T-1 aqueous dispersion solution (solid content: 20 weight %) containing the copolymer to acidify, whereby a 7.5 weight % T-1 aqueous dispersion solution (pH=3) was obtained. 15 weight parts of methanol as the solvent (C) was added to 10 weight parts of tetramethoxysilane (TMOS) as the component (B), and stirred at room temperature. Furthermore, 15 weight parts of 0.1N hydrochloric acid as the catalyst (D) was added dropwise thereto and then stirred at room temperature for 1.5 hours to give a solution e4. Incidentally, the solution e4 is a solution containing the components (B) and (D).

Thereafter, 10.5 weight parts of the solution e4 was added to 150 weight parts of the 7.5 weight % T-1 aqueous dispersion solution containing a copolymer, and stirred at room temperature for 5 minutes to give a mixed composition.

The mixed composition prepared according to the above-described method was applied to a glass substrate having a thickness of 1 mm (MICRO SLIDE GLASS, a product of Matsunami Glass Ind., Ltd.) and a PET (polyethylene terephthalate) substrate having a thickness of 50 µm (Cosmoshine A4100, a product of Toyobo Co. , Ltd.) using an applicator, such that the thickness after curing was about 1 µm. Thereafter, the substrate was heated at 110 degrees centigrade for 30 minutes to prevent coloration or deformation of the substrate, thus to obtain a laminate.

Incidentally, also in the following Examples B5 to B8, the 7.5 weight % T-1 aqueous dispersion solution containing the copolymer and the solution e4 were used in the same manner as in Example B4.

### Example B5

26. 3 weight parts of the solution e4 was added to 150 weight parts of the 7.5 weight % T-1 aqueous dispersion solution containing a copolymer, and stirred at room temperature for 5 minutes, thus to obtain a mixed composition.

The composition prepared according to the above-described method was applied to a glass substrate having a thickness of 1 mm (MICRO SLIDE GLASS, a product of Matsunami Glass Ind., Ltd.) and a PET (polyethylene terephthalate) substrate having a thickness of 50 µm (Cosmoshine A4100, a product of Toyobo Co. , Ltd.) using an applicator, such that the thickness after curing was about 1 µm. Thereafter, the substrate was heated at 110 degrees centigrade for 30 minutes to prevent coloration or deformation of the substrate, thus to obtain a laminate.

### Example B6

43.8 weight parts of the solution e4 was added to 150 weight parts of the 7.5 weight % T-1 aqueous dispersion solution containing the copolymer, and the mixture was stirred at room temperature for 5 minutes, thus to obtain a mixed composition.

The mixed composition prepared according to the above-described method was applied to a glass substrate having a thickness of 1 mm (MICRO SLIDE GLASS, a product of Matsunami Glass Ind., Ltd.) and a PET (polyethylene terephthalate) substrate having a thickness of 50 µm (Cosmoshine A4100, a product of Toyobo Co. , Ltd.) using an applicator, such that the thickness after curing was about 1 µm. Thereafter, the substrate was heated at 110 degrees centigrade for 30 minutes to prevent coloration or deformation of the substrate, thus to obtain a laminate.

### Example B7

70 weight parts of the solution e4 was added to 150 weight parts of the 7.5 weight % T-1 aqueous dispersion solution containing the copolymer, and the mixture was stirred at room temperature for 5 minutes, thus to obtain a mixed composition.

The mixed composition prepared according to the above-described method was applied to a glass substrate having a thickness of 1 mm (MICRO SLIDE GLASS, a product of Matsunami Glass Ind., Ltd.) and a PET (polyethylene terephthalate) substrate having a thickness of 50 µm (Cosmoshine A4100, a product of Toyobo Co. , Ltd.) using an applicator, such that the thickness after curing was about 1 µm. Thereafter, the substrate was heated at 110 degrees centigrade for 30 minutes to prevent coloration or deformation of the substrate, thus to obtain a laminate.

### Example B8

105 weight parts of the solution e4 was added to 150 weight parts of the 7.5 weight % T-1 aqueous dispersion solution containing the copolymer, and the mixture was stirred at room temperature for 5 minutes, thus to obtain a mixed composition.

The mixed composition prepared according to the above-described method was applied to a glass substrate having a thickness of 1 mm (MICRO SLIDE GLASS, a product of Matsunami Glass Ind., Ltd.) and a PET (polyethylene terephthalate) substrate having a thickness of 50 µm (Cosmoshine A4100, a product of Toyobo Co. , Ltd.) using an applicator, such that the thickness after curing was about 1 µm. Thereafter, the substrate was heated at 110 degrees centigrade for 30 minutes to prevent coloration or deformation of the substrate, thus to obtain a laminate.

### Example B9

### Preparation of 5 weight % Polyolefin Based Terminally Branched Copolymer (T-2) Aqueous Dispersion Solution

10 weight parts of the polyolefin based terminally branched copolymer (T-2) constituting the polymer particles (A) of Synthesis Example B2 and 40 weight parts of distilled water as the solvent (C) were introduced into a 100-ml autoclave, heated with stirring at a rate of 800 rpm at 140 degrees centigrade for 30 minutes, and then cooled to room temperature while stirring. Furthermore, 225 weight parts of distilled water was added to 75 weight parts of this T-2 aqueous dispersion solution (solid content: 20 weight %), whereby a 5 weight % T-2 aqueous dispersion solution was obtained.

### Preparation of Mixed Composition and Preparation of Laminate

15 weight parts of methanol as the solvent (C) was added to 10 weight parts of tetramethoxysilane (TMOS) as the component (B), and stirred at room temperature. Furthermore, 15 weight parts of 0.1N hydrochloric acid as the catalyst (D) was added dropwise thereto, and then stirred at room temperature for 1.5 hours. Thereafter, 60 weight parts of the 5 weight % T-2 aqueous dispersion solution containing the copolymer was added thereto, and stirred at room temperature for 5 minutes to give a solution e91. On the other hand, 15 weight parts of methanol as the solvent (C) was added to 10 weight parts of tetramethoxysilane (TMOS) as the component (B), and stirred at room temperature. Thereafter, 10 weight parts of 0.1N hydrochloric acid as the catalyst (D) was added dropwise thereto, and stirred at room temperature for 1 hour to give a solution e92. Incidentally, the solutions e91 and e92 are each a solution containing the components (B) and (D).

The solutions e91 and e92 were mixed at a weight ratio of 7/3, and further stirred at room temperature for 5 minutes to obtain a mixed composition. This composition was applied to an Al₂O₃ deposited PET film having a thickness of 12 µm (TL-PET, a product of Tohcello Co., Ltd.) in the same manner as in Example B1, such that the thickness was about 0.5 µm, and the contents were heated at 110 degrees centigrade for 1.5 hours, thus to obtain a laminate.

### Example B10

The solutions e91 and e92 were mixed at a weight ratio of 6/4, and further stirred at room temperature for 5 minutes to obtain a mixed composition. This composition was applied to an Al₂O₃ deposited PET film having a thickness of 12 µm (TL-PET, a product of Tohcello Co., Ltd.) in the same manner as in Example B1, such that the thickness was about 0.5 µm, and the contents were heated at 110 degrees centigrade for 1.5 hours, thus to obtain a laminate.

### Example B11

### Preparation of 5 weight % Polyolefin Based Terminally Branched Copolymer (T-3) Aqueous Dispersion Solution

10 weight parts of the polyolefin based terminally branched copolymer (T-3) of Synthesis Example B3 and 40 weight parts of distilled water as the solvent (C) were introduced into a 100-ml autoclave, heated with stirring at a rate of 800 rpm at 140 degrees centigrade for 30 minutes, and then cooled to room temperature while stirring. Furthermore, 225 weight parts of distilled water was added to 75 weight parts of this T-3 aqueous dispersion solution (solid content: 20 weight %), whereby a 5 weight % T-3 aqueous dispersion solution was obtained.

### Preparation of Composition and Preparation of Laminate

15 weight parts of methanol as the solvent (C) was added to 10 weight parts of tetramethoxysilane (TMOS) as the component (B), and stirred at room temperature. Furthermore, 15 weight parts of 0.1N hydrochloric acid as the catalyst (D) was added dropwise thereto, and then stirred at room temperature for 1.5 hours. Thereafter, 60 weight parts of the 5 weight % T-3 aqueous dispersion solution containing the copolymer was added thereto, and stirred at room temperature for 5 minutes to give a solution e111. On the other hand, 15 weight parts of methanol as the solvent (C) was added to 10 weight parts of tetramethoxysilane (TMOS) as the component (B), and stirred at room temperature. Thereafter, 10 weight parts of 0.1N hydrochloric acid as the catalyst (D) was added dropwise thereto, and stirred at room temperature for 1 hour to give a solution e112. Incidentally, the solutions e111 and e112 are each a solution containing the components (B) and (D).

The solutions e111 and e112 were mixed at a weight ratio of 7/3, and further stirred at room temperature for 5 minutes to obtain a mixed composition. This composition was applied to an Al₂O₃ deposited PET film having a thickness of 12 µm (TL-PET, a product of Tohcello Co., Ltd.) in the same manner as in Example B1, such that the thickness was about 0.5 µm, and the contents were heated at 110 degrees centigrade for 1.5 hours, thus to obtain a laminate.

### Example B12

The solutions e111 and e112 were mixed at a weight ratio of 6/4, and further stirred at room temperature for 5 minutes to obtain a mixed composition. This composition was applied to an Al₂O₃ deposited PET film having a thickness of 12 µm (TL-PET, a product of Tohcello Co., Ltd.) in the same manner as in Example B1, such that the thickness was about 0.5 µm, and the contents were heated at 110 degrees centigrade for 1.5 hours, thus to obtain a laminate.

### Example B13

### Preparation of 5 weight % Polyolefin Based Terminally Branched Copolymer (T-4) Aqueous Dispersion Solution

10 weight parts of the polyolefin based terminally branched copolymer (T-4) of Synthesis Example B4 and 40 weight parts of distilled water as the solvent (C) were introduced into a 100-ml autoclave, heated with stirring at a rate of 800 rpm at 140 degrees centigrade for 30 minutes, and then cooled to room temperature while stirring. Furthermore, 225 weight parts of distilled water was added to 75 weight parts of this T-4 aqueous dispersion solution (solid content: 20 weight %), whereby a 5 weight % T-4 aqueous dispersion solution was obtained.

### Preparation of Mixed Composition and Preparation of Laminate

15 weight parts of methanol as the solvent (C) was added to 10 weight parts of tetramethoxysilane (TMOS) as the component (B), and stirred at room temperature. Furthermore, 15 weight parts of 0.1N hydrochloric acid as the catalyst (D) was added dropwise thereto, and then stirred at room temperature for 1.5 hours. Thereafter, 60 weight parts of the 5 weight % T-4 aqueous dispersion solution containing the copolymer was added thereto, and stirred at room temperature for 5 minutes to give a solution e131. On the other hand, 15 weight parts of methanol as the solvent (C) was added to 10 weight parts of tetramethoxysilane (TMOS) as the component (B), and stirred at room temperature. Thereafter, 10 weight parts of 0.1N hydrochloric acid as the catalyst (D) was added dropwise thereto, and stirred at room temperature for 1 hour to give a solution e132. Incidentally, the solutions e131 and e132 are each a solution containing the components (B) and (D).

The solutions e131 and e132 were mixed at a weight ratio of 5/3, and further stirred at room temperature for 5 minutes to obtain a mixed composition. This composition was applied to an Al₂O₃ deposited PET film having a thickness of 12 µm (TL-PET, a product of Tohcello Co., Ltd.) in the same manner as in Example B1, such that the thickness was about 0.5 µm, and the contents were heated at 110 degrees centigrade for 1.5 hours, thus to obtain a laminate.

### Example B14

The solutions e131 and e132 were mixed at a weight ratio of 5/4, and further stirred at room temperature for 5 minutes to obtain a mixed composition. This composition was applied to an Al₂O₃ deposited PET film having a thickness of 12 µm (TL-PET, a product of Tohcello Co., Ltd.) in the same manner as in Example B1, such that the thickness was about 0.5 µm, and the contents were heated at 110 degrees centigrade for 1.5 hours, thus to obtain a laminate.

### Example B15

100 weight parts of distilled water was further added to 100 weight parts of the aqueous dispersion solution (solid content: 20 weight %) of the polyolefin based terminally branched copolymer (T-1) constituting the polymer particles (A) of Synthesis Example B1, whereby a 10 weight % T-1 aqueous dispersion solution was prepared.

This aqueous dispersion solution was applied to an Al₂O₃ deposited PET film having a thickness of 12 µm (TL-PET, a product of Tohcello Co., Ltd.) in the same manner as in Example B1, such that the thickness was about 0.5 µm, except that this aqueous dispersion solution was used instead of the solutions e11 and e12 containing components corresponding to the components (B) and (D), and the contents were heated at 110 degrees centigrade for 2 minutes, thus to obtain a laminate.

### Example B16

625 weight parts of 0.1N hydrochloric acid was added to 375 weight parts of the aqueous dispersion solution (solid content: 20 weight %) of the polyolefin based terminally branched copolymer (T-1) of Example B15 to acidify, whereby a 7.5 weight % T-1 aqueous dispersion solution (pH=3) was obtained.

This aqueous dispersion solution was applied to a glass substrate having a thickness of 1 mm (MICRO SLIDE GLASS, a product of Matsunami Glass Ind., Ltd.) and a PET (polyethylene terephthalate) substrate having a thickness of 50 µm (Cosmoshine A4100, a product of Toyobo Co., Ltd.) in the same manner as in Example B4, such that the thickness was about 1 µm, except that this aqueous dispersion solution was used instead of the solution e4 containing components corresponding to the components (B) and (D), and the contents were heated at 110 degrees centigrade for 30 minutes, thus to obtain a laminate.

Combinations of the components in the above Example B are illustrated in Tables 1 and 3 along with the contents of SiO₂ (silica). Incidentally, the content of silica refers to the ratio of silica contained in the laminate, and it is calculated in the following method.

### Method of Calculating Content of Silica (SiO₂)

The content of silica was calculated on the assumption that 100 weight % of TMOS as the component (B) in the above Example B was reacted to be SiO₂. For example, when the component (B) was TMOS, 100% thereof was reacted to be SiO₂. That is, SiO₂/TMOS (60/152=0.395) was calculated from TMOS (Mw=152) and SiO₂ (Mw=60). Namely, a value obtained by multiplying the amount of TMOS by 0. 395 is the content of SiO₂ in the film.

For example, in case of Example B1, the content of silica (SiO₂) is as follows.
Content of silica (SiO₂) in the prepared solution = (10×0.395)/100×8+(10×0.395)/35×2 = 0.54
Content of emulsion particles in the prepared solution = (60×0.05)/100×8 = 0.24
Content of silica (SiO₂) (weight %) = 0.54/(0.54+0.24)×100 = 69.

### Evaluation of Coating Film

Physical properties of the laminate in the above Example B were evaluated in the following method. Evaluation results are shown in Tables 1 to 3.

### Transparency

A sample obtained by laminating a coating film having a thickness of 0.5 µm on an Al₂O₃ deposited PET film (TL-PET, a product of Tohcello Co., Ltd.) was used for an evaluation with naked eyes. Being transparent as used herein refers to the status that light is not scattered to cause whitening. In addition, for all samples, coloration and deformation of the laminate were not recognized.

### Measurement of Particle Size

A laminate sample applied to an Al₂O₃ deposited PET substrate (TL-PET, a product of Tohcello Co., Ltd.) or a PET substrate (Cosmoshine A4100, a product of Toyobo Co., Ltd.) was cut into pieces by focused ion beam (FIB) processing. Subsequently, the shape of the cross section of this film was observed by a transmission electron microscope (JEM-2200FS, a product of JEOL Ltd.) to calculate the particle size of the polyolefin based terminally branched copolymer microparticles.

### Measurement of Oxygen Permeability

### Examples B1 to B3, and B9 to B15

The permeability of oxygen was measured by using an oxygen permeability measuring apparatus (OXTRAN 2/21MH, a product of MOCON, Inc.) under atmosphere of a temperature of 23 degrees centigrade and a humidity of 90%RH. For the measurement, the sample subjected to coating to an Al₂O₃ deposited PET film (TL-PET, a product of Tohcello Co., Ltd.) was used.

### Abrasion Resistance Test

### Examples B2 and B15

The laminate surface was rubbed back and forth 30 times by applying a load of 600 weight parts using a steel wool (No.0000), and then existence of scratches on the surface of the film was visually confirmed.

### Evaluation of Water Resistance

### Examples B4 to B8 and B16

Water resistance was evaluated in accordance with JIS K 5400-8.19. The sample obtained by laminating a laminate having a thickness of 1 µm on the glass substrate or the PET film was dipped in distilled water and allowed to stand at a water temperature of 20 ± 2 degrees centigrade for 18 hours. After 18 hours, the sample was taken out and dried using a dryer at 100 degrees centigrade for 2 hours, and then the weight of the sample and the change in the haze were measured.

The laminates prepared in Examples B1 to B14 caused no cracks at all, thus to obtain transparent and smooth films.

Furthermore, the cross sections of the laminates were observed with a transmission electron microscope, from which the particle sizes of the polyolefin based terminally branched copolymers were from 10 to 20 m.

From the evaluation results shown in Table 1, it was found that the laminate using a gas barrier mixed composition having the polyolefin based terminally branched copolymer of Examples B1 to B3 and B9 to B14, metal alkoxide and/or a hydrolysis condensate thereof (B), water and/or the solvent for dissolving a part of water or entire water in any proportions (C), and the catalyst to be used for the sol-gel reaction (D) as main components had high transparency and gas barrier properties under high humidity (90%RH).

Meanwhile, as in Table 1, the combined amount of metal alkoxide and/or a hydrolysis condensate thereof as the component (B) was increased, and the content of silica was increased, whereby the gas barrier properties of the obtained laminate could be further improved.

On the other hand, as in Table 1, the laminate (Example B15) composed only of the polyolefin based terminally branched copolymer had lower gas barrier properties under high humidity (90%RH), as compared to Examples B1 to B14.

In addition, as in Table 2, abrasion resistance was tested for the gas barrier composition having the polyolefin based terminally branched copolymer of Example B2, metal alkoxide and/or a hydrolysis condensate thereof (B), water and/or the solvent for dissolving a part of water or entire water in any proportions (C), and the catalyst to be used for the sol-gel reaction (D) as main components. Then, it was found that abrasion resistance was excellent such that any scratches could not be visually recognized on the surface of the laminate.

On the other hand, as in Table 2, the laminate (Example B15) composed only of the polyolefin based terminally branched copolymer had lower abrasion resistance, as compared to Examples B1 to B14, and scratches were found on its surface.

From the evaluation results of water resistance shown in Tables 3-1 and 3-2, for the laminate using the mixed composition having the polymer particles (A) composed of the polyolefin based terminally branched copolymer of Examples B4 to B8, metal alkoxide and/or a hydrolysis condensate thereof (B), water and/or the solvent for dissolving a part of water or entire water in any proportions (C), and the catalyst to be used for the sol-gel reaction (D) as main components, the change in the weight caused by immersion into water was hardly found. Furthermore, it was found that haze was not changed and water resistance was high in Examples B5 to B8.

On the other hand, as in Tables 3-1 and 3-2, for the coating film (Example B16) composed only of the polyolefin based terminally branched copolymer, it was found that the weight caused by immersion into water and haze were changed, and water resistance was low.

**[Table 1]**

| | Component A | Component B | Silica Content (wt%) | Oxygen Permeability (cc/m²/day,atm) |
|---|---|---|---|---|
| Base material ^{*1} | - | - | - | 3.7 |
| Example B1 | T1 | TMOS | 69 | 3.3 |
| Example B2 | T1 | TMOS | 74 | 1.3 |
| Example B3 | T1 | TMOS | 79 | 0.7 |
| Example B9 | T2 | TMOS | 74 | 0.7 |
| Example B10 | T2 | TMOS | 79 | 0.5 |
| Example B11 | T3 | TMOS | 74 | 0.6 |
| Example B12 | T3 | TMOS | 79 | 0.5 |
| Example B13 | T4 | TMOS | 74 | 0.6 |
| Example B14 | T4 | TMOS | 82 | 0.4 |
| Example B15 | T1 | - | - | 4.6 |

| | | | | |
|---|---|---|---|---|
| *1 indicates a base material, i.e., an Al₂O₃ deposited PET (TL-PET, a product of Tohcello Co., Ltd.). | | | | |

**[Table 2]**

| | Example B2 | Example B15 |
|---|---|---|
| Existence of scratch | No | Yes |

**[Table 3-1]**

| | Unit | Example B4 | Example B5 | Example B6 |
|---|---|---|---|---|
| Silica Content | Wt% | 10 | 20 | 30 |
| Weight Reduction Rate *1 | % | 0.01 or less | 0.01 or less | 0.01 or less |
| Haze *2 | - | 1.4 | 0.4 | 0.4 |

**[Table 3-2]**

| | Unit | Example B7 | Example B8 | Example B16 |
|---|---|---|---|---|
| Silica Content | Wt% | 40 | 50 | 0 |
| Weight Reduction Rate *1 | % | 0.01 or less | 0.01 or less | 0.03 |
| Haze *2 | - | 0.4 | 0.4 | 2.2 |

| | | | | |
|---|---|---|---|---|
| *1 indicates the weight change rate of the laminate (base material: glass substrate) after water resistance test. *2 indicates the haze value of the laminate (base material: PET film) after water resistance test. All haze values before water resistance test were 0.4. | | | | |

### Example B17

15 weight parts of methanol was added to 10 weight parts of tetramethoxysilane (TMOS) as the component (B), and stirred at room temperature. Furthermore, 15 weight parts of 0.1N hydrochloric acid was added dropwise thereto, and then stirred at room temperature for 1.5 hours. Thereafter, 60 weight parts of the 5 weight % T-4 aqueous dispersion was added thereto, and stirred at room temperature for 5 minutes. Subsequently, 20 weight parts of an aqueous solution of lithium trifluoromethanesulfonate (LiSO₃CF₃) (LiSO₃CF₃ in the aqueous solution: 12.5 × 10⁻² weight parts) was added dropwise, such that the molar ratio of Li⁺/PEO in the solution was 0.05, and further stirred to give a solution e171.

On the other hand, 15 weight parts of methanol was added to 10 weight parts of tetramethoxysilane (TMOS) as the component (B), and stirred at room temperature. Thereafter, 10 weight parts of 0.1N hydrochloric acid was added dropwise, and stirred at room temperature for 1 hour to give a solution e172.

The solutions e171 and e172 were mixed at a weight ratio of 7/3, and further stirred at room temperature for 5 minutes to obtain a composition. This composition was applied to an Al₂O₃ deposited PET film having a thickness of 12 µm (TL-PET, a product of Tohcello Co., Ltd.) in the same manner as in Example B1, such that the thickness was about 0.5 µm, and the contents were heated at 110 degrees centigrade for 1.5 hours, thus to obtain a coating film.

### Example B18

15 weight parts of methanol was added to 10 weight parts of tetramethoxysilane (TMOS) as the component (B), and stirred at room temperature. Furthermore, 15 weight parts of 0.1N hydrochloric acid was added dropwise thereto, and then stirred at room temperature for 1.5 hours. Thereafter, 60 weight parts of the 5 weight % T-4 aqueous dispersion was added thereto, and stirred at room temperature for 5 minutes. Subsequently, 20 weight parts of an aqueous solution of lithium trifluoromethanesulfonate (LiSO₃CF₃) (LiSO₃CF₃ in the aqueous solution: 25.0 × 10⁻² weight parts) was added dropwise, such that the molar ratio of Li⁺/PEO in the solution was 0.1, and further stirred to give a solution e181.

On the other hand, 15 weight parts of methanol was added to 10 weight parts of tetramethoxysilane (TMOS) as the component (B), and stirred at room temperature. Thereafter, 10 weight parts of 0.1N hydrochloric acid was added dropwise, and stirred at room temperature for 1 hour to give a solution e182.

The solutions e181 and e182 were mixed at a weight ratio of 8/2, and further stirred at room temperature for 5 minutes to obtain a composition. This composition was applied to an Al₂O₃ deposited PET film having a thickness of 12 µm (TL-PET, a product of Tohcello Co., Ltd.) in the same manner as in Example B1, such that the thickness was about 0.5 µm, and the contents were heated at 110 degrees centigrade for 1.5 hours, thus to obtain a coating film.

### Example B19

The solutions e161 and e162 were mixed at a weight ratio of 7/3, and further stirred at room temperature for 5 minutes to obtain a composition. This composition was applied to an Al₂O₃ deposited PET film having a thickness of 12 µm (TL-PET, a product of Tohcello Co., Ltd.) in the same manner as in Example B1, such that the thickness was about 0.5 µm, and the contents were heated at 110 degrees centigrade for 1.5 hours, thus to obtain a coating film.

In Examples B7 and B17 to B19, the surface specific resistivity was measured. The surface specific resistivity was measured in accordance with JIS-K6911 after adjusting conditions to a temperature of 23 ± 2 degrees centigrade and a humidity of 50 ± 5%RH.

From the measurement results shown in Table 4, it was found that the surface specific resistivity in Examples B17 to B19 with Li ions introduced thereinto was low and high anti-static properties were exhibited, as compared to the surface specific resistivity of Example B7 without introducing Li ions.

Furthermore, as in Table 4, the molar ratio of Li⁺/PEO was increased, or the content of silica was reduced, whereby anti-static properties of the obtained coating film could be further improved.

**[Table 4]**

| | Molar ratio of Li⁺/PEO | Content of Silica | Surface specific resistivity |
|---|---|---|---|
| | - | wt% | Ω |
| Example B7 | 0 | 74 | 5.20E+14 |
| Example B17 | 0.05 | 76 | 1.02E+12 |
| Example B18 | 0.1 | 70 | 2.62E+10 |
| Example B19 | 0.1 | 76 | 5.95E+10 |

### Example B20

15 weight parts of methanol was added to 10 weight parts of tetramethoxysilane (TMOS) as the component (B), and stirred at room temperature. Furthermore, 15 weight parts of 0.1N hydrochloric acid was added dropwise thereto, and then stirred at room temperature for 1.5 hours. Thereafter, 60 weight parts of the 5 weight % T-1 aqueous dispersion solution containing the copolymer was added thereto, and stirred at room temperature for 5 minutes to give a solution e201. On the other hand, 15 weight parts of methanol was added to 10 weight parts of tetramethoxysilane (TMOS) as the component (B), and stirred at room temperature. Thereafter, 10 weight parts of 0.1N hydrochloric acid was added dropwise, and stirred at room temperature for 1 hour to give a solution e202. Incidentally, the solutions e201 and e202 are each a solution containing the components (B) and (D).

The solutions e201 and e202 were mixed at a weight ratio of 8/2, and further stirred at room temperature for 5 minutes to obtain a composition.

This composition was poured into a spray dryer apparatus (PULVIS BASIC UNIT MODEL GB-21, Yamato) at a flow rate of 6 cc/min and pressurized (2.6 kg/cm²) under heating atmosphere at 120 degrees centigrade for spraying, whereby composite particles of the copolymer and silica (silica content: 74 wt%) were obtained.

### Example B21

Using the 20 weight % T-1 aqueous dispersion solution containing the copolymer, in the same manner as in Example B20, this was poured into a spray dryer apparatus (PULVIS BASIC UNIT MODEL GB-21, Yamato) at a flow rate of 6 cc/min and pressurized (2.6 kg/cm²) under heating atmosphere at 120 degrees centigrade for spraying, whereby copolymer particles were obtained.

### Measurement of Particle Size

The particle samples prepared in Examples B20 and B21 were observed with a scanning electron microscope (S-4700, a product of Hitachi, Ltd.) to measure the particle size.

### Observation of Microparticle Cross Section

The particle sample fixed with a resin was cut into pieces by focused ion beam (FIB) processing. Subsequently, the shape of the cross section of this film was observed using a transmission electron microscope (H-7650, a product of Hitachi, Ltd.).

The microparticle samples prepared in Example B20 and B21 were observed with a scanning electron microscope and as a result, the particle sizes were from 1 to 10 µm.

It was found that the composite particles of the copolymer and silica of Example B20 had a structure regularly filled with a limitless number of copolymer microparticles (white portion) at the inside of silica (black portion), as compared to Example B21, by TEM observation of the cross section of the particle.

### Example B22

### Preparation of Solution of Polyolefin Based Terminally Branched Copolymer and TMOS Dehydrated Condensate

0.25 weight parts of methanol as the solvent was added to 0.5 weight parts of tetramethoxysilane (TMOS) and stirred at room temperature. Furthermore, 0.5 weight parts of an aqueous solution of 0.1N hydrochloric acid as the catalyst was added dropwise, and then stirred at 50 degrees centigrade for 1 hour to obtain a TMOS dehydrated condensate. An aqueous solution of 0.1N hydrochloric acid was further added dropwise to the obtained TMOS dehydrated condensate (to have the pH of 3 after addition of the polyolefin based terminally branched copolymer) and then stirred at room temperature. An aqueous dispersion (solid content: 10 weight %) of the polyolefin based terminally branched copolymer (T-1) was further added dropwise and stirred at room temperature to prepare a solution of the polyolefin based terminally branched copolymer and the TMOS dehydrated condensate. Incidentally, a solution was prepared with weight parts of Table 5, such that the weight ratio of the polyolefin based terminally branched copolymer and silica (in terms of SiO₂) was from 30/70 to 70/30 (solution pH=3).

### Formation of Composite Film of Polyolefin Based Terminally Branched Copolymer and Silica

The obtained solution was spin-coated on a silicon substrate and a quartz substrate, and heated at 110 degrees centigrade for 1.5 hours to obtain a composite film of the polyolefin based terminally branched copolymer and silica having a film thickness of 150 to 400 nm.

### Examples B23 to B29

A precursor solution was prepared with weight parts of Table 5 in the same manner as in Example B22, except that the polyolefin based terminally branched copolymer (T-1) of Example B22 was changed to (T-2) to (T-7) to obtain a composite film of the polyolefin based terminally branched copolymer and silica.

### Comparative Example B1

0.25 weight parts of methanol as the solvent was added to 0.5 weight parts of tetramethoxysilane (TMOS) and stirred at room temperature. Furthermore, 0.5 weight parts of an aqueous solution of 0.1N hydrochloric acid as the catalyst was added dropwise, and then stirred at 50 degrees centigrade for 1 hour to obtain a solution of a TMOS dehydrated condensate. The obtained solution was spin-coated on a silicon substrate and a quartz substrate, and heated 110 degrees centigrade for 1.5 hours.

**[Table 5]**

| | Polyolefin based terminally branched copolymer/ Silica (weight ratio) | Polyolefin based terminally branched copolymer/silica composite film precursor solution | | | | | |
|---|---|---|---|---|---|---|---|
| | | TMOS dehydrated condensate | | | 0.1N hydrochloric acid water (pH preparation) (g) | Polyolefin based terminally branched copolymer (10 weight % aqueous solution) (g) | |
| | | TMOS (g) | MeOH (g) | 0.1N hydrochloric acid water (g) | | | |
| Example B22 | 30/70 | 0.5 | 0.25 | 0.5 | 0 | T-1 | 0.8 |
| | 40/60 | | | | 0.4 | | 1.3 |
| | 50/50 | | | | 0.8 | | 1.95 |
| | 60/40 | | | | 1.4 | | 2.92 |
| | 70/30 | | | | 2 | | 4.56 |
| Example B23 | 30/70 | 0.5 | 0.25 | 0.5 | 0 | T-2 | 0.8 |
| | 40/60 | | | | 0.4 | | 1.3 |
| | 50/50 | | | | 0.8 | | 1.95 |
| | 60/40 | | | | 1.4 | | 2.92 |
| | 70/30 | | | | 2 | | 4.56 |
| Example B24 | 30/70 | 0.5 | 0.25 | 0.5 | 0 | T-3 | 0.8 |
| | 40/60 | | | | 0.4 | | 1.3 |
| | 50/50 | | | | 0.8 | | 1.95 |
| | 60/40 | | | | 1.4 | | 2.92 |
| | 70/30 | | | | 2 | | 4.56 |
| Example B25 | 30/70 | 0.5 | 0.25 | 0.5 | 0 | T-4 | 0.8 |
| | 40/60 | | | | 0.4 | | 1.3 |
| | 50/50 | | | | 0.8 | | 1.95 |
| | 60/40 | | | | 1.4 | | 2.92 |
| | 70/30 | | | | 2 | | 4.56 |
| Example B26 | 30/70 | 0.5 | 0.25 | 0.5 | 0 | T-5 | 0.8 |
| | 40/60 | | | | 0.4 | | 1.3 |
| | 50/50 | | | | 0.8 | | 1.95 |
| | 60/40 | | | | 1.4 | | 2.92 |
| | 70/30 | | | | 2 | | 4.56 |
| Example B27 | 30/70 | 0.5 | 0.25 | 0.5 | 0 | T-6 | 0.8 |
| | 40/60 | | | | 0.4 | | 1.3 |
| | 50/50 | | | | 0.8 | | 1.95 |
| | 60/40 | | | | 1.4 | | 2.92 |
| | 70/30 | | | | 2 | | 4.56 |
| Example B28 | 30/70 | 0.5 | 0.25 | 0.5 | 0 | T-7 | 0.8 |
| | 40/60 | | | | 0.4 | | 1.3 |
| | 50/50 | | | | 0.8 | | 1.95 |
| | 60/40 | | | | 1.4 | | 2.92 |
| | 70/30 | | | | 2 | | 4.56 |
| Example B29 | 30/70 | 0.5 | 0.25 | 0.5 | 0 | T-8 | 0.8 |
| | 40/60 | | | | 0.4 | | 1.3 |
| | 50/50 | | | | 0.8 | | 1.95 |
| | 60/40 | | | | 1.4 | | 2.92 |
| | 70/30 | | | | 2 | | 4.56 |
| Comparative Example B1 | 0/100 | 0.5 | 0.25 | 0.5 | 0 | - | - |

### Evaluation of Composite Film of Polyolefin Based Terminally Branched Copolymer and Silica

The thus-obtained composite films of the polyolefin based terminally branched copolymer and silica of Examples B22 to B29, and the silica film of Comparative Example B1 were evaluated in the following manner.

### 1. Film Quality

Films prepared in Examples B22 to B29 and Comparative Example B1 were observed with naked eyes and with an optical microscope (450 magnifications).

The evaluation results are shown in Table 6 below. The evaluation criteria are as follows.
A: No defects such as cracks or the like were found by observation with naked eyes and observation with an optical microscope.
B: No defects such as cracks or the like were found by observation with naked eyes, but defects were found by observation with an optical microscope.
C: Defects such as crack or the like were observed with naked eyes.

### 2. Permeability

For the films each prepared on a quartz substrate in Examples B22 to B29 and Comparative Example B1, the permeability was measured at a wavelength range of 400 to 600 nm using a UV spectrophotometer UV2200 manufactured by Shimadzu Corporation. The evaluation results are shown in the following Table 6.
A: Permeability of not less than 80% at a wavelength range of 400 to 600 nm
B: Permeability of from not less than 70% and less than 80% at a wavelength range of 400 to 600 nm
C: Permeability of less than 70% at a wavelength range of 400 to 600 nm

**[Table 6]**

| | Polyolefin based terminally branched copolymer/silica (weight ratio) | Evaluation Results | |
|---|---|---|---|
| | | Composite film of Polyolefin based terminally branched copolymer/silica | |
| | | Film Quality | Permeability |
| Example B22 | 30/70 | A | A |
| | 40/60 | A | A |
| | 50/50 | A | A |
| | 60/40 | A | A |
| | 70/30 | A | A |
| Example B23 | 30/70 | A | A |
| | 40/60 | A | A |
| | 50/50 | A | A |
| | 60/40 | A | A |
| | 70/30 | A | A |
| Example B24 | 30/70 | A | A |
| | 40/60 | A | A |
| | 50/50 | A | A |
| | 60/40 | A | A |
| | 70/30 | A | A |
| Example B25 | 30/70 | A | A |
| | 40/60 | A | A |
| | 50/50 | A | A |
| | 60/40 | A | A |
| | 70/30 | A | A |
| Example B26 | 30/70 | A | A |
| | 40/60 | A | A |
| | 50/50 | A | A |
| | 60/40 | A | A |
| | 70/30 | A | A |
| Example B27 | 30/70 | A | A |
| | 40/60 | A | A |
| | 50/50 | A | A |
| | 60/40 | A | A |
| | 70/30 | A | A |
| Example B28 | 30/70 | A | A |
| | 40/60 | A | A |
| | 50/50 | A | A |
| | 60/40 | A | A |
| | 70/30 | A | A |
| Example B29 | 30/70 | A | A |
| | 40/60 | A | A |
| | 50/50 | A | A |
| | 60/40 | A | A |
| | 70/30 | A | A |
| Comparative Example B1 | 0/100 | B | A |

In Examples B22 to B29, in all weight ratios of the polyolefin based terminally branched copolymer to silica (in terms of SiO₂), both film quality and permeability were excellent. On the other hand, micro cracks were observed in Comparative Example B1.

### 3. Refractive Index

The evaluation results are shown in the following Tables 7-1 and 7-2. For the films each prepared on a silicon substrate in Examples B22 to B29 and Comparative Example B1, the refractive index at 590 nm was measured using an ellipsometer (JASCO M-150).

**[Table 7-1]**

| Polyolefin based terminally branched copolymer/ silica (weight ratio) | Example B22 | Example B23 | Example B24 | Example B25 | Example B26 |
|---|---|---|---|---|---|
| 30/70 | 1.52 | 1.52 | 1.53 | 1.46 | 1.53 |
| 40/60 | 1.52 | 1.52 | 1.52 | 1.46 | 1.52 |
| 50/50 | 1.51 | 1.48 | 1.50 | 1.47 | 1.50 |
| 60/40 | 1.49 | 1.48 | 1.49 | 1.48 | 1.49 |
| 70/30 | 1.46 | 1.43 | 1.45 | 1.50 | 1.45 |
| 0/100 | - | | | | |

**[Table 7-2]**

| Polyolefin based terminally branched copolymer/ silica (weight ratio) | Example B27 | Example B28 | Example B29 | Comparative Example B1 |
|---|---|---|---|---|
| 30/70 | 1.51 | 1.55 | 1.51 | - |
| 40/60 | 1.47 | 1.49 | 1.49 | - |
| 50/50 | 1.47 | 1.48 | 1.48 | - |
| 60/40 | 1.48 | 1.46 | 1.46 | - |
| 70/30 | 1.50 | 1.47 | 1.43 | - |
| 0/100 | | | | 1.43 |

In Examples B22 to B29, the refractive index was changed depending on the ratio of the polyolefin based terminally branched copolymer to silica.

### Example B30

### Preparation of Solution of Polyolefin Based Terminally Branched Copolymer and TTIP dehydration Condensate

1. 32 weight parts of an aqueous hydrochloric acid solution (37%) was added to 2 weight parts of titanium isopropoxide (TTIP), and stirred at room temperature for 10 minutes to obtain a TTIP dehydrated condensate. As a pore forming material, the aqueous dispersion (solid content: 10 weight %) of the polyolefin based terminally branched copolymer (T-3) was added dropwise, and stirred at room temperature to obtain to prepare a precursor solution of a porous titania material. Incidentally, a solution was prepared with weight parts of Table 8, such that the weight ratio of the polyolefin based terminally branched copolymer to titania (in terms of TiO₂) was 15/85 to 50/50. Incidentally, the pHs of all solutions were not more than 1.

### Formation of Composite Film of Polyolefin Based Terminally Branched Copolymer and Titania

The obtained solution was spin-coated on the silicon substrate, and heated at 110 degrees centigrade for 1.5 hours to obtain a composite film of the polyolefin based terminally branched copolymer and titania.

### Comparative Example B2

1. 32 weight parts of an aqueous hydrochloric acid solution (37%) was added to 2 weight parts of titanium tetraisopropoxide (TTIP), and heated at room temperature for 10 minutes to obtain a solution of TTIP dehydrated condensate. The obtained solution (Table 4) was spin-coated on the silicon substrate, and heated at 110 degrees centigrade for 1.5 hours, thus to obtain a titania film.

**[Table 8]**

| | Polyolefin based terminally branched copolymer/ titania (weight ratio) | Polyolefin based terminally branched copolymer/titania Composite film Precursor solution | | | |
|---|---|---|---|---|---|
| | | TMOS dehydrated condensate | | Polyolefin based terminally branched copolymer (10 weight % aqueous solution) (g) | |
| | | TTIP (g) | Hydrochloric acid water (g) | | |
| Example B30 | 15/85 | 2.0 | 1.32 | T-3 | 1.0 |
| | 20/80 | | | | 1.4 |
| | 30/70 | | | | 2.4 |
| | 40/60 | | | | 3.7 |
| | 50/50 | | | | 5.6 |
| Comparative Example B2 | 0/100 | 2.0 | 1.32 | - | - |

### Evaluation of Composite Film of Polyolefin Based Terminally Branched Copolymer and Titania

The thus-obtained composite film of the polyolefin based terminally branched copolymer and titania in Example B30 and the titania film in Comparative Example B5 were evaluated in the following manner.

### 1. Film Quality

The films prepared in Example B30 and Comparative Example B2 were observed with naked eyes and with an optical microscope (450 magnifications).

The evaluation results are shown in the following Table 9. The evaluation criteria are as follows.
A: No defects such as cracks or the like were found by observation with naked eyes and observation with an optical microscope.
B: No defects such as cracks or the like were found by observation with naked eyes, but defects were found by observation with an optical microscope.
C: Defects such as crack or the like were observed with naked eyes.

### 2. Permeability

For the films each prepared on a quartz substrate in Example B30 and Comparative Example B2, the permeability was measured at a wavelength range of 400 to 600 nm using a UV spectrophotometer UV2200 manufactured by Shimadzu Corporation. The evaluation results are shown in the following Table 9.
A: Permeability of not less than 80% at a wavelength range of 400 to 600 nm
B: Permeability of from not less than 70% and less than 80% at a wavelength range of 400 to 600 nm
C: Permeability of less than 70% at a wavelength range of 400 to 600 nm

### 3. Refractive Index

For the films each prepared on a silicon substrate in Example B30 and Comparative Example B2, the refractive index at 590 nm was measured using an ellipsometer (JASCO M-150). The evaluation results are shown in the following Table 9.

**[Table 9]**

| | Polyolefin based terminally branched copolymer/ titania (weight ratio) | Evaluation Results | | |
|---|---|---|---|---|
| | | Composite film of Polyolefin based terminally branched copolymer/titania (110°C, 1.5 hr) | | |
| | | Film Quality | Permeability | Refractive Index |
| Example B30 | 15/85 | A | A | 1.64 |
| | 20/80 | A | A | 1.72 |
| | 30/70 | A | A | 1.74 |
| | 40/60 | A | A | 1.80 |
| | 50/50 | A | A | 1.88 |
| Comparative Example B2 | 0/100 | C | B | 1.99 |

In Example B30, in all weight ratios of the polyolefin based terminally branched copolymer to titania (in terms of TiO₂), both film quality and permeability were excellent. On the other hand, in Comparative Example B2, micro cracks were also observed in any films having a film thickness of 100 to 500 nm. The permeability was also low. In Example B30, the refractive index was changed depending on the ratio of the polyolefin based terminally branched copolymer to silica.

Incidentally, the present invention also includes the following embodiments.
[a1] A dispersion system containing a dispersoid having a terminally branched copolymer represented by the following general formula (1) and having a number average molecular weight of not more than 25,000, and water and/or an organic solvent having an affinity for water with the dispersoid dispersed therein, 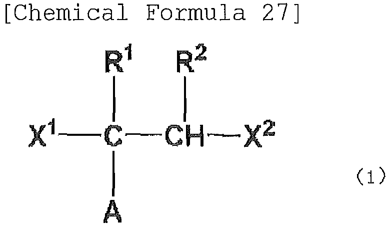 wherein, in the formula, A represents a group having a number average molecular weight of 400 to 8,000 in which an olefin having 2 to 20 carbon atoms is polymerized; R¹ and R² each represent a hydrogen atom or an alkyl group having 1 to 18 carbon atoms, and at least one of R¹ and R² is a hydrogen atom; and X¹ and X² may be the same or different, and each represent a linear or branched polyalkylene glycol group having a number average molecular weight of 50 to 10,000.
[a2] The dispersion system as set forth in [a1], wherein, in the terminally branched copolymer represented by the general formula (1), X¹ and X² may be the same or different, and each represent the general formula (2) or (4),

   [Chemical Formula 28] -E-X³ (2)

   wherein, in the formula, E represents an oxygen atom or a sulfur atom; and X³ represents a polyalkylene glycol group or a group represented by the following general formula (3),

   [Chemical Formula 29] -R³-(G)ₘ (3)

   wherein, in the formula, R³ represents an (m+1)-valent hydrocarbon group; G may be the same or different, and represents a group represented by -OX⁴ or -NX⁵X⁶ (X⁴ to X⁶ each represent a polyalkylene glycol group); and m is the bonding number of R³ and G, and represents an integer of 1 to 10, 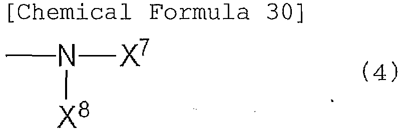 wherein, in the formula, X⁷ and X⁸ may be the same or different, and each represent a polyalkylene glycol group or a group represented by the above-described general formula (3).
[a3] The dispersion system as set forth in [a1], wherein, in the terminally branched copolymer represented by the general formula (1), any one of X¹ and X² represents the following general formula (5), 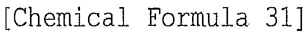 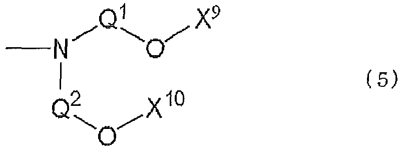 wherein, in the formula, X⁹ and X¹⁰ may be the same or different, and each represent a polyalkylene glycol group; and Q¹ and Q² may be the same or different, and each represent a divalent alkylene group.
[a4] The dispersion system as set forth in [a1], wherein, in the terminally branched copolymer represented by the general formula (1), at least one of X¹ and X² is a group represented by the general formula (6),

   [Chemical Formula 32] -O-X¹¹ (6)

   wherein, in the formula, X¹¹ represents a polyalkylene glycol group.
[a5] The dispersion system as set forth in any one of [a1] to [a4], wherein an average particle size of 50% by volume of particles composed of the terminally branched copolymer is from 0.01 µm to 1 µm.
[a6] The dispersion system as set forth in any one of [a1] to [a5], wherein the pH is from 1 to 13.
[a7] The dispersion system as set forth in any one of [a1] to [a6], wherein other dispersoid is contained in an amount of 0.001 weight parts to 20 weight parts, based on 100 weight parts of the terminally branched copolymer.
[a8] Particles composed of the terminally branched copolymer obtained from the dispersion system as set forth in any one of [a1] to [a7].
[a9] Particles composed of the terminally branched copolymer used for the dispersion system as set forth in any one of [a1] to [a7].
[a10] A dispersion system obtained by dispersing the dispersoid containing the particles as set forth in [a8] or [a9] in water and/or an organic solvent having an affinity for water.
[a11] An ink composition containing the dispersion system as set forth in any one of [a1] to [a7] and [a10].
[a12] An ink composition containing the particles as set forth in [a8] or [a9].
[a13] A coating agent containing the dispersion system as set forth in any one of [a1] to [a7] and [a10].
[a14] A coating agent containing the particles as set forth in [a8] or [a9].
[a15] A cosmetic preparation containing the dispersion system as set forth in any one of [a1] to [a7] and [a10].
[a16] A cosmetic preparation containing the particles as set forth in [a8] or [a9].

Furthermore, the present invention also contains the following embodiments.
[b1] A mixed composition containing the following (A) to (D),
   (A) a polyolefin based terminally branched copolymer represented by the following general formula (1) and having a number average molecular weight of not more than 25,000, 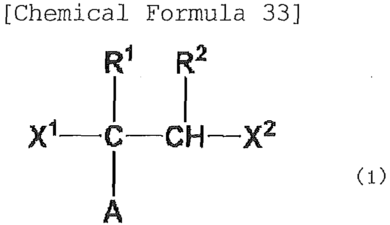 wherein, in the formula, A represents a group having a number average molecular weight of 400 to 8,000 in which an olefin having 2 to 20 carbon atoms is polymerized; R¹ and R² each represent a hydrogen atom or an alkyl group having 1 to 18 carbon atoms, and at least one of R¹ and R² is a hydrogen atom; and X¹ and X² may be the same or different, and each represent a linear or branched polyalkylene glycol group having a number average molecular weight of 50 to 10,000,
   (B) metal alkoxide and/or a hydrolysis condensate thereof,
   (C) water and/or a solvent for dissolving a part of water or entire water in any proportions, and
   (D) a catalyst to be used for the sol-gel reaction.
[b2] The mixed composition as set forth in [b1], wherein, for the aforementioned polyolefin based terminally branched copolymer, in the aforementioned general formula (b1), X¹ and X² may be the same or different, and each represent the general formula (2) or (4),

   [Chemical Formula 34] -E-X³ (2)

   wherein, in the formula, E represents an oxygen atom or a sulfur atom; and X³ represents a polyalkylene glycol group or a group represented by the following general formula (3),

   [Chemical Formula 35] -R³-(G)ₘ (3)

   wherein, in the formula, R³ represents an (m+1)-valent hydrocarbon group; G may be the same or different, and represents a group represented by -OX⁴ or -NX⁵X⁶ (X⁴ to X⁶ each represent a polyalkylene glycol group); and m represents the bonding number of G, and represents an integer of 1 to 10, 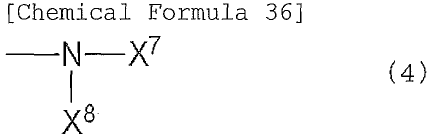 wherein, in the formula, X⁷ and X⁸ may be the same or different, and each represent a polyalkylene glycol group or a group represented by the above-described general formula (3).
[b3] The mixed composition as set forth in [b1] or [b2], wherein, for the aforementioned polyolefin based terminally branched copolymer, in the aforementioned general formula (1), one of X¹ and X² represents the following general formula (5), 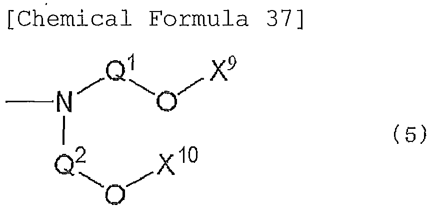 wherein, in the formula, X⁹ and X¹⁰ may be the same or different, and each represent a polyalkylene glycol group; and Q¹ and Q² may be the same or different, and each represent a divalent alkylene group.
[b4] The mixed composition as set forth in any one of [b1] to [b3], wherein, for the aforementioned polyolefin based terminally branched copolymer, in the aforementioned general formula (1), at least one of X¹ and X² represents the general formula (6),

   [Chemical Formula 38] -O-X¹¹ (6)

   wherein, in the formula, X¹¹ represents a polyalkylene glycol group.
[b5] The mixed composition as set forth in any one of [b1] to [b4], wherein a metal of the aforementioned metal alkoxide and/or a hydrolysis condensate thereof (B) is one or more kinds selected from the group consisting of silicon, zirconium, aluminum and titanium.
[b6] The mixed composition as set forth in any one of [b1] to [b5], wherein the aforementioned solvent (C) is water.
[b7] The mixed composition as set forth in any one of [b1] to [b6], wherein the aforementioned solvent (C) is mono alcohol having 1 to 3 carbon atoms.
[b8] A composition obtained by the sol-gel reaction of the mixed composition as set forth in any one of [b1] to [b7].
[b9] The composition as set forth in [b8], wherein microparticles of the polyolefin based terminally branched copolymer (A) defined in (1) are dispersed in a matrix having mainly metal oxide formed by the sol-gel reaction of the aforementioned metal alkoxide and/or a hydrolysis condensate thereof (B).
[b10] The composition as set forth in [b8] or [b9], wherein an average particle size of microparticles of the polyolefin based terminally branched copolymer contained in the aforementioned composition is in the range of 0.01 µm to 1 µm.
[b11] A coated laminate, wherein the mixed composition as set forth in [b1] is provided on the plastic base material (I) as the coating film layer (II-a).
[b12] The coated laminate as set forth in [b11] wherein the deposited film layer (III) composed of an inorganic compound is provided between the aforementioned plastic base material (I) and the aforementioned coating film layer (II-a).
[b13] A plastic laminate, wherein the coating film layer (II-b) is provided on the plastic base material (I), the coating film layer (II-b) is obtained by applying the mixed composition as set forth in [b1], removing water and/or the solvent for dissolving a part of water or entire water in any proportions (C) contained in the mixed composition, and carrying out the sol-gel reaction of the mixed composition at the same time.
[b14] The plastic laminate as set forth in [b13], wherein the deposited film layer (III) composed of an inorganic compound is provided between the aforementioned plastic base material (I) and the aforementioned coating film layer (II-b).
[b15] A gas barrier film composed of the plastic laminate as set forth in [b13] or [b14].
[b16] A hard coat film composed of the plastic laminate as set forth in [b13] or [b14].

## Claims

1. Polymer particles comprising a terminally branched copolymer represented by the following general formula (1) and having a number average molecular weight of not more than 2.5 x 10⁴, 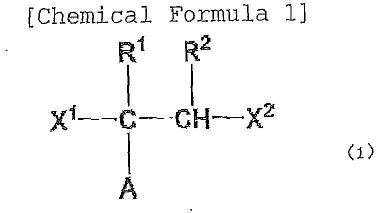 wherein, in the formula, A represents a polyolefin chain; R¹ and R² each represent a hydrogen atom or an alkyl group having 1 to 18 carbon atoms, and at least one of R¹ and R² is a hydrogen atom; and X¹ and X² may be the same or different, and each represent a linear or branched polyalkylene glycol group, or the following general formula (2) or (4),
[Chemical Formula 2] -E-X³ (2)
wherein, in the formula, E represents an oxygen atom or a sulfur atom; and X³ represents a polyalkylene glycol group or a group represented by the following general formula (3),
[Chemical Formula 3] -R³-(G)ₘ (3)
wherein, in the formula, R³ represents an (m+1)-valent hydrocarbon group; G may be the same or different, and represents a group represented by -OX⁴ or -NX⁵X⁶ (X⁴ to X⁶ each represent a polyalkylene glycol group); and m is the bonding number of R³ and G, and represents an integer of 1 to 10, 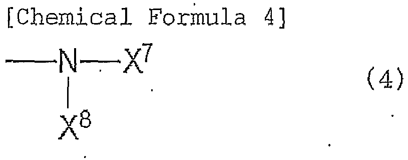 wherein, in the formula, X⁷ and X⁸ may be the same or different, and each represent a polyalkylene glycol group or a group represented by the above-described general formula (3),
wherein, in the polymer particles, the melting point of the polyolefin chain portion of the terminally branched copolymer is not less than 80 degrees centigrade, and
wherein an average particle size of 50% by volume is from not less than 1nm and not more than 100 nm.

2. The polymer particles as set forth in claim 1, wherein, in the terminally branched copolymer represented by the general formula (1), any one of X¹ and X² represents the following general formula (5), 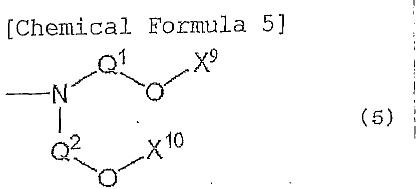 wherein, in the formula, X⁹ and X¹⁰ may be the same or different, and each represent a polyalkylene glycol group; and Q¹ and Q² may be the same or different, and each represent a divalent alkylene group.

3. The polymer particles as set forth in claim 1 or 2, wherein, in the terminally branched copolymer represented by the general formula (1), at least one of X¹ and X² represents the following general formula (6),
[Chemical Formula 6] -O-X¹¹ (6)
wherein, in the formula, X¹¹ represents a polyalkylene glycol group.

4. The polymer particles as set forth in any one of claims 1 to 3, wherein the terminally branched copolymer is represented by the following general formula (1b), 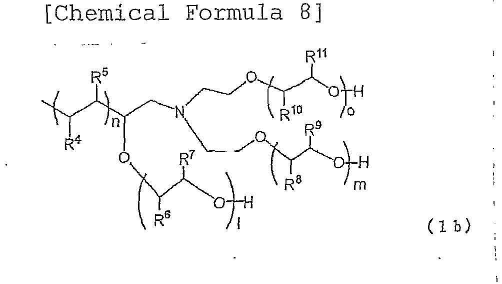 wherein, in the formula, R⁴ and R⁵ each represent a hydrogen atom or an alkyl group having 1 to 18 carbon atoms, and at least one of R⁴ and R⁵ is a hydrogen atom; R⁶ and R⁷ each represent a hydrogen atom or a methyl group, and at least one of R⁶ and R⁷ is a hydrogen atom; R⁸ and R⁹ each represent a hydrogen atom or a methyl group, and at least one of R⁸ and R⁹ is a hydrogen atom; R¹⁰ and R¹¹ each represent a hydrogen atom or a methyl group, and at least one of R¹⁰ and R¹¹ is a hydrogen atom; 1+m+o represents an integer of not less than 3 and not more than 450; and n represents an integer of not less than 20 and not more than 300.

5. The polymer particles as set forth in any one of claims 1 to 3, wherein the terminally branched copolymer is represented by the following general formula (1a), 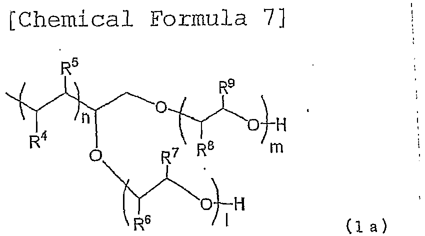 wherein, in the formula, R⁴ and R⁵ each represent a hydrogen atom or an alkyl group having 1 to 18 carbon atoms, and at least one of R⁴ and R⁵ is a hydrogen atom; R⁶ and R⁷ each represent a hydrogen atom or a methyl group, and at least one of R⁶ and R⁷ is a hydrogen atom; R⁸ and R⁹ each represent a hydrogen atom or a methyl group, and at least one of R⁸ and R⁹ is a hydrogen atom; 1+m represents an integer of not less than 2 and not more than 450; and n represents an integer of not less than 20 and not more than 300.

6. The polymer particles as set forth in any one of claims 1 to 5, wherein in the polymer particles, the polyolefin chain portion of the terminally branched copolymer has crystallinity.

7. The polymer particles as set forth in any one of claims 1 to 6, wherein other dispersoid is contained in an amount of 0.001 weight parts to 20 weight parts, based on 100 weight parts of the terminally branched copolymer.

8. The polymer particles as set forth in claim 7, wherein other dispersoid is encapsulated in the polymer particles.

9. A dispersion solution comprising a dispersoid composed of the polymer particles as set forth in any one of claims 1 to 8, and water and/or an organic solvent having an affinity for water with the dispersoid dispersed therein.

10. The dispersion solution as set forth in claim 9, wherein said dispersoid is dispersed in water and/or an organic solvent having an affinity for water.

11. The dispersion solution as set forth in claim 9 or 10, wherein the pH is from 1 to 13.

12. A mixed composition comprising the following (A) to (D),
(A) polymer particles as defined in claim 1,
(B) metal alkoxide and/or a hydrolysis condensate thereof,
(C) water and/or a solvent for dissolving a part of water or entire water in any proportions, and
(D) a catalyst to be used for the sol-gel reaction.

13. The mixed composition as set forth in claim 12, wherein, in the terminally branched copolymer represented by the general formula (1), any one of X¹ and X² represents the following general formula (5), 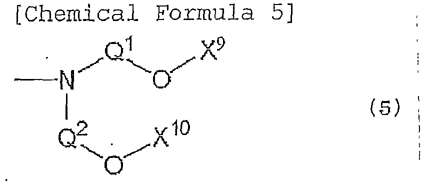 wherein, in the formula, X⁹ and X¹⁰ may be the same or different, and each represent a polyalkylene glycol group; and Q¹ and Q² may be the same or different, and each represent a divalent alkylene group.

14. The mixed composition as set forth in any one of claims 12 or 13, wherein, in the terminally branched copolymer represented by the general formula (1), at least one of X¹ and X² represents the following general formula (6),
[Chemical Formula 6] -O-X¹¹ (6)
wherein, in the formula, X¹¹ represents a polyalkylene glycol group

15. The mixed composition as set forth in any one of claims 12 to 14, wherein the terminally branched copolymer is represented by the following general formula (1b), 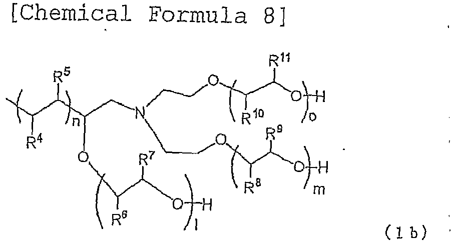 wherein, in the formula, R⁴ and R⁵ each represent a hydrogen atom or an alkyl group having 1 to 18 carbon atoms, and at least one of R⁴ and R⁵ is a hydrogen atom; R⁶ and R⁷ each represent a hydrogen atom or a methyl group, and at least one of R⁶ and R⁷ is a hydrogen atom; R⁸ and R⁹ each represent a hydrogen atom or a methyl group, and at least one of R⁸ and R⁹ is a hydrogen atom; R¹⁰ and R¹¹ each represent a hydrogen atom or a methyl group, and at least one of R¹⁰ and R¹¹ is a hydrogen atom; 1+m+o represents an integer of not less than 3 and not more than 450; and n represents an integer of not less than 20 and not more than 300.

16. The mixed composition as set forth in any one of claims 12 to 14, wherein the terminally branched copolymer is represented by the following general formula (1a), 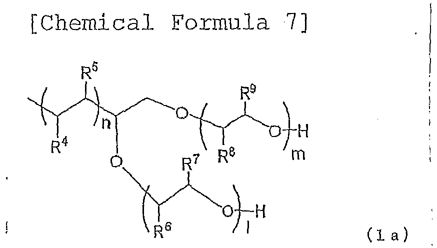 wherein, in the formula, R⁴ and R⁵ each represent a hydrogen atom or an alkyl group having 1 to 18 carbon atoms, and at least one of R⁴ and R⁵ is a hydrogen atom; R⁶ and R⁷ each represent a hydrogen atom or a methyl group, and at least one of R⁶ and R⁷ is a hydrogen atom; R⁸ and R⁹ each represent a hydrogen atom or a methyl group, and at least one of R⁸ and R⁹ is a hydrogen atom; 1+m represents an integer of not less than 2 and not more than 450; and n represents an integer of not less than 20 and not more than 300.

17. The mixed composition as set forth in any one of claims 12 to 16, wherein in the polymer particles, the polyolefin chain portion of the terminally branched copolymer has crystallinity.

18. The mixed composition as set forth in any one of claims 12 to 17, wherein other dispersoid is contained in an amount of 0.001 weight parts to 20 weight parts, based on 100 weight parts of the terminally branched copolymer.

19. The mixed composition polymer particles as set forth in claim 18, wherein other dispersoid is encapsulated in the polymer particles.

20. The mixed composition as set forth in any one of claims 12 to 19, wherein a metal of said metal alkoxide and/or hydrolysis condensate thereof (B) is one or more kinds selected from the group consisting of silicon, zirconium, aluminum and titanium.

21. The mixed composition as set forth in any one of claims 12 to 20, wherein said solvent (C) is water.

22. The mixed composition as set forth in any one of claims 12 to 21, wherein said solvent (C) is mono alcohol having 1 to 3 carbon atoms.

23. A composite obtained by dispersing the polymer particles of any one of claims 1 to 8 in a matrix composed of a metal oxide.

24. The composite as set forth in claim 23 obtained by the sol-gel reaction of the mixed composition as set forth in any one of claims 12 to 22.

25. The composite as set forth in claim 23 or 24, wherein said metal oxide is formed by the sol-gel reaction of metal alkoxide and/or a hydrolysis condensate thereof.

26. The composite as set forth in any one of claims 23 to 25, further comprising alkali metal salts as an anti-static agent.

27. The composite as set forth in claim 26, wherein alkali metal salts contain at least one anionic lithium salt selected from the group consisting of trifluoromethanesulfonic acid, bis(trifluoromethanesulfonyl)imide and tri(trifluoromethanesulfonyl)methane.

28. A plastic laminate comprising a coating layer composed of the composite as set forth in any one of claims 23 to 27 on a plastic base material.

29. The plastic laminate as set forth in claim 28, comprising a deposited film layer composed of an inorganic compound between said plastic base material and said coating layer.

30. A gas barrier material comprising the plastic laminate as set forth in claim 28 or 29.

31. A hard coat material comprising the plastic laminate as set forth in claim 28 or 29.

32. An anti-static film comprising the plastic laminate as set forth in claim 28 or 29.

33. An ink composition comprising the polymer particles as set forth in any one of claims 1 to 8 or the dispersion solution as set forth in any one of claims 9 to 11.

34. A coating agent comprising the polymer particles as set forth in any one of claims 1 to 8 or the dispersion solution as set forth in any one of claims 9 to 11.

35. A cosmetic preparation comprising the polymer particles as set forth in any one of claims 1 to 8 or the dispersion solution as set forth in any one of claims 9 to 11.

36. An anti-static film obtained by applying the coating agent as set forth in claim 34.

## Patentansprüche

1. Polymerpartikel, umfassend ein endverzweigtes Copolymer, das durch die folgende allgemeine Formel (1) dargestellt ist, und ein durchschnittliches Molekulargewicht (Zahlenmittel) von nicht mehr als 2,5 x 10⁴ hat, 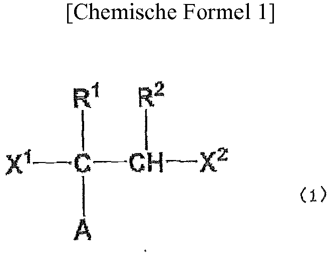 wobei A in der Formel eine Polyolefinkette darstellt; R¹ und R² jeweils ein Wasserstoffatom oder eine Alkylgruppe mit 1-18 Kohlenstoffatomen darstellen, und mindestens eines von R¹ oder R² ein Wasserstoffatom ist; und X¹ und X² gleich oder verschieden sein können und jedes eine lineare oder eine verzweigte Polyalkylenglykolgruppe, oder die folgende allgemeine Formel (2) oder (4), darstellt
[Chemische Formel 2] -E-X³ (2)
wobei E in der Formel ein Sauerstoffatom oder ein Schwefelatom darstellt; und X³ eine Polyalkylenglykolgruppe oder eine Gruppe darstellt, die durch die folgende allgemeine Formel (3) dargestellt ist,
[Chemische Formel 3] -R³-(G)ₘ (3)
wobei R³ in der Formel eine (m+1)-wertige Kohlenwasserstoffgruppe darstellt; G gleich oder verschieden sein kann und eine Gruppe darstellt, die durch -OX⁴ oder -NX⁵X⁶ dargestellt ist (X⁴-X⁶ stellen jeweils eine Polyalkylenglykolgruppe dar); und m die Bindungsanzahl von R³ und G ist und eine Ganzzahl von 1-10 darstellt, 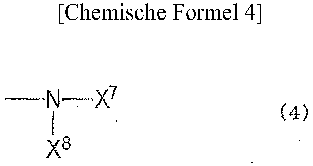 wobei X⁷ und X⁸ in der Formel gleich oder verschieden sein können und jeweils eine Polyalkylenglykolgruppe oder eine Gruppe darstellen, die durch die zuvor beschriebene allgemeine Formel (3) dargestellt ist,
wobei in den Polymerpartikeln der Schmelzpunkt des Polyolefinkettenabschnitts des endverzweigten Copolymers nicht weniger als 80 Grad Celsius beträgt, und
wobei eine Durchschnittspartikelgröße von 50 Vol.-% nicht weniger als 1 nm und nicht mehr als 100 nm beträgt.

2. Polymerpartikel nach Anspruch 1, wobei im durch die allgemeine Formel (1) dargestellten endverzweigten Copolymer X¹ oder X² die folgende allgemeine Formel (5) darstellt, 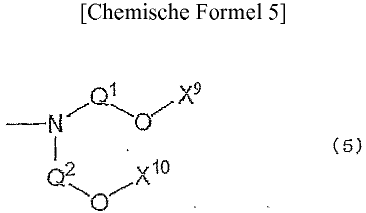 wobei in der Formel X⁹ und X¹⁰ gleich oder verschieden sein können und jeweils eine Polyalkylenglykolgruppe darstellen; und Q¹ und Q² gleich oder verschieden sein können und jeweils eine zweiwertige Alkylengruppe darstellen.

3. Polymerpartikel nach Anspruch 1 oder 2, wobei im durch die allgemeine Formel (1) dargestellten endverzweigten Copolymer mindestens eines von X¹ oder X² die folgende allgemeine Formel (6) darstellt,
[Chemische Formel 6] -O-X¹¹ (6)
wobei X¹¹ in der Formel eine Polyalkylenglykolgruppe darstellt.

4. Polymerpartikel nach einem der Ansprüche 1 bis 3, wobei das endverzweigten Copolymer durch die folgende allgemeine Formel (1b) dargestellt ist, 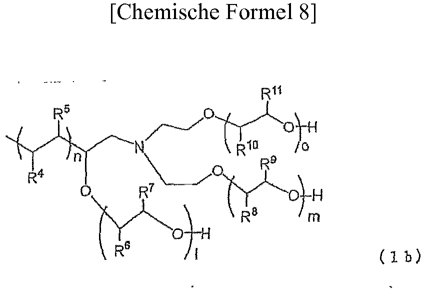 wobei R⁴ und R⁵ in der Formel jeweils ein Wasserstoffatom oder eine Alkylgruppe mit 1 bis 18 Kohlenstoffatomen darstellen und mindestens eines von R⁴ und R⁵ ein Hydrogenatom ist; R⁶ und R⁷ jeweils ein Wasserstoffatom oder eine Methylgruppe darstellen, und mindestens eines von R⁶ und R⁷ ein Wasserstoffatom ist; R⁸ und R⁹ jeweils ein Wasserstoffatom oder eine Methylgruppe darstellen, und mindestens eines von R⁸ und R⁹ ein Wasserstoffatom ist; R¹⁰ und R¹¹ jeweils ein Wasserstoffatom oder eine Methylgruppe darstellen, und mindestens eines von R¹⁰ und R¹¹ ein Wasserstoffatom ist; 1+m+o eine Ganzzahl von nicht weniger als 3 und nicht mehr als 450 darstellt; und n eine Ganzzahl von nicht weniger als 20 und nicht mehr als 300 darstellt.

5. Polymerpartikel nach einem der Ansprüche 1 bis 3, wobei das endverzweigte Copolymer durch die folgende allgemeine Formel (1a) dargestellt ist, 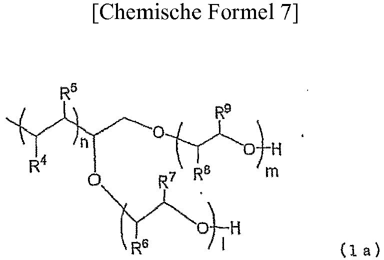 wobei in der Formel R⁴ und R⁵ jeweils ein Wasserstoffatom oder eine Alkylgruppe mit 1 bis 18 Kohlenstoffatomen darstellen, und mindestens eines von R⁴ und R⁵ ein Hydrogenatom ist; R⁶ und R⁷ jeweils ein Wasserstoffatom oder eine Methylgruppe darstellen, und mindestens eines von R⁶ und R⁷ ein Wasserstoffatom ist; R⁸ und R⁹ jeweils ein Wasserstoffatom oder eine Methylgruppe darstellen, und mindestens eines von R⁸ und R⁹ ein Wasserstoffatom ist; 1+m eine Ganzzahl von nicht weniger als 2 und nicht mehr als 450 darstellt; und n eine Ganzzahl von nicht weniger als 20 und nicht mehr als 300 darstellt.

6. Polymerpartikel nach einem der Ansprüche 1 bis 5, wobei in den Polymerpartikeln der Polyolefinkettenabschnitt des endverzweigten Copolymers Kristallinität hat.

7. Polymerpartikel nach einem der Ansprüche 1 bis 6, wobei ein anderes Dispersoid in einer Menge von 0,001 Gewichtsteilen bis zu 20 Gewichtsteilen enthalten ist, basierend auf 100 Gewichtsteilen des endverzweigten Copolymers.

8. Polymerpartikel nach Anspruch 7, wobei ein anderes Dispersoid in den Polymerpartikeln eingeschlossen ist.

9. Dispersionslösung, umfassend ein Dispersoid, das aus den Polymerpartikeln nach einem der Ansprüche 1-8 und Wasser und/oder organischem Lösungsmittel mit einer Affinität zu Wasser mit dem darin dispergierten Dispersoid besteht.

10. Dispersionslösung nach Anspruch 9, wobei das Dispersoid in Wasser und/oder einem organischen Lösungsmittel mit einer Affinität zu Wasser dispergiert ist.

11. Dispersionslösung nach Anspruch 9 oder 10, wobei der pH-Wert ein Wert von 1 bis 13 ist.

12. Gemischte Zusammensetzung, umfassend das folgende (A) bis (D),
(A) Polymerpartikel nach Anspruch 1,
(B) Metallalkoxid und/oder ein Hydrolysekondensat davon,
(C) Wasser und/oder ein Lösungsmittel zum Auflösen eines Teils des Wassers oder des ganzen Wassers in beliebigen Anteilen, und
(D) einen Katalysator zur Verwendung für die Sol-Gel-Reaktion.

13. Gemischte Zusammensetzung nach Anspruch 12, wobei im durch die allgemeine Formel (1) dargestellten endverzweigten Copolymer eines von X¹ und X² die folgende allgemeine Formel (5) darstellt, 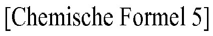 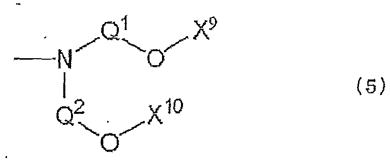 wobei in der Formel X⁹ und X¹⁰ gleich oder verschieden sein können und jeweils eine Polyalkylenglykolgruppe darstellen und Q¹ und Q² gleich oder verschieden sein können und jeweils eine zweiwertige Alkylengruppe darstellen.

14. Gemischte Zusammensetzung nach einem der Ansprüche 12 oder 13, wobei im durch die allgemeine Formel (1) dargestellten endverzweigten Copolymer mindestens eines von X¹ und X² die folgende allgemeine Formel (6) darstellt,
[Chemische Formel 6] -O-X¹¹ (6)
wobei in der Formel X¹¹ eine Polyalkylenglykolgruppe darstellt.

15. Gemischte Zusammensetzung nach einem der Ansprüche 12 bis 14, wobei das endverzweigte Copolymer durch die folgende allgemeine Formel (1b) dargestellt ist, 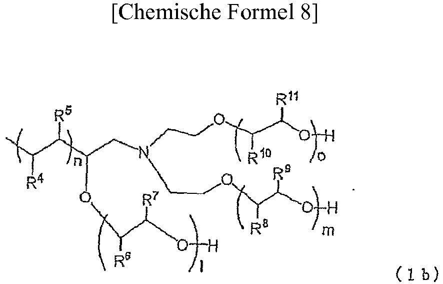 wobei R⁴ und R⁵ in der Formel jeweils ein Wasserstoffatom oder eine Alkylgruppe mit 1 bis 18 Kohlenstoffatomen darstellen und mindestens eines von R⁴ und R⁵ ein Hydrogenatom ist; R⁶ und R⁷ jeweils ein Wasserstoffatom oder eine Methylgruppe darstellen, und mindestens eines von R⁶ und R⁷ ein Wasserstoffatom ist; R⁸ und R⁹ jeweils ein Wasserstoffatom oder eine Methylgruppe darstellen, und mindestens eines von R⁸ und R⁹ ein Wasserstoffatom ist; R¹⁰ und R¹¹ jeweils ein Wasserstoffatom oder eine Methylgruppe darstellen, und mindestens eines von R¹⁰ und R¹¹ ein Wasserstoffatom ist; 1+m+o eine Ganzzahl von nicht weniger als 3 und nicht mehr als 450 darstellt; und n eine Ganzzahl von nicht weniger als 20 und nicht mehr als 300 darstellt.

16. Gemischte Zusammensetzung nach einem der Ansprüche 12 bis 14, wobei das endverzweigte Copolymer durch die folgende allgemeine Formel (1a) dargestellt ist, 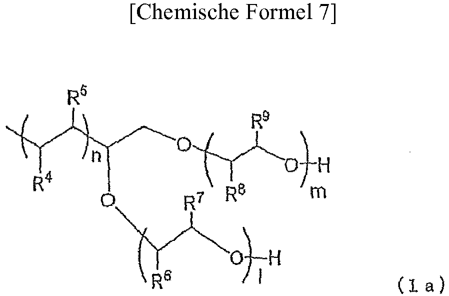 wobei R⁴ und R⁵ in der Formel jeweils ein Wasserstoffatom oder eine Alkylgruppe mit 1 bis 18 Kohlenstoffatomen darstellen, und mindestens eines von R⁴ und R⁵ ein Wasserstoffatom ist; R⁶ und R⁷ jeweils ein Wasserstoffatom oder eine Methylgruppe darstellen, und mindestens eines von R⁶ und R⁷ ein Wasserstoffatom ist; R⁸ und R⁹ jeweils ein Wasserstoffatom oder eine Methylgruppe darstellen, und mindestens eines von R⁸ und R⁹ ein Wasserstoffatom ist; 1+m eine Ganzzahl von nicht weniger als 2 und nicht mehr als 450 darstellt; und n eine Ganzzahl von nicht weniger als 20 und nicht mehr als 300 darstellt.

17. Gemischte Zusammensetzung nach einem der Ansprüche 12 bis 16, wobei in den Polymerpartikeln der Polyolefinkettenabschnitt des endverzweigten Copolymers Kristallinität hat.

18. Gemischte Zusammensetzung nach einem der Ansprüche 12 bis 17, wobei ein anderes Dispersoid in einer Menge von 0,001 Gewichtsteilen bis zu 20 Gewichtsteilen enthalten ist, basierend auf 100 Gewichtsteilen des endverzweigten Copolymers.

19. Gemischte Zusammensetzung Polymerpartikel nach Anspruch 18, wobei ein anderes Dispersoid in den Polymerpartikeln eingeschlossen ist.

20. Gemischte Zusammensetzung nach einem der Ansprüche 12 bis 19, wobei ein Metall des Metallalkoxids und/oder Hydrolysekondensats davon (B) eine oder mehrere Arten ist, ausgewählt aus der Gruppe bestehend aus Silicium, Zirconium, Aluminium und Titan.

21. Gemischte Zusammensetzung nach einem der Ansprüche 12 bis 20, wobei das Lösungsmittel (C) Wasser ist.

22. Gemischte Zusammensetzung nach einem der Ansprüche 12 bis 21, wobei das Lösungsmittel (C) reiner Alkohol mit 1-3 Kohlenstoffatomen ist.

23. Verbundstoff, erlangt durch Dispergieren der Polymerpartikel nach einem der Ansprüche 1-8 in einer aus einem Metalloxid bestehenden Matrix.

24. Verbundstoff nach Anspruch 23, erhalten durch die Sol-Gel-Reaktion der gemischten Zusammensetzung nach einem der Ansprüche 12 bis 22.

25. Verbundstoff nach Anspruch 23 oder 24, wobei das Metalloxid durch Sol-Gel-Reaktion von Metallalkoxid und/oder einem Hydrolysekondensat davon gebildet ist.

26. Verbundstoff nach einem der Ansprüche 23 bis 25, ferner umfassend Alkalimetallsalze als ein antistatisches Mittel.

27. Verbundstoff nach Anspruch 26, wobei Alkalimetallsalze mindestens ein anionisches Lithiumsalz enthalten, ausgewählt aus der Gruppe bestehend aus Trifluormethansulfonsäure, Bis(trifluormethansulfonyl)imid und Tri(trifluormethansulfonyl)methan.

28. Kunststoffschichtstoff, umfassend eine Überzugsschicht, die aus dem Verbundstoff nach einem der Ansprüche 23 bis 27 auf einem Kunststoffgrundmaterial besteht.

29. Kunststoffschichtstoff nach Anspruch 28, umfassend eine aufgebrachte Filmschicht, die aus einer anorganischen Verbindung zwischen dem Kunststoffgrundmaterial und der Überzugsschicht besteht.

30. Gasbarrierenmaterial, umfassend den Kunststoffschichtstoff nach Anspruch 28 oder 29.

31. Hartschichtmaterial, umfassend den Kunststoffschichtstoff nach Anspruch 28 oder 29.

32. Antistatischer Film, umfassend den Kunststoffschichtstoff nach Anspruch 28 oder 29.

33. Farbzusammensetzung, umfassend die Polymerpartikel nach einem der Ansprüche 1 bis 8 oder die Dispersionslösung nach einem der Ansprüche 9 bis 11.

34. Beschichtungsmittel, umfassend die Polymerpartikel nach einem der Ansprüche 1 bis 8 oder die Dispersionslösung nach einem der Ansprüche 9 bis 11.

35. Kosmetische Zubereitung, umfassend die Polymerpartikel nach einem der Ansprüche 1 bis 8 oder die Dispersionslösung nach einem der Ansprüche 9 bis 11.

36. Antistatischer Film, erlangt durch Anwenden des Beschichtungsmittels nach Anspruch 34.

## Revendications

1. Particules de polymère comprenant un copolymère à ramification terminale représenté par la formule générale (1) suivante et ayant un poids moléculaire moyen en nombre n'excédant pas 2,5 x 10⁴, 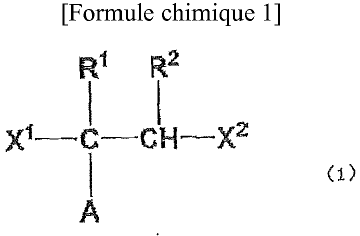 dans laquelle A représente une chaîne polyoléfinique ; R¹ et R² représentent chacun un atome d'hydrogène ou un groupe alkyle ayant 1 à 18 atomes de carbone, l'un au moins des groupes R¹ et R² étant un atome d'hydrogène ; et X¹ et X² peuvent être identiques ou différents, chacun représentant un groupe polyalkylène glycol linéaire ou ramifié ou la formule générale (2) ou (4) suivante :
[Formule chimique 2] -E-X³ (2)
dans laquelle E représente un atome d'oxygène ou un atome de soufre, et X³ représente un groupe polyalkylène glycol ou un groupe représenté par la formule générale (3) suivante :
[Formule chimique 3] -R³-(G)ₘ (3)
dans laquelle R³ représente un groupe hydrocarboné de valence m+1, G peut être identique ou différent et représente un groupe représenté par -OX⁴ ou -NX⁵X⁶ (X⁴ à X⁶ représentant chacun un groupe polyalkylène glycol) ; et m est l'indice de valence formelle de R³ et G et représente un nombre entier compris entre 1 et 10, 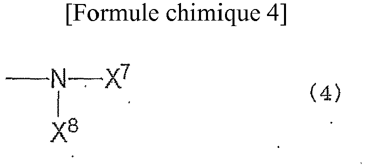 dans laquelle X⁷ et X⁸ peuvent être identiques ou différents, chacun représentant un groupe polyalkylène glycol ou un groupe représenté par la formule générale (3) décrite ci-dessus,
dans les particules de polymère, le point de fusion de la partie chaîne polyoléfinique du copolymère à ramification terminale n'étant pas inférieur à 80 degrés centigrades, et
une taille particulaire moyenne de 50 % en volume étant comprise entre pas moins de 1 nm et pas plus de 100 nm.

2. Particules de polymère selon la revendication 1, dans lesquelles, dans le copolymère à ramification terminale représenté par la formule générale (1), l'un quelconque des groupes X¹ et X² représente la formule générale (5) suivante : 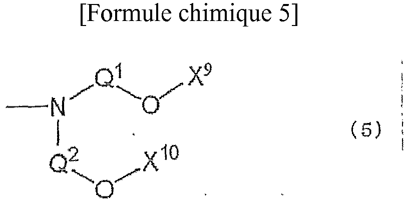 dans laquelle X⁹ et X¹⁰ peuvent être identiques ou différents, chacun représentant un groupe polyalkylène glycol ; et Q¹ et Q² peuvent être identiques ou différents, chacun représentant un groupe alkylène divalent.

3. Particules de polymère selon la revendication 1 ou 2, dans lesquelles, dans le copolymère à ramification terminale représenté par la formule générale (1), X¹ et/ou X² représentent la formule générale (6) suivante :
[Formule chimique 6] -O-X¹¹ (6)
dans laquelle X¹¹ représente un groupe polyalkylène glycol.

4. Particules de polymère selon l'une quelconque des revendications 1 à 3, dans lesquelles le copolymère à ramification terminale est représenté par la formule générale (1b) suivante : 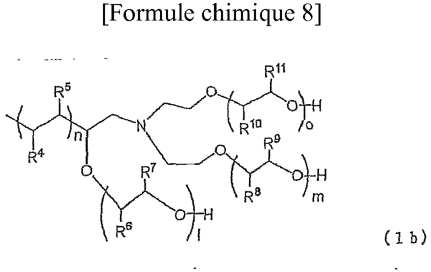 dans laquelle R⁴ et R⁵ représentent chacun un atome d'hydrogène ou un groupe alkyle ayant entre 1 et 18 atomes de carbone, l'un au moins des groupes R⁴ et R⁵ étant un atome d'hydrogène ; R⁶ et R⁷ représentent chacun un atome d'hydrogène ou un groupe méthyle, l'un au moins des groupes R⁶ et R⁷ étant un atome d'hydrogène ; R⁸ et R⁹ représentent chacun un atome d'hydrogène ou un groupe méthyle, l'un au moins des groupes R⁸ et R⁹ étant un atome d'hydrogène ; R¹⁰ et R¹¹ représentent chacun un atome d'hydrogène ou un groupe méthyle, l'un au moins des groupes R¹⁰ et R¹¹ étant un atome d'hydrogène ; 1+m+o représente un nombre entier non inférieur à 3 et non supérieur à 450 et n représente un nombre entier non inférieur à 20 et non supérieur à 300.

5. Particules de polymère selon l'une quelconque des revendications 1 à 3, dans lesquelles le copolymère à ramification terminale est représenté par la formule générale (la) suivante : 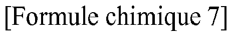 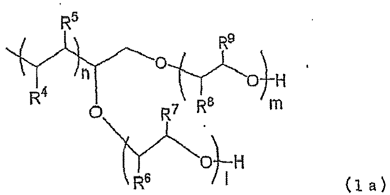 dans laquelle R⁴ et R⁵ représentent chacun un atome d'hydrogène ou un groupe alkyle ayant entre 1 et 18 atomes de carbone, l'un au moins des groupes R⁴ et R⁵ étant un atome d'hydrogène ; R⁶ et R⁷ représentent chacun un atome d'hydrogène ou un groupe méthyle, l'un au moins des groupes R⁶ et R⁷ étant un atome d'hydrogène ; R⁸ et R⁹ représentent chacun un atome d'hydrogène ou un groupe méthyle, l'un au moins des groupes R⁸ et R⁹ étant un atome d'hydrogène ; 1+m représente un nombre entier non inférieur à 2 et non supérieur à 450 et n représente un nombre entier non inférieur à 20 et non supérieur à 300.

6. Particules de polymère selon l'une quelconque des revendications 1 à 5, dans lesquelles la partie chaîne polyoléfinique du copolymère à ramification terminale présente une certaine cristallinité.

7. Particules de polymère selon l'une quelconque des revendications 1 à 6, dans lesquelles un autre dispersoïde est contenu dans une quantité comprise entre 0,001 partie en poids et 20 parties en poids, rapportées à 100 parties en poids de copolymère à ramification terminale.

8. Particules de polymère selon la revendication 7, un autre dispersoïde étant encapsulé dans les particules de polymère.

9. Solution de dispersion comprenant un dispersoïde composé des particules de polymère selon l'une quelconque des revendications 1 à 8 et d'eau et/ou d'un solvant organique ayant une affinité pour l'eau contenant le dispersoïde dispersé.

10. Solution de dispersion selon la revendication 9, ledit dispersoïde étant dispersé dans l'eau et/ou un solvant organique ayant une affinité pour l'eau.

11. Solution de dispersion selon la revendication 9 ou 10, dans laquelle le pH est compris entre 1 et 13.

12. Composition mixte comprenant les éléments (A) à (D) suivants :
(A) des particules de copolymère selon la revendication 1,
(B) un alcoxyde métallique et/ou un condensat d'hydrolyse de celui-ci,
(C) de l'eau et/ou un solvant permettant de dissoudre une partie de l'eau ou la totalité de l'eau dans toute proportion, et
(D) un catalyseur à utiliser pour la réaction sol-gel.

13. Composition mixte selon la revendication 12, dans laquelle, dans le copolymère à ramification terminale représenté par la formule générale (1), l'un quelconque des groupes X¹ et X² représente la formule générale (5) suivante : 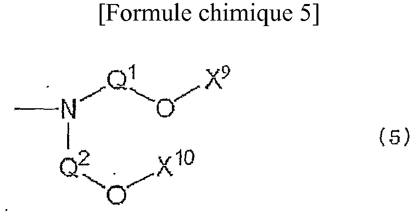 dans laquelle X⁹ et X¹⁰ peuvent être identiques ou différents, chacun représentant un groupe polyalkylène glycol ; et Q¹ et Q² pouvant être identiques ou différents, chacun représentant un groupe alkylène divalent.

14. Composition mixte selon l'une quelconque des revendications 12 et 13, dans laquelle, dans le copolymère à ramification terminale représenté par la formule générale (1), X¹ et/ou X² représentent la formule générale (6) suivante :
[Formule chimique 6] -Q-X¹¹ (6)
dans laquelle X¹¹ représente un groupe polyalkylène glycol.

15. Composition mixte selon l'une quelconque des revendications 12 à 14, le copolymère à ramification terminale étant représenté par la formule générale (1b) suivante : 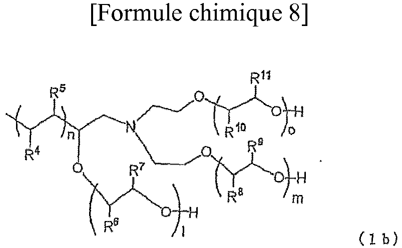 dans laquelle R⁴ et R⁵ représentent chacun un atome d'hydrogène ou un groupe alkyle ayant entre 1 et 18 atomes de carbone, l'un au moins des groupes R⁴ et R⁵ étant un atome d'hydrogène ; R⁶ et R⁷ représentent chacun un atome d'hydrogène ou un groupe méthyle, l'un au moins des groupes R⁶ et R⁷ étant un atome d'hydrogène ; R⁸ et R⁹ représentent chacun un atome d'hydrogène ou un groupe méthyle, l'un au moins des groupes R⁸ et R⁹ étant un atome d'hydrogène ; R¹⁰ et R¹¹ représentent chacun un atome d'hydrogène ou un groupe méthyle, l'un au moins des groupes R¹⁰ et R¹¹ étant un atome d'hydrogène ; 1+m+o représente un nombre entier non inférieur à 3 et non supérieur à 450 et n représente un nombre entier non inférieur à 20 et non supérieur à 300.

16. Composition mixte selon l'une quelconque des revendications 12 à 14, dans laquelle le copolymère à ramification terminale est représenté par la formule générale (la) suivante : 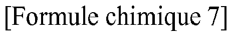 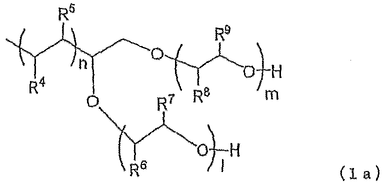 dans laquelle R⁴ et R⁵ représentent chacun un atome d'hydrogène ou un groupe alkyle ayant entre 1 et 18 atomes de carbone, l'un au moins des groupes R⁴ et R⁵ étant un atome d'hydrogène ; R⁶ et R⁷ représentent chacun un atome d'hydrogène ou un groupe méthyle, l'un au moins des groupes R⁶ et R⁷ étant un atome d'hydrogène ; R⁸ et R⁹ représentent chacun un atome d'hydrogène ou un groupe méthyle, l'un au moins des groupes R⁸ et R⁹ étant un atome d'hydrogène ; 1+m représente un nombre entier non inférieur à 2 et non supérieur à 450 et n représente un nombre entier non inférieur à 20 et non supérieur à 300.

17. Composition mixte selon l'une quelconque des revendications 12 à 16, dans laquelle, dans les particules de polymère, la partie chaîne polyoléfinique du copolymère à ramification terminale a une certaine cristallinité.

18. Composition mixte selon l'une quelconque des revendications 12 à 17, dans laquelle un autre dispersoïde est contenu dans une quantité comprise entre 0,001 partie en poids et 20 parties en poids, rapportées à 100 parties en poids du copolymère à ramification terminale.

19. Particules de polymère de la composition mixte selon la revendication 18, dans laquelle un autre dispersoïde est encapsulé dans les particules de polymère.

20. Composition mixte selon l'une quelconque des revendications 12 à 19, dans laquelle un métal dudit alcoxyde métallique et/ou un condensat d'hydrolyse de celui-ci (B) consistent en un ou plusieurs des éléments du groupe constitué par le silicium, le zirconium, l'aluminium et le titane.

21. Composition mixte selon l'une quelconque des revendications 12 à 20, dans laquelle ledit solvant (C) est de l'eau.

22. Composition mixte selon l'une quelconque des revendications 12 à 21, dans laquelle ledit solvant (C) est un monoalcool ayant entre 1 et 3 atomes de carbone.

23. Composite obtenu par dispersion de particules de polymère selon l'une quelconque des revendications 1 à 8 dans une matrice composée d'oxyde métallique.

24. Composite selon la revendication 23 obtenu par la réaction sol-gel de la composition mixte selon l'une quelconque des revendications 12 à 22.

25. Composite selon la revendication 23 ou 24, ledit oxyde métallique étant formé par la réaction sol-gel d'un alcoxyde métallique et/ou d'un condensat d'hydrolyse de celui-ci.

26. Composite selon l'une quelconque des revendications 23 à 25, comprenant en outre des sels de métal alcalin en tant qu'agent antistatique.

27. Composite selon la revendication 26, dans lequel les sels de métal alcalin contiennent au moins un sel de lithium anionique choisi dans le groupe constitué par l'acide trifluorométhanesulfonique, le bis(trifluorométhanesulfonyl)imide et le tri(trifluorométhanesulfonyl)méthane.

28. Stratifié plastique comprenant une couche de revêtement composée du composite selon l'une quelconque des revendications 23 à 27 sur un matériau de base en plastique.

29. Stratifié plastique selon la revendication 28, comprenant une couche pelliculaire déposée, composée d'un composé inorganique, entre ledit matériau de base en plastique et ladite couche de revêtement.

30. Matériau formant barrière aux gaz comprenant le stratifié en plastique selon la revendication 28 ou 29.

31. Matériau de revêtement dur comprenant le stratifié en plastique selon la revendication 28 ou 29.

32. Film antistatique comprenant le stratifié en plastique selon la revendication 28 ou 29.

33. Composition d'encre comprenant des particules de polymère selon l'une quelconque des revendications 1 à 8 ou la solution de dispersion selon l'une quelconque des revendications 9 à 11.

34. Agent de revêtement comprenant les particules de polymère selon l'une quelconque des revendications 1 à 8 ou la solution de dispersion selon l'une quelconque des revendications 9 à 11.

35. Préparation cosmétique comprenant les particules de polymère selon l'une quelconque des revendications 1 à 8 ou la solution de dispersion selon l'une quelconque des revendications 9 à 11.

36. Film antistatique obtenu par application d'un agent de revêtement selon la revendication 34.
